(19) Europäisches Patentamt
European Patent Office
Office européen des brevets

(11) Numéro de publication: **0 210 084 B1**

(12) # FASCICULE DE BREVET EUROPEEN

(45) Date de publication du fascicule du brevet: **08.02.89**

(21) Numéro de dépôt: **86401140.8**

(22) Date de dépôt: **29.05.86**

(51) Int. Cl.⁴: **C 07 D 239/74**, C 07 D 217/14, C 07 D 215/12, C 07 D 215/22, C 07 D 239/82, C 07 D 239/91, C 07 D 217/24, C 07 D 215/36, C 07 D 215/42, C 07 C 103/34, C 07 C 125/067

(54) **Amides, procédés pour leur préparation et médicaments les contenant.**

(30) Priorité: **30.05.85 FR 8508111**

(43) Date de publication de la demande: **28.01.87 Bulletin 87/5**

(45) Mention de la délivrance du brevet: **08.02.89 Bulletin 89/6**

(84) Etats contractants désignés: **AT BE CH DE FR GB IT LI LU NL SE**

(56) Documents cités:
**EP-A- 0 112 776**
**FR-M- 8 411**
**GB-A- 1 064 252**
**GB-A- 1 177 548**
**US-A- 3 595 861**

(73) Titulaire: **RHONE-POULENC SANTE, 20, avenue Raymond Aron, F-92160 Antony (FR)**

(72) Inventeur: **Benavides, Jesus, 47 Avenue de Seine, F-92500 Rueil-Malmaison (FR)**
Inventeur: **Dubroeucq, Marie-Christine, 13 Villa Malleville, F-95880 Enghien les Bains (FR)**
Inventeur: **Le Fur, Gérard, 19 ter rue des Carrières, F-95160 Montmorency (FR)**
Inventeur: **Renault, Christian, 61 rue des Mallets, F-95150 Taverny (FR)**

(74) Mandataire: **Savina, Jacques et al, RHONE-POULENC INTERSERVICES Service Brevets Pharma 25, quai Paul Doumer, F-92408 Courbevoie Cédex (FR)**

EP 0 210 084 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

La présente invention a pour objet de nouveaux amides, leurs procédés de préparation et les médicament les contenant.

Des amides dérivés de quinazoline utiles comme tranquillisants sont décrits dans le brevet américain 3 595 861.

Le brevet EP 112 776 décrit des amides dérivés de quinazoline, de quinoléine, d'isoquinoléine et de naphtalène utiles comme tranquillisants.

Les amides selon l'invention peuvent être représentés par la formule générale:

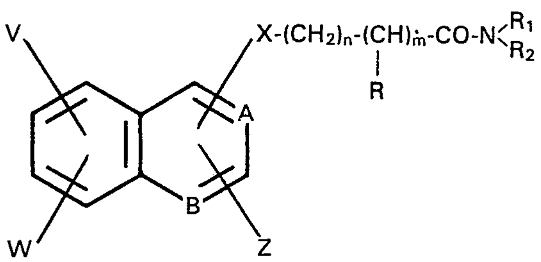

(I)

dans laquelle A représente un atome d'azote ou un groupe CH,

B représente un atome d'azote ou un groupe CH,

V et W, identiques ou différents, représentent des atomes d'hydrogène ou d'halogène (fluor, chlore, brome), des groupes alkyle ou alcoxy comportant 1 à 3 atomes de carbone, des groupes nitro ou trifluorométhyle,

Z est fixé en position ortho ou para par rapport à B et représente un radical phényle, thiényle, pyridyle ou phényle substitué par un ou deux substituants pris parmi les atomes d'halogène, les groupes alkyle et alcoxy comportant 1 à 4 atomes de carbone, les groupes trifluorométhyle ou nitro,

la chaîne $X-(CH_2)_n-(CHR)_m-CONR_1R_2$ est fixée en position ortho ou para par rapport à B,

E représente un atome d'hydrogène ou un groupe alkyle comportant 1 à 3 atomes de carbone,

$R_1$ et $R_2$, identiques ou différents, représentent un groupe alkyle linéaire ou ramifié comportant 1 à 6 atomes de carbone, cycloalkyle comportant 3 à 6 atomes de carbone, phényle, phénylalkyle ou cycloalkylalkyle dont la partie alkyle comporte 1 à 3 atomes de carbone et la partie cycloalkyle comporte 3 à 6 atomes de carbone, alcényle comportant 3 à 6 atomes de carbone à condition que la double liaison ne soit pas située en position 1, 2 par rapport à l'atome d'azote,

$R_1$ et $R_2$ peuvent également former ensemble avec l'atome d'azote auquel ils sont rattachés un cycle pipéridine ou thiomorpholine,

X représente un groupe $CH-R_3$, $N-R_4$, SO, $SO_2$ ou un atome d'oxygène ou de soufre,

$R_3$ représente un atome d'hydrogène ou un groupe alkyle comportant 1 à 3 atomes de carbone,

$R_4$ représente un groupe alkyle comportant 1 à 3 atomes de carbone

m est égal à 0 ou 1,

n est égal à 0, 1 ou 2,

étant entendu que lorsque X représente un groupe SO, $SO_2$ ou $N-R_4$, la somme m + n est au moins égale à 1, étant entendu que lorsque A et B représentent chacun un atome d'azote et Z est en position para par rapport à B, X ne peut pas représenter le groupe $CH-R_3$, étant entendu que lorsque A représente un groupe CH, B représente un atome d'azote, Z est en position ortho par rapport à B, X représente un atome d'oxygène et R représente un atome d'hydrogène, la somme m + n est différente de 1; étant entendu que lorsque A représente un groupe CH, B représente un atome d'azote, X représente un groupe $CH_2$ et R représente un atome d'hydrogène, la somme m + n est différente de 2, et à l'exception du N,N-diméthylcarbamate de phényl-2 quinolyle-4.

Autrement dit, les composés de formule I répondent à l'une des deux formules Ia ou Ib

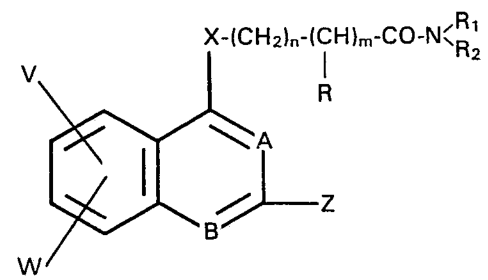

(Ia)

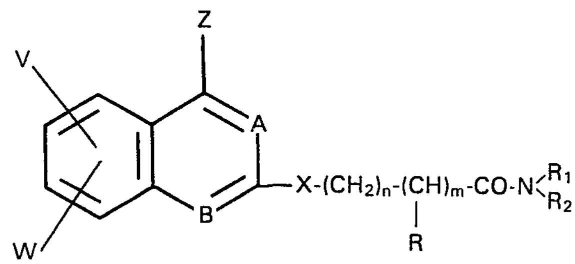

(Ib)

dans lesquelles A, B, V, W, X, Z, R, $R_1$, $R_2$, n et m ont les significations mentionnées ci-dessus.

Lorsque la chaîne $X-(CH_2)_n-(CHR)_m-CO-NR_1R_2$ comporte 1 ou 2 atomes de carbone asymétriques, il y a plusieurs stéréoisomères correspondant à la formule plane I. Ces divers stéréoisomères font également partie de l'invention, de même que lorsqu'ils peuvent exister, les sels d'addition des composés racémiques ou stéréoisomères de formule I avec des acides minéraux ou organiques.

Les composés de formule I dans laquelle X représente un atome d'oxygène ou de soufre, n et m sont égaux à 0, et A, B, V, W, Z, $R_1$ et $R_2$ ont les mêmes significations que dans la formule I c'est-à-dire les carbamates et thiocarbamates, peuvent être obtenus par action d'un composé de formule:

$$Cl-CO-N<^{R_1}_{R_2} \quad (II)$$

dans laquelle $R_1$ et $R_2$ ont les mêmes significations que dans la formule I, sur un dérivé de formule:

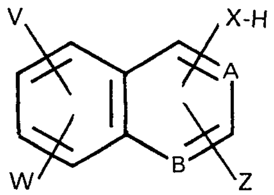

(III)

dans laquelle A, B, V, W et Z ont les mêmes significations que dans la formule I et X représente un atome d'oxygène ou de soufre.

Cette réaction est réalisée selon des procédés connus en soi, permettant de transformer un groupe OH ou SH respectivement en carbamate ou thiocarbamate, tels que ceux décrits dans R.B. WAGNER et H.D. HAGEMANN, Houben Weyl, Methoden der Organischen Chemie, Kohlensäurederivate, B et E4, p. 154 et 297 (1983). On peut par exemple effectuer cette réaction au sein d'un solvant inerte tel que le tétrahydrofuranne ou le diméthylformamide en présence d'une base organique tertiaire telle que la triéthylamine et éventuellement en présence d'un catalyseur tel que la diméthylaminopyridine, à une température comprise entre 20°C et 70°C.

La plupart des composés de formule III sont connus; les composés nouveaux peuvent être obtenus par application ou adaptation des méthodes décrites par C. HAUSER et A. REYNOLDS, J.A.C.S., 70, 2402-2404 (1948), GABRIEL, Ber. 29, 131 (1896), H. STEPHEN, J. Chem. Soc., 4420 (1956), D.W. JONES, J. Cem. Soc., 1729 (1969), GABRIEL, Chem. Ber. 18, 3471 (1885), A. KASAHARA, Chem. Ind. 16, 666 (1980) et 4, 121 (1981), W.I. BOYCE, J. Org. Chem., 31, 3807 (1966), SORM, Chem. Listy, 49, 901 (1954).

Les composés de formule I dans laquelle X représente un atome d'oxygène ou de soufre, n est égal à 0, m est égal à 1 et A, B, V, W, Z, R, $R_1$, $R_2$ ont les mêmes significations que dans la formule I peuvent être préparés par action d'un composé de formule:

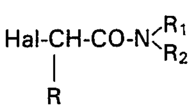

(IV)

dans laquelle R, $R_1$ et $R_2$ ont les mêmes significations que dans la formule I et Hal représente un atome d'halogène (chlore ou brome) sur un dérivé de formule III dans laquelle X représente un atome d'oxygène ou de soufre, A, B, V, W et Z one les mêmes significations que dans la formule I. Cette réaction peut être réalisée selon des procédés connus comme celui décrit dans Chem. Abst. 95, 203 770K (1981) qui consiste à opérer en présence d'un base telle que le carbonate de potassium, de préférence en présence d'iodure cuivreux, au sein d'un solvant tel que la butanone-2 et à une température comprise entre 20°C et la température d'ébullition du solvant.

Les composés de formule I dans laquelle soit X représente un atome d'oxygène ou de soufre, n est égal à 0, 1 ou 2, m est égal à 1 soit X représente un groupe CH-$R_3$, n est égal à 0, 1 ou 2, m est égal à 0 ou 1 et A, B, V, W, Z, R, $R_1$ et $R_2$ ont les mêmes significations que dans la formule I peuvent être préparés par action d'une amine de formule:

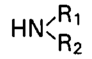

(V)

dans laquelle $R_1$ et $R_2$ ont les mêmes significations que dans la formule I, sur un dérivé de formule:

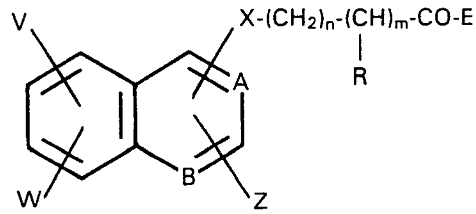

(VI)

dans laquelle A, B, V, W, Z, R et n ont les mêmes significations que dans la formule I, E représente un groupe alcoxy comportant 1 à 4 atomes de carbone, un groupe alcoxycarbonyloxy comportant 2 à 5 atomes de carbone, un atome de chlore ou un reste N-imidazolyle, soit X représente un atome d'oxygène ou de soufre, m est égal à 1, soit X représente un groupe CH-$R_3$, m est égal à 0 ou 1 et $R_3$ représente un atome d'hydrogène ou un groupe alkyle comportant 1 à 3 atomes de carbone.

Cette réaction peut être réalisée selon des procédés connus en soi, permettant de transformer un ester d'acide carboxylique, un chlorure d'acide carboxylique, un anhydride mixte ou un azolide en carboxamide tels que ceux décrits par C.A. BUEHLER et D.E. PEARSON, Survey of Organic Synthesis, Wiley interscience, p. 894, 1970.

Lorsque E est un groupe alcoxy comportant 1 à 4 atomes de carbone, un procédé avantageux consiste à chauffer à une température comprise entre 120°C et 180°C l'ester de formule VI au sein de l'amine de formule V en excès.

Lorsque E est un atome de chlore, on peut traiter le chlorure d'acide de formule VI par un excès de l'amine de formule V au sein d'un solvant inerte tel que le toluène, le chloroforme ou le chlorure de méthylène à une température comprise entre 20°C et la température d'ébullition du solvant utilisé. L'excès d'amine utilisé, qui joue le rôle de base neutralisant l'acide chlorhydrique formé dans la réaction est d'au moins un équivalent c'est-à-dire que la quantité totale d'amine employée est au moins de deux équivalents. Dans le cas où A ou B représente un atome d'azote, le chlorure d'acide de formule VI peut être utilisé sous forme de chlorhydrate à condition d'employer au moins un équivalent supplémentaire de l'amine de formule V afin de faire passer le chlorure d'acide de la forme chlorhydrate à la forme base libre.

Lorsque E est un atome de chlore, on peut aussi faire réagir le chlorure d'acide de formule VI avec l'amine de formule V en présence d'une amine tertiaire telle que la triéthylamine, au sein d'un solvant inerte tel que le toluène, le chloroforme ou le chlorure de méthylène, à une température comprise entre 20°C et la température d'ébullition du solvant.

On peut aussi faire réagir le chlorure d'acide de formule VI avec l'amine de formule V au sein de la pyridine qui sert à la fois de base fixant l'acide formé et de solvant.

Lorsque E est un groupe alcoxycarbonyloxy comportant 2 à 5 atomes de carbone, on peut traiter l'anhydride mixte de formule VI avec l'amine de formule V au sein d'un solvant inerte tel que le benzène, le toluène, le chloroforme ou le chlorure de méthylène à une température de −5°C à +25°C.

Lorsque E est un reste N-imidazolyle, on peut faire réagir l'azolide de formule VI avec l'amine de formule V dans un solvant inerte tel que te tétrahydrofuranne ou le diméthylformamide à une température comprise entre 20°C et la température d'ébullition du solvant.

Les composés de formule VI peuvent être obtenus par action sur un acide de formule:

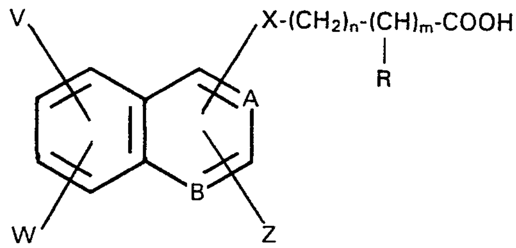

(VII)

dans laquelle A, B, V, W, Z, X, R, m et n ont les mêmes significations que dans la formule VI d'un alcool aliphatique saturé de bas poids moléculaire (1-4C) tel que le méthanol de l'éthanol (cas où E est un groupe alcoxy), d'un agent de chloruration tel que le chlorure de thionyle (cas où E est un atome de chlore), d'un chloroformiate d'alkyle de bas poids moléculaire (1-4C) tel que le chloroformiate de méthyle ou d'éthyle (cas où E est un groupe alcoxycarbonyloxy) ou du carbonyldiimidazole (cas où E est un reste N-imidazolyle).

La réaction de l'acide de formule VII avec l'alcool aliphatique saturé de bas poids moléculaire peut être réalisée en chauffant à la température du reflux l'acide de formule VII au sein dudit alcool, en présence d'un acide minéral tel que l'acide sulfurique ou l'acide chlorhydrique.

La réaction de l'acide de formule VII avec l'agent de chloruration peut être réalisée en l'absence de solvant ou au sein d'un solvant inerte tel que le chloroforme ou le toluène, de préférence à la température de reflux du milieu.

La réaction de l'acide de formule VII avec le chloroformiate d'alkyle de bas poids moléculaire (1-4C) peut être réalisée au sein d'un solvant inerte tel que le chloroforme ou le chlorure de méthylène, à une température de −5°C à +25°C, en présence d'une amine tertiaire telle que la triéthylamine, on peut ensuite faire réagir in situ, l'anhydride mixte ainsi formé avec l'amine de formule V.

La réaction de l'acide de formule VII avec le carbonyldiimidazole peut être effectuée, sous azote, au sein d'un solvant inerte tel que le tétrahydrofuranne ou le diméthylformamide, à une température comprise entre −5°C et +30°C. On peut ensuite faire réagir in situ, l'azolide ainsi formé avec l'amine de formule V.

Certains acides de formule VII sont connus tel l'acide phényl-2 quinoléine-4 propionique [J. HANNS, Ber. 58, 2799 (1925)]. Ceux qui ne le sont pas peuvent être obtenus par application ou adaptation des méthodes décrites dans les exemples 7, 8, 10 à 14, 20, 21, 22, 24, 25, 26, 43, 44, 51, 54 à 56, 68, 85 et 86 et dans R.B. WAGNER et H.D. ZOOK, Synthetic Organic Chemistry, J. Wiley, p. 411-478 (1953), C.A. BUEHLER et D.E. PEARSON, Survey of organic synthesis, Wiley interscience, p. 655-710 (1970).

Les composés de formule I dans laquelle A est un groupe CH, B est un atome d'azote, Z est fixé en position ortho par rapport à B, la chaîne $X-(CH_2)_n-(CHR)_m-CO-NR_1R_2$ est fixée en position para par rapport à B, X représente le groupe $CH-R_3$, $R_3$ représente un groupe alkyle comportant 1 à 3 atomes de carbone, m est égal à 0, n est égal à 0, V, W, Z, $R_1$ et $R_2$ ayant les mêmes significations que dans la formule I, peuvent être préparés par alkylation des composés de formule:

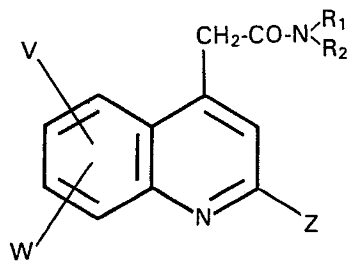

(VIII)

dans laquelle V, W, Z, $R_1$ et $R_2$ ont les mêmes significations que dans la formule I avec un composé de formule $R_3$-Hal dans laquelle $R_3$ est une groupe alkyle comportant 1 à 3 atomes de carbone et Hal représente un atome d'halogène (brome ou iode). Cette réaction peut être effectuée selon des méthodes connues telles que celles décrites par J.C. STOWELL, Carbanions in Organic Synthesis, p. 161 (1979), Wiley Sons.

Un procédé avantageux consiste à opérer sous azote, à une température comprise entre −70°C et 0°C, en présence d'une base forte telle que le diisopropylamidure de lithium.

Les composés de formule I dans laquelle X représente un groupe sulfinyle ou sulfonyle, la somme m+n est au moins égale à 1, A, B, V, W, Z, R, $R_1$ et $R_2$ ont les mêmes significations que dans la formule I peuvent être préparés par oxydation des composés correspondants de formule I dans laquelle X représente un atome de soufre.

Cette réaction peut être effectuée selon des procédés connus en soi, permettant de transformer un sulfure en sulfoxyde ou sulfone, tels que ceux décrits par D. BARTON et W.D. OLLIS, Comp. Organic Chemistry, tome 3, p. 124 et 174 (1979), Pergamon Press.

Ces procédés consistent à oxyder un sulfure en sulfoxyde ou un sulfoxyde en sulfone au moyen d'un agent oxydant tel que l'eau oxygénée, le métapériodate de sodium ou un peracide dans un solvant tel qu'un alcool comme l'éthanol ou un acide tel que l'acide acétique, à une température comprise entre 0°C et 80°C.

Les composés de formule I dans laquelle X représente un groupe $N-R_4$, m est égal à 1, A, B, V, W, Z, R, $R_1$, $R_2$ et n ont les mêmes significations que dans la formule I et $R_4$ représente un groupe alkyle comportant 1 à 3 atomes de carbone peuvent être préparés par réaction d'un aminoalkylamide de formule:

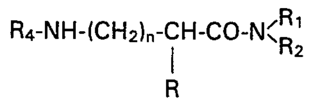

(IX)

sur un dérivé de formule:

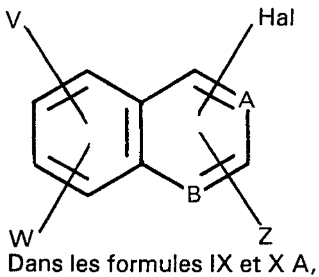

(X)

Dans les formules IX et X A, B, V, W, Z, R, $R_1$, $R_2$ et n ont les mêmes significations que dans la formule I, $R_4$ représente un groupe alkyle comportant 1 à 3 atomes de carbone et Hal représente un atome d'halogène (chlore ou brome).

Cette réaction peut être réalisée selon des procédés connus en soi permettant d'effectuer des substitutions nucléophiles aromatiques sur des dérivés halogénés tels que ceux décrits par A.R. SURVEY et Coll., J. Am. Chem. Soc., 73, 2623 (1951).

Dans le cas où A ou B est un atome d'azote, un procédé avantageux consiste à opérer en présence de phénol à une température comprise entre 125°C et 180°C.

Les composés de formule I dans laquelle A et B représentent chacun un atome d'azote, Z est fixé en position ortho par rapport à B, la chaîne $X-(CH_2)_n-(CHR)_m-CO-NR_1R_2$ est fixée en position para par rapport à B, X est un groupe $CH-R_3$, $R_3$ est un atome d'hydrogène, n est égal à 1, m est égal à 0, V, W, Z, $R_1$ et $R_2$ ont les mêmes significations que dans la formule I peuvent être préparés à partir des composés de formule:

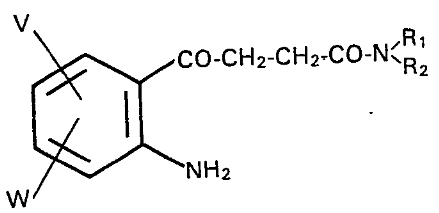

(XI)

dans laquelle V, W, $R_1$ et $R_2$ ont les mêmes significations que dans la formule I par action d'un chlorure ZCOCl dans lequel Z a les mêmes significations que dans la formule I, et cyclisation.

Cette réaction peut être effectuée selon W.L.F. ARMAREGO, Fused Pyrimidines, Part I, quinazolines, p. 39, Intersciences Publishers, Wiley, 1967.

Ce procédé consiste à traiter le composé XI par le chlorure d'acide ZCOCl, au sein d'un solvant inerte tel que le chloroforme, entre 20°C et 25°C puis par l'acétate d'ammonium, de préférence en présence acétique, à une température comprise entre 100°C et 120°C.

Les énantiomères des composés de formule I dans laquelle la chaîne $X-(CH_2)_n-(CHR)_m-CO-NR_1R_2$ comporte un ou deux carbones asymétriques peuvent être obtenus par dédoublement des racémiques, par exemple, par chromatographie sur colonne chirale selon W.H. PIRKLE et Coll., asymetric synthesis, vol. 1, Academic Press (1983) ou encore par synthèse à partir de précurseurs chiraux.

Les mélanges réactionnels obtenus par les divers procédés décrits précédemment sont traités suivant des méthodes classiques physiques (évaporation, extraction, distillation, cristallisation, chromatographie) ou chimiques le cas échéant (formation de sel et régénération de la base ou de l'acide) afin d'isoler les composés de formule I à l'état pur.

Lorsque cela est possible, les composés de formule I sous forme de base libre peuvent être éventuellement transformés en sels d'addition avec un acide minéral ou organique par action d'un tel acide au sein d'un solvant organique tel qu'un alcool, une cétone, un éther ou un solvant chloré.

Les composés de formule I et leurs sels présentent des propriétés pharmacologiques intéressantes. Ces composés se lient aux récepteurs des benzodiazépines du type périphérique et sont donc utiles comme anxiolytiques, anticonvulsivants, antiangoreux et pour le traitement des états d'immunodépression.

L'affinité des composés de formule I pour les sites récepteurs des benzodiazépines de type périphérique a été déterminée en utilisant le protocole de BRAESTRUP et Coll., Proc. Natl. Acad. Sci. USA, 74, 3805 (1977) sur des membranes de rein de rat avec comme ligant le $^3H$ - PK 11195 (N-méthyl N-(méthyl-1 propyl) (chloro-2 phényl)-1 isoquinoléinecarboxamide-3). Cette affinité est comprise entre 0,001 et 1,5 µM.

Les composés selon l'invention présentent une faible toxicité. Leur $DL_{50}$ par voie orale chez la souris est supérieure à 200 mg/kg. Les $DL_{50}$ ont été calculées après 3 jours d'observation par la méthode cumulative de J.J. REED et H. MUENCH, Amer.J. Hyg., 27, 493 (1938).

Sont particulièrement intéressants les composés de formule I dans laquelle V et W, identiques ou différents, représentent des atomes d'hydrogène ou des groupes alkyle comportant 1 à 3 atomes de carbone,

Z est fixé en position ortho par rapport à B et représente un radical phényle éventuellement substitué par un groupe alkyle, alcoxy comportant 1 à 4 atomes de carbone, un groupe nitro ou trifluorométhyle ou un radical thiényle,

la chaîne $X-(CH_2)_n-(CHR)_m-CO-NR_1R_2$ est fixée en position para par rapport à B,

R représente un atome d'hydrogène ou un groupe alkyle comportant 1 à 3 atomes de carbone,

$R_1$ et $R_2$, identiques ou différents, représentent un groupe alkyle linéaire ou ramifié comportant 1 à 6 atomes de carbone ou un groupe phényle,

$R_1$ et $R_2$ peuvent également former ensemble avec l'atome d'azote auquel ils sont rattachés un cycle pipéridine,

X représente un groupe $CH-R_3$ ou un atome d'oxygène ou de soufre, $R_3$ représente un atome d'hydrogène,

m est égal à 0 ou 1,

n est égal à 0, 1 ou 2,

et soit A représente un groupe CH et B un atome d'azode, soit A représente un atome d'azote et B un groupe CH, soit A et B représentent un atome d'azote étant entendu que lorsque A et B représentent chacun un atome d'azote et Z est en position para par

rapport à B, X ne peut pas représenter le groupe $CH-R_3$, étant entendu que lorsque A représente une groupe CH, B représente un atome d'azote, Z est en position ortho par rapport à B, X représente un atome d'oxygène et R représente un atome d'hydrogène, la somme m + n est différente de 1; étant entendu que lorsque A représente un groupe CH, B représente un atome d'azote, X représente un groupe $CH_2$ et R représente un atome d'hydrogène, la somme m + n est différente de 2, et à l'exception du N,N-diméthyl-carbamate de phényl-2 quinolyle-4.

Sont particulièrement intéressants les composés suivants:

- N,N-diéthyl phényl-2 quinazoline-4 propanamide
- N,N-diéthyl (méthoxy-3 phényl)-2 quinazoline-4 propanamide
- N,N-diéthyl phényl-3 isoquinoléine-1 propanamide
- N,N-diéthyl phényl-2 quinoléine-4 acétamide
- N,N-diéthyl α-méthyl phényl-2 quinazoline-4 propanamide
- N-méthyl N-phényl-2 quinazoline-4 propanamide
- [(phényl-2 quinazolinyl-4)-3 propionyl]-1 pipéridine
- N,N-diéthyl (nitro-4 phényl)-2 quinazoline-4 propanamide
- N,N-diéthyl α-méthyl phényl-3 isoquinoléine-1 propanamide
- N,N-diéthyl α-méthyl phényl-2 quinoléine-4 propanamide
- N,N-diéthyl α-méthyl phényl-2 quinoléine-4 propanamide lévogyre
- N,N-diéthyl (trifluorométhyl-3 phényl)-2 quinazoline-4 propanamide
- N,N-diéthyl thiényle-2 quinazoline-4 propanamide
- N,N-diéthyl méthyl-8 phényl-2 quinazoline-4 propanamide
- Diéthylcarbamate de phényl-4 quinolyle-2
- Diéthylcarbamate de phényl-2 quinazolyle-4
- Diéthylcarbamate de phényl-3 quinolyle-1
- Diéthylcarbamate de (méthyl-4 phényl)-3 isoquinolyle-1
- N,N-diéthyl [(phényl-2 quinolyl-4) oxy]-2 propanamide
- N,N-diéthyl (phényl-2 quinazolinyl-4) oxyacétamide
- N,N-diéthyl (phényl-3 isoquinolyl-1) oxyacétamide
- N,N-diéthyl [(phényl-3 isoquinolyl-1) oxy]-2 propanamide
- N,N-diéthyl [(phényl-2 quinazolinyl-4) oxy]-2 propanamide
- N,N-diéthyl [(phényl-2 quinazolinyl-4) oxy]-2 propanamide dextrogyre
- N,N-diéthyl [(phényl-2 quinolyl-4) thio]-2 propanamide
- [(phényl-2 quinolyl-4)-3 propionyl]-4 morpholine
- Diéthylcarbamate de (méthoxy-4 phényl)-2 quinolyle-4
- Diéthylcarbamate de nitro-6 phényl-2 quinolyle-4
- Diéthylcarbamate de (méthyl-4 phényl)-2 quinolyle-4
- Diéthylcarbamate de (fluoro-2 phényl)-2 quinolyle-4
- Diéthylcarbamate de (thiényl-2)-2 quinolyle-4
- Diéthylcarbamate de (chloro-3 phényl)-2 quinolyle-4

Pour l'emploi médicinal, il peut être fait usage des produits de formule I tels quels ou lorsqu'ils peuvent exister, à l'état de sels avec un acide fort pharmaceutiquement acceptable.

Comme sels pharmaceutiquement acceptables, on peut citer les sels d'addition avec les acides minéraux tels que chlorhydrates, sulfates, nitrates, phosphates ou organiques tels que acétates, propionates, succinates, benzoates, fumarates, théophillineacétates, salicylates, phénolphtalinates, méthylène bis-β-oxynaphtoates ou des dérivés de substitution de ces composés.

Les exemples suivants, donnés à titre non limimatif, montrent comment l'invention peut être mise en pratique.

*Exemple 1*

Un mélange de 4,3 g de phényl-2 quinazoline-4 propionate d'éthyle et de 30 $cm^3$ de diéthylamine est chauffé à 250°C pendant 40 heures. Après refroidissement, l'excès de diéthylamine est évaporé. Le résidu est chromatographié sur du gel de silice avec un mélange cyclohexane-acétate d'éthyle (1-1 en volume) comme éluant. On récupère 3,2 g de produit qui est recristallisé dans de l'éther isopropylique. On obtient 2,2 g de N,N-diéthyl phényl-2 quinazoline-4 propanamide qui fond à 103°C.

Le phényl-2 quinazoline-4 propionate d'éthyle est préparé par estérification de l'acide correspondant au moyen d'éthanol en présence d'acide sulfurique.

*Exemple 2*

On opère comme à l'exemple 1, à partir de 1,2 g de (méthoxy-3 phényl)-2 quinazoline-4 propionate d'éthyl et de 30 $cm^3$ de diéthylamine, Après chromatographie sur du gel de silice avec un mélange cyclohexane-acétate d'éthyle (1-1 en volume) comme éluant et cristallisation dans l'éther isopropylique on obtient 0,36 g de N,N-diéthyl (méthoxy-3 phényl)-2 quinazoline-4 propanamide qui fond à 87°C.

Le (méthoxy-3 phényl)-2 quinazoline-4 propionate d'éthyle est préparé selon le procédé suivant:

On agite 17 heures à la température ambiante (20°C environ) 26,7 g d'acide (amino-2 benzoyl)-2 propionique et 25 $cm^3$ d'acide sulfurique concentré dans 250 $cm^3$ d'éthanol absolu. On évapore l'éthanol sous pression réduite, ajoute 200 $cm^3$ d'eau, 200 $cm^3$ d'acétate d'éthyle et du carbonate de potassium jusqu'à pH 8. On filtre, décante et réextrait la phase aqueuse avec 2 fois 100 $cm^3$ d'acétate d'éthyle. La phase organique est séchée sur sulfate de magnésium, filtrée et évaporée à sec sous pression réduite. On obtient 24,8 g d'(amino-2 benzoyl)-3 propionate d'éthyle sous forme d'huile. Le spectre RMN du proton, dans le chloroforme deutérié présente les caractéristiques suivantes:

$CH_2$-$\underline{CH_3}$ δ : 1,2 ppm $\underline{CH_2}$ - $COOC_2H_5$ δ : 2,8 ppm
$\underline{CH_2}$-$CH_3$ δ : 4,2 ppm $H_6$ δ : 7,9 ppm

| | | | |
|---|---|---|---|
| Ar-CO-$\underline{CH_2}$- | δ : 3,3 ppm | $H_4$ | δ : 7,3 ppm |
| $NH_2$ | δ : 5,7 ppm | $H_3$ et $H_5$ | δ : 6,7 ppm |

A 2,21 g d'(amino-2 benzoyl)-3 propionate d'éthyle et 4,2 $cm^3$ de triéthylamine dans 25 $cm^3$ de chloroforme on ajoute, à 5°C, 2,81 $cm^3$ de chlorure de méthoxy-3 benzoyle. On laisse 1 heure à la température ambiante (environ 20°C), ajoute 25 $cm^3$ d'eau et décante. On évapore la phase organique sous pression réduite et reprend le résidu avec 17 g d'acétate d'ammonium. On porte l'ensemble à 100°C pendant 7 heures puis évapore l'acide acétique formé sous pression réduite. On verse le résidu sur 100 $cm^3$ d'eau et extrait la phase aqueuse avec 3 fois 50 $cm^3$ d'acétate d'éthyle. On sèche la phase aqueuse sur sulfate de magnésium, la filtre et l'évapore à sec sous pression réduite. On chromatographie le produit résiduel sur du gel de silice avec un mélange cyclohexane-acétate d'éthyle (1-1 en volume) comme éluant. Après recristallisation dans l'éthanol on obtient 1,5 g de (méthoxy-3 phényl)-2 quinazoline-4 propionate d'éthyle fondant à 70°C.

L'acide (amino-2 benzoyl)-3 propionate peut être préparé selon D.E. RIVETT et Coll., Aust. J. Chem., 24, 2717 (1971).

*Exemple 3*

On opère comme à l'exemple 1 à partir de 4,2 g de phényl-1 isoquinoléine-3 propionate d'éthyle et de 20 $cm^3$ de diéthylamine. Après une première chromatographie sur du gel de silice avec comme éluant un mélange cyclohexane-acétate d'éthyle (1-1 en volume) puis une deuxième chromatographie avec comme éluant de l'acétate d'éthyle et une cristallisation dans de l'éther de pétrole, on obtient 0,6 g de N,N-diéthyl phényl-1 isoquinoléine-3 propanamide fondant à 70°C.

Le phényl-1 isoquinoléine-3 propionate d'éthyle est préparé selon la technique suivante:

On porte 20 heures à ébullition un mélange de 20 g de méthyl-3 phényl-1 isoquinoléine, de 17,8 g de N-bromosuccinimide et de 0,2 g de peroxyde de benzoyle dans 685 $cm^3$ de tétrachlorure de carbone. On filtre et évapore le filtrat sous pression réduite. On chromatographie le résidu sur du gel de silice avec un mélange cyclohexane-acétate d'éthyle (9-1 en volume) comme éluant. On récupère 8,7 g de bromométhyl-3 phényl-1 isoquinoléine fondant à 109°C.

A 3,36 g d'hydrure de sodium à 80% dans l'huile et 80 $cm^3$ de tétrahydrofuranne anhydre, on ajoute, goutte à goutte, sous azote, une solution de 17,9 g de malonate de diéthyle dans 100 $cm^3$ de tétrahydrofuranne anhydre. Après 1 heure d'agitation, on introduit, goutte à goutte, une solution de 8,3 g de bromométhyl-3 phényl-1 isoquinoléine dans 100 $cm^3$ de tétrahydrofuranne et laisse sous agitation à la température ambiante (20°C environ) 1 heure. On ajoute 200 $cm^3$ d'eau et extrait la phase aqueuse avec 3 fois 200 $cm^3$ d'acétate d'éthyle. On sèche la phase organique sur du sulfate de magnésium, la fitre et l'évapore à sec sous pression réduite. On chromatographie le résidu sur du gel de silice avec un mélange cyclohexane-acétate d'éthyle (4-1 en volume) comme éluant. On récupère 9,6 g d'huile que l'on reprend dans 95 $cm^3$ d'acide chlorhydrique concentré et que l'on porte 20 heures à ébullition. Après refroidissement, on ajoute 200 $cm^3$ d'eau, lave la phase aqueuse avec 2 fois 50 $cm^3$ d'acétate d'éthyle, ajoute une lessive d'hydroxyde de potassium jusqu'à pH 5 et l'extrait avec 3 fois 50 $cm^3$ de chloroforme. On sèche la phase organique sur du sulfate de magnésium et l'évapore à sec sous pression réduite. On obtient 2,9 g d'acide phényl-1 isoquinoléine-3 propanoïque fondant à 146°C.

On agite 20 heures à la température ambiante (environ 20°C) 2 g d'acide phényl-1 isoquinoléine-3 propanoïque et 2 $cm^3$ d'acide sulfurique concentré dans 20 $cm^3$ d'éthanol absolu. On dilue avec 100 $cm^3$ d'eau, alcalinise à pH 9 avec une solution concentrée d'ammoniaque et extrait avec 3 fois 50 $cm^3$ de chlorure de méthylène. On sèche la phase organique sur du sulfate de magnésium, la filtre et l'évapore à sec sous pression réduite. On obtient 1,9 g de phényl-1 isoquinoléine-3 propionate d'éthyle sous forme d'huile dont le spectre RMN du proton, dans le chloroforme deutérié a les caractéristiques suivantes:

$H_8$ δ : 8 ppm
autres H aromatiques δ : 7,3 à 8 ppm.

| | | | |
|---|---|---|---|
| Ar-$CH_2$ | δ : 3,2 ppm | $CH_2$-$CH_3$ | δ : 4,2 ppm |
| -$CH_2$-$\underline{CH_2}$- | δ : 2,9 ppm | $CH_3$ | δ : 1,2 ppm |

La méthyl-3 phényl-1 isoquinoléine peut être préparée selon W. M. WHALEY et Coll., J. Org. Chem., 14, 650 (1949).

*Exemple 4*

On opère comme à l'exemple 1 à partir de 6,1 g de phényl-3 isoquinoléine-1 propionate d'éthyle et de 30 $cm^3$ de diéthylamine. Le produit brut est purifié au moyen de 4 chromatographies successives sur du gel de silice en utilisant un mélange cyclohexane-acétate d'éthyle (7-3 en volume) comme éluant. On obtient 1,4 g de N,N-diéthyl phényl-3 isoquinoléine-1 propanamide fondant à 58°C.

Le phényl-3 isoquinoléine-1 propionate d'éthyle est préparé selon le procédé suivant:

On porte à l'ébullition, 48 heures, un mélange de 21 g de méthyl-1 phényl-3 isoquinoléine, de 30,6 g de N-bromosuccinimide et de 1 g de peroxyde de benzoyle dans 730 $cm^3$ de tétrachlorure de carbone. Après refroidissement, on filtre et évapore le filtrat à sec sous pression réduite. On chromatographie le résidu sur du gel de silice avec un mélange toluène-méthanol (98-2 en volume) comme éluant. Après cristallisation dans l'éther isopropylique on obtient 11 g de bromométhyl-1 phényl-2 isoquinoléine fondant à 84°C.

Sous azote, on place 6,5 g d'hydrure de sodium à 80% dans l'huile avec 160 $cm^3$ de tétrahydrofuranne, on ajoute, goutte à goutte, une solution de 34,9 g de malonate de diéthyle dans 200 $cm^3$ de tétrahydrofuranne anhydre. Après 1 heure d'agitation à la température ambiante (20°C environ), on ajoute une solution de 16,2 g de bromométhyl-1 phényl-3 isoquinoléine dans 200 $cm^3$ de tétrahydrofuranne anhydre. Après 20 heures d'agitation à 20°C on ajoute 200 $cm^3$ d'eau et extrait la phase aqueuse à l'acétate d'éthyle. On sèche la phase organique et l'évapore à sec sous pression réduite. On

chromatographie le produit résiduel sur du gel de silice avec un mélange cyclohexane-acétate (9-2 en volume) comme éluant. On récupère 11,5 g de produit que l'on reprend dans 115 $cm^3$ d'acide chlorhydrique concentré et porte à ébullition 20 heures. On ajoute 200 $cm^3$ d'eau, filtre le précipité, le lave à l'eau et à l'acétone. On obtient 6,7 g d'acide phényl-3 isoquinoléine-1 propanoïque fondant à 160°C.

On agite 20 heures à 20°C, 6,7 g d'acide phényl-3 isoquinoléine-1 propanoïque et 7 $cm^3$ d'acide sulfurique concentré dans 70 $cm^3$ d'éthanol. On verse la solution dans 400 $cm^3$ d'eau et alcalinise la phase aqueuse avec une solution d'ammoniaque concentrée. On extrait avec 3 fois 100 $cm^3$ de chlorure de méthylène, sèche la phase organique et l'évapore à sec sous pression réduite. On obtient 6,3 g de phényl-3 isoquinoléine-1 propionate d'éthyle fondant à 60°C.

La méthyl-1 phényl-3 isoquinoléine peut être préparée selon S. COSZCZYNSKI, Rocznicki chem. 38(5), 893-5 (1964); Chem. Abst. 62, 16188 a (1965).

*Exemple 5*

On opère comme à l'exemple 1 à partir de 3 g de phényl-2 quinoléine-4 acétate d'éthyle et de 60 $cm^3$ de diéthylamine.

Le résidu est chromatographié sur du gel de silice avec un mélange cyclohexane-acétate d'éthyle (50-50 en volume) comme éluant. Après recristallisation dans l'acétate d'éthyle, on isole, 2,05 g de N,N-diéthyl phényl-2 quinoléine-4 acétamide fondant à 86°C.

Le phényl-2 quinoléine-3 acétate d'éthyle est préparé selon le procédé suivant:

A 40 $cm^3$ de tétrahydrofuranne sec, sous atmosphère d'azote, on ajoute 12,9 $cm^3$ de diisopropylamine. La solution est agitée puis refroidie à −70°C. On introduit alors en 15 minutes 46 $cm^3$ d'une solution 1,6 M de butyllithium dans l'hexane, puis après stabilisation de la température à −60°C on introduit en 15 minutes 8,1 g de phényl-2 lépidine dans 20 $cm^3$ de tétrahydrofuranne, puis on revient à température ambiante (20°C environ). Cette solution est ajoutée, goutte à goutte et sous azote, à une solution de 9 $cm^3$ de carbonate de diéthyle dans 50 $cm^3$ de tétrahydrofuranne préalablement refroidie à −20°C. Après la fin de l'introduction on laisse sous agitation à température ambiante (20°C environ) pendant une heure.

On ajoute ensuite, goutte à goutte, 25 $cm^3$ d'éthanol absolu puis 10 $cm^3$ d'acide acétique glacial et enfin 100 $cm^3$ d'eau. Le tétrahydrofuranne est évaporé sous pression réduite et la phase aqueuse est reprise par 200 $cm^3$ d'éther éthylique. La phase éthérée est lavée à l'eau, séchée sur sulfate de magnésium et évaporée sous pression réduite. Le résidu est repris par 100 $cm^3$ de toluène, que l'on évapore à nouveau afin déliminer l'acide acétique.

Le résidu est chromatographié sur du gel de silice avec un mélange cyclohexane-acétate d'éthyle (90-10 en volume) comme éluant. On obtient 7 g de phényl-2 quinoléine-4 acétate d'éthyle sous forme d'huile jaune. Ce produit peut être repris dans l'acétone et après addition d'une solution d'acide chlorhydrique dans l'éther éthylique, on isole 5,13 g de chlorhydrate de phényl-2 quinoléine-4 acétate d'éthyle fondant à 180°C.

La phényl-2 lépidine peut être préparée selon GOLDBERG et Coll., J. Amer. Chem. Soc., 77, 3647 (1955).

*Exemple 6*

A 25 $cm^3$ de tétrahydrofuranne sec, sous atmosphère d'azote, on ajoute 1,84 $cm^3$ de diisopropylamine. La solution est agitée, refroidie à −70°C, et on y introduit, en 10 minutes, 7 $cm^3$ d'une solution 1,6 M de butyllithium dans l'hexane.

Après stabilisation de la température à −70°C, on introduit une solution de 2,4 g de N,N-diéthyl phényl-2 quinoléine-4 acétamide préparé selon l'exemple 5 dans 10 $cm^3$ de tétrahydrofuranne. On laisse 30 minutes sous agitation à −70°C, puis on ajoute lentement une solution de 0,58 $cm^3$ d'iodure de méthyle dans 10 $cm^3$ de tétrahydrofuranne et 0,52 $cm^3$ d'hexaméthylphosphoramide.

On agite 3 heures à −70°C puis 30 minutes à −50°C. On ajoute ensuite, goutte à goutte, 3 $cm^3$ d'éthanol absolu, puis 2 $cm^3$ d'acide acétique glacial. On ramène la température à 0°C puis on ajoute 50 $cm^3$ d'eau, et extrait par 3 fois 50 $cm^3$ d'éther éthylique. La phase organique est lavée à l'eau, séchée sur sulfate de magnésium et évaporée sous pression réduite.

Après recristallisation du résidu dans l'éther isopropylique, on obtient 1,65 g de N,N-diéthyl α-méthyl phényl-2 quinoléine-4 acétamide fondant à 132°C.

*Exemple 7*

On ajoute, sous azote, 3 g de carbonyldiimidazole à une suspension de 2,67 g d'acide α-méthyl phényl-2 quinazoline-4 propanoïque dans 30 $cm^3$ de tétrahydrofuranne anhydre. Après 2 heures d'agitation, on ajoute 6 $cm^3$ de diéthylamine et agite encore 4 heures. On ajoute 150 $cm^3$ d'eau et 100 $cm^3$ d'acétate d'éthyle. On décante, extrait la phase aqueuse avec 2 fois 100 $cm^3$ d'acétate d'éthyle, sèche la phase organique sur du sulfate de magnésium et l'évapore à sec. On chromatographie le résidu sur du gel de silice, une première fois avec un mélange cyclohexane-acétate d'éthyle (1-1 en volume) puis une deuxième fois avec un mélange cyclohexane-acétate d'éthyle (8-2 en volume).

Après recristallisation dans de l'éther isopropylique on obtient 1 g de N,N-diéthyl α-méthyl phényl-2 quinazoline-4 propanamide fondant à 124°C.

L'acide α-méthyl phényl-2 quinazoline-4 propanoïque est préparé selon le procédé suivant:

On porte 3 heures à 90°C un mélange de 15 g de méthyl-4 phényl-2 quinazoline, de 13,3 g de N-bromosuccinimide et de 1,65 g de peroxyde de benzoyle dans 150 $cm^3$ de tétrachlorure de carbone. On filtre, évapore le filtrat et chromatographie le résidu sur du gel de silice avec un mélange cyclohexane-acétate d'éthyle (9-1 en volume) comme éluant. On obtient 11 g de bromométhyl-4 phényl-2 quinazoline fondant à 110°C.

A 4 g d'hydrure de sodium à 80% dans l'huile et 60 $cm^3$ de tétrahydrofuranne anhydre, sous azote, on

ajoute une solution de 23 g méthylmalonate de diéthyle dans 100 $cm^3$ de tétrahydrofuranne anhydre. Après 1 heure d'agitation, on ajoute une solution de 9,9 g de bromométhyl-4 phényl-2 quinazoline dans 100 $cm^3$ de tétrahydrofuranne anhydre et agite encore 2 heures à la température ambiante (20°C environ). On ajoute 100 $cm^3$ d'eau et extrait avec 3 fois 100 $cm^3$ d'acétate d'éthyle. On sèche la phase organique sur du sulfate de magnésium puis l'évapore à sec sous pression réduite. On reprend le résidu dans 100 $cm^3$ d'acide chlorhydrique concentré et 100 $cm^3$ d'acide acétique et porte l'ensemble à 110°C pendant 24 heures. Après refroidissement, on filtre le précipité, le lave à l'eau puis à l'éther isopropylique. Après séchage on obtient 4 g d'acide α-méthyl phényl-2 quinazoline-4 propanoïque fondant à 180°C.

La méthyl-4 phényl-2 quinazoline peut être obtenue selon W.L.F. ARMAREGO, Fused pyrimidines, quinazolines Part I p. 39, Intersciences Publishers (1967).

*Exemple 8*

On opère comme à l'exemple 7 à partir de 1,95 g d'acide phényl-2 quinazoline-4 propanoïque, de 1,36 g de carbonyldiimidazole et de 3 $cm^3$ de N-méthylaniline dans 40 $cm^3$ de tétrahydrofuranne anhydre. Après purification par chromatographie sur du gel de silice avec l'acétate d'éthyle comme éluant et recristallisation dans un mélange acétate d'éthyle-éther isopropylique (1-5 en volume), on obtient 0,63 g de N-méthyl N-phényl phényl-2 quinazoline-4 propanamide fondant à 116°C.

L'acide phényl-2 quinazoline-4 propanoïque est préparé selon le procédé suivant:

Sous azote, on place 5,4 g d'hydrure de sodium à 80% dans l'huile avec 250 $cm^3$ de tétrahydrofuranne anhydre puis, en refroidissant vers 5°C on ajoute 25,6 g de malonate de diéthyle. Lorsque le dégagement d'hydrogène a cessé, on ajoute une solution de 23,9 g de bromométhyl-4 phényl-2 quinazoline dans 100 $cm^3$ de tétrahydrofuranne anhydre. Après 1 heure d'agitation à la température ambiante (20°C environ), on ajoute 25 $cm^3$ d'acide acétique, évapore le solvant sous pression réduite et reprend le résidu dans 150 $cm^3$ d'acide chlorhydrique concentré et 150 $cm^3$ d'acide acétique. On porte l'ensemble à 120°C pendant 15 heures, évapore de nouveau, ajoute 200 $cm^3$ d'eau, 150 $cm^3$ d'éther éthylique et alcalinise à pH 11 avec une lessive d'hydroxyde de sodium. On décante la phase organique et lave la solution aqueuse avec 2 fois 100 $cm^3$ d'éther éthylique. On ajuste la phase aqueuse à pH 4 et l'extrait avec 2 fois 100 $cm^3$ d'acétate d'éthyle. On sèche la phase organique sur du sulfate de magnésium et l'évapore à sec sous pression réduite. On cristallise le résidu dans l'acétate d'éthyle. On obtient 9 g d'acide phényl-2 quinazoline-4 propanoïque fondant à 159°C.

*Exemple 9*

On opère comme à l'exemple 7 à partir de 1,95 g d'acide phényl-2 quinazoline-4 propanoïque, de 1,36 g de carbonyldiimidazole et de 1,38 $cm^3$ de pipéridine dans 40 $cm^3$ de tétrahydrofuranne. Après chromatographie sur gel de silice avec de l'acétate d'éthyle comme éluant et recristallisation dans un mélange acétate d'éthyle-éther isopropylique (1-2 en volume) on obtient 0,88 g de [(phényl-2 quinazoline-4)-3 propionyl]-1 pipéridine fondant à 115°C.

*Exemple 10*

On opère comme à l'exemple 7 à partir de 2,17 g d'acide (chloro-2 phényl)-2 quinazoline-4 propanoïque, de 1,35 g de carbonyldiimidazole et de 1,5 $cm^3$ de diéthylamine dans 20 $cm^3$ de tétrahydrofuranne.

Après chromatographie sur du gel de silice avec l'acétate d'éthyle comme éluant et cristallisation dans l'éther isopropylique, on obtient 1,5 g de N,N-diéthyl (chloro-2 phényl)-2 quinazoline-4 propanamide fondant à 90°C.

L'acide (chloro-2 phényl)-2 quinazoline-4 propanoïque est préparé selon le procédé suivant:

A une solution de 3,3 g d'(amino-2 benzoyl)-3 propionate d'éthyle et de 6,3 $cm^3$ de triéthylamine dans 35 $cm^3$ de chloroforme, on ajoute à 5°C, 3,2 $cm^3$ de chlorure d'ortho-chlorobenzoyle. On agite 20 heures à la température ambiante (20°C environ) et élimine le solvant par évaporation sous pression réduite. On reprend le résidu avec 50 $cm^3$ d'éther éthylique, filtre l'insoluble et évapore le filtrat.

Le produit résiduel est mélangé avec 15 g d'acétate d'ammonium et porté 4 heures à 110°C. Après refroidissement, on ajoute 100 $cm^3$ d'eau et extrait la phase aqueuse avec 3 fois 50 $cm^3$ de chloroforme. On élimine le solvant sous pression réduite et reprend le résidu dans 50 $cm^3$ d'éthanol et 10 $cm^3$ d'une solution concentrée d'hydroxyde de sodium. On porte 1 heure à 80°C, évapore l'éthanol, ajoute 100 $cm^3$ d'eau et lave la phase aqueuse avec 3 fois 50 $cm^3$ d'éther éthylique. On acidifie la phase aqueuse à pH 1 et l'extrait avec 5 fois 100 $cm^3$ d'éther éthylique. On sèche la phase organique sur du sulfate de magnésium, la filtre et l'évapore à sec sous pression réduite.

On obtient 4,8 g de produit que l'on recristallise dans l'éthanol, on récupère 2,2 g d'acide (chloro-2 phényl)-2 quinazoline-4 propanoïque fondant à 175 °C.

*Exemple 11*

On opère comme à l'exemple 7 à partir de 1,35 g d'acide (nitro-4 phényl)-2 quinazoline-4 propanoïque, de 0,82 g de carbonyldiimidazole et de 0,9 $cm^3$ de diéthylamine dans 20 $cm^3$ de tétrahydrofuranne anhydre. Après chromatographie sur du gel de silice avec l'acétate d'éthyle comme éluant et recristallisation dans l'acétate d'éthyle on obtient 0,35 g de N,N-diéthyl (nitro-4 phényl)-2 quinazoline-4 propanamide fondant à 168°C.

L'acide (nitro-4 phényl)-2 quinazoline-4 propanoïque est préparé selon le procédé suivant:

On porte au reflux pendant 3 heures un mélange de 3,34 g d'acide para-nitrobenzoïque et de 20 $cm^3$ de chlorure de thionyle. On élimine l'excès de chlorure de thionyle par évaporation sous pression réduite et on ajoute au produit résiduel 20 $cm^3$ de chloroforme, 5,5 $cm^3$ de triéthylamine et 2,21 g d'(amino-2 benzoyl)-3 propionate d'éthyle. On agite à la température ambiante (environ 20°C) pendant 2 heures. On élimine le solvant sous pression réduite,

reprend le résidu dans 50 $cm^3$ d'acétate d'éthyle, filtre et concentre le filtrat à sec. Le produit résiduel est mis en contact avec 20 g d'acétate d'ammonium et porté à 150°C pendant 6 heures. Après refroidissement, on ajoute 250 $cm^3$ d'eau et extrait avec 4 fois 100 $cm^3$ d'acétate d'éthyle. On lave la phase organique avec 2 fois 100 $cm^3$ d'une solution d'hydroxyde de sodium N et 50 $cm^3$ d'eau. On sèche la phase organique sur du sulfate de magnésium, la filtre et l'évapore à sec sous pression réduite. On obtient 2,8 g de produit que l'on met en contact avec 50 $cm^3$ d'éthanol et 2,5 $cm^3$ d'une solution concentrée d'hydroxyde de sodium. Après 30 minutes à la température ambiante, on élimine l'éthanol par évaporation sous pression réduite et ajoute 200 $cm^3$ d'eau. On lave la phase aqueuse avec 3 fois 50 $cm^3$ éthylique, on l'acidifie à pH 1 et filtre le précipité formé. Après lavage à l'eau, au chlorure de méthylène et séchage, on obtient 1,4 g d'acide (nitro-4 phényl)-2 quinazoline-4 propanoïque dont le spectre de RMN du proton dans le diméthylsulfoxyde deutérié a les caractéristiques suivantes:

Ar-$\underline{CH_2}$ δ : 3,7 ppm -$\underline{CH_2}$ δ : 3 ppm

H aromatiques en méta du $NO_2$ δ : 8,4 ppm
H aromatiques en ortho du $NO_2$ δ : 8,9 ppm
autres H aromatiques de 7,7 à 8,4 ppm.

*Exemple 12*

On opère comme à l'exemple 7 à partir de 1,32 g d'acide (méthyl-4 phényl)-2 quinazoline-4 propanoïque, de 0,88 g de carbonyldiimidazole et de 0,95 $cm^3$ de diéthylamine dans 20 $cm^3$ de tétrahydrofuranne anhydre.

Après chromatographie sur du gel de silice avec de l'acétate d'éthyle comme éluant et recristallisation dans de l'éthanol aqueux à 50%, on obtient 0,75 g de N,N-diéthyl (méthyl-4 phényl)-2 quinazoline-4 propanamide fondant à 80°C.

L'acide (méthyl-4 phényl)-2 quinazoline-4 propanoïque est obtenu selon le procédé suivant:

On porte 4 heures à l'ébullition un mélange de 2,72 g d'acide méthyl-4 benzoïque et de 20 $cm^3$ de chlorure de thionyle. On élimine l'excès de chlorure de thionyle par évaporation sous pression réduite et ajoute au résidu 2,21 g d'(amino-2 benzoyl)-3 propionate d'éthyle, 20 $cm^3$ de toluène et 5,5 $cm^3$ de triéthylamine. On agite 1 heure à la température ambiante (20°C environ), filtre et évapore le filtrat sous pression réduite. Au produit résiduel on ajoute 20 g d'acétate d'ammonium et on porte l'ensemble 7 heures à 110°C. Après refroidissement on ajoute 100 $cm^3$ d'eau et extrait la phase aqueuse avec 3 fois 100 $cm^3$ d'acétate d'éthyle. On évapore le solvant sous pression réduite et ajoute au résidu 20 $cm^3$ d'éthanol et 3 $cm^3$ d'une solution concentrée d'hydroxyde de sodium. On porte 1 heure à 80°C, évapore l'éthanol sous pression réduite, ajoute 100 $cm^3$ d'eau et lave la phase aqueuse par 3 fois 100 $cm^3$ d'éther éthylique. On acidifie la phase aqueuse à pH 1 et extrait le solide avec 3 fois 100 $cm^3$ d'acétate d'éthyle. On sèche la phase organique sur du sulfate de magnésium, la filtre et l'évapore à sec sous pression réduite. On obtient 3,1 g de produit que l'on recristallise dans l'éthanol absolu, on récupère 1,5 g d'acide (méthyl-4 phényl)-2 quinazoline-4 propanoïque fondant à 180°C.

*Exemple 13*

On opère comme à l'exemple 7 à partir de 1,34 g d'acide (pyridyl-2)-2 quinazoline-4 propanoïque, de 0,93 g de carbonyldiimidazole et de 1 $cm^3$ de diéthylamine dans 25 $cm^3$ de diméthylformamide. Après chromatographie sur du gel de silice avec un mélange chloroforme-méthanol (95-5 en volume), comme éluant et cristallisation dans l'acétate d'éthyle, on obtient 0,58 g de N,N-diéthyl (pyridyl-2)-2 quinazoline-4 propanamide fondant à 130°C.

L'acide (pyridyl-2)-2 quinazoline-4 propanoïque est préparé selon le procédé suivant:

Sous azote, on place 2,46 g d'acide pyridyl-2 carboxylique et 15 $cm^3$ de diméthylformamide sec. On ajoute 3, 89 g de carbonyldiimidazole, agite 20 minutes et ajoute une solution de 2,21 g d'(amino-2 benzoyl)-3 propionate d'éthyle dans 10 $cm^3$ de diméthylformamide sec. On porte le mélange 20 heures à 110°C et évapore le solvant sous pression réduite. On ajoute 50 $cm^3$ d'eau et extrait avec 4 fois 50 $cm^3$ d'éther éthylique.

On sèche la phase organique sur du sulfate de magnésium et l'évapore à sec sous pression réduite. On obtient 3,17 g de produit brut que l'on recristallise dans de l'éthanol absolu. On obtient 1,7 g de [(pyridyl-2 carboxamido)-2 benzoyl]-3 propionate d'éthyle que l'on met en contact avec 10 g d'acétate d'ammonium et 5 $cm^3$ d'acide acétique. On porte 10 heures à 110°C. Après refroidissement on ajoute 100 $cm^3$ d'eau et extrait la phase aqueuse avec 3 fois 50 $cm^3$ d'acétate d'éthyle. On évapore le solvant sous pression réduite et reprend le produit résiduel avec 20 $cm^3$ d'éthanol et 2 $cm^3$ de soude concentrée. On porte l'ensemble à 80°C pendant 1 heure, évapore l'éthanol, ajoute 25 $cm^3$ d'eau et de l'acide acétique jusqu'à pH 4. On filtre le précipité, le lave à l'eau, au chlorure de méthylène et le sèche. On obtient 0,82 g d'acide (pyridyl-2)-2 quinazoline-4 propanoïque dont le spectre de RMN du proton dans le chloroforme deutérié plus diméthyl sulfoxyde deutérié a les caractéristiques suivantes:

Ar-$\underline{CH_2}$- δ : 3,7 ppm -$\underline{CH_2}$-COOH δ : 3 ppm
$H_6$ pyridyl δ : 8,9 ppm $H_5$ pyridyl δ : 7,5 ppm

autres H aromatiques de 7,6 à 8,3 ppm.

*Exemple 14*

On opère comme à l'exemple 7 à partir de 8 g d'acide α-méthyl phényl-3 isoquinoléine-1 propanoïque, de 1,62 g de carbonyldiimidazole et de 5 $cm^3$ de diéthylamine dans 25 $cm^3$ de tétrahydrofuranne.

Après trois chromatographies sur du gel de silice avec un mélange cyclohexane-acétate d'éthyle (7-3 en volume) comme éluant et cristallisation dans l'éther isopropylique on obtient 1,3 g de N,N-diéthyl α-méthyl phényl-3 isoquinoléine-1 propanamide fondant à 57°C.

L'acide α-méthyl phényl-3 isoquinoléine-1 propanoïque est préparé selon le procédé suivant:

On place, sous azote, 2,1 g d'hydrure de sodium à 60% dans l'huile et 50 $cm^3$ de tétrahydrofuranne anhydre. On ajoute, goutte à goutte, une solution de

9,1 g de méthylmalonate de diéthyle dans 50 $cm^3$ de tétrahydrofuranne anhydre. On poursuit l'agitation 1 heure à la température ambiante (20°C environ) et ajoute une solution de 10,4 g de bromométhyl-1 phényl-3 isoquinoléine dans 100 $cm^3$ de tétrahydrofuranne anhydre. Après 20 heures de contact on ajoute 200 $cm^3$ d'eau et extrait la phase aqueuse avec 3 fois 50 $cm^3$ d'acétate d'éthyle. Après élimination des solvants sous pression réduite on récupère 19 g de produit que l'on porte à ébullition pendant 20 heures dans 70 $cm^3$ d'acide chlorhydrique concentré et 70 $cm^3$ d'acide acétique. Après refroidissement on verse le mélange dans 1000 $cm^3$ d'eau, alcalinise à pH 10 avec une lessive d'hydroxyde de sodium, lave la phase aqueuse avec 100 $cm^3$ d'acétate d'éthyle, l'acidifie avec de l'acide chlorhydrique et l'extrait avec 3 fois 200 $cm^3$ de chloroforme. On sèche la phase organique sur du sulfate de magnésium, la filtre et l'évapore à sec sous pression réduite. On obtient 7,4 g d'acide α-méthyl phényl-3 isoquinoléine-1 propanoïque fondant à 184°C.

*Exemple 15*

On chauffe au reflux, pendant 90 minutes, 3 g d'acide phényl-2 quinoléine-4 propanoïque dans 9 $cm^3$ de chlorure de thionyle. On évapore le chlorure de thionyle, reprend le résidu par 100 $cm^3$ de toluène et évapore à nouveau. On ajoute alors au résidu obtenu 60 $cm^3$ de toluène sec et introduit, goutte à goutte, sous agitation, en 20 minutes, 10 $cm^3$ de diéthylamine. On agite une heure à température ambiante (20°C environ) et reprend par 60 $cm^3$ d'eau. La phase organique est décantée. La phase aqueuse est extraite par 2 fois 30 $cm^3$ de toluène. Les phases organiques sont rassemblées, séchées sur sulfate de magnésium et évaporées sous pression réduite.

Le résidu obtenu est repris dans l'acétone et après addition d'une solution d'acide chlorhydrique dans l'éther éthylique on isole 2,3 g de chlorhydrate de N,N-diéthyl phényl-2 quinoléine-4 propanamide fondant à 126°C.

L'acide phényl-2 quinoléine-4 propanoïque peut être préparé selon J. HANNS, Ber. 58 (B), 2799 à 2805 (1925).

*Exemple 16*

On chauffe au reflux pendant une nuit 5 g d'acide phényl-2 quinoléine-4 propanoïque et 1,43 $cm^3$ de chlorure de thionyle dans 250 $cm^3$ de chloroforme. On procède ensuite comme à l'exemple 15 mais en utilisant 5,3 $cm^3$ de pipéridine.

Le résidu est agité pendant une heure avec 60 g de gel de silice dans 100 $cm^3$ d'acétate d'éthyle. La silice est éliminée par filtration et lavée avec 7 fois 10 $cm^3$ d'acétate d'éthyle. Les phases organiques sont rassemblées et évaporées sous pression réduite. Après recristallisation du résidu dans l'acétate d'éthyle on obtient 2 g de [(phényl-2 quinolyl-4)-3 propionyl]-1 pipéridine fondant à 110°C.

*Exemple 17*

On opère comme à l'exemple 16, en partant de 3 g d'acide phényl-2 quinoléine-4 propanoïque, de 2,3 $cm^3$ de chlorure de thionyle dans 150 $cm^3$ de chloroforme et de 4,4 $cm^3$ de dipropylamine. Le résidu est agité pendant une heure avec 40 g de gel de silice dans 80 $cm^3$ d'acétate d'éthyle. La silice est éliminée par filtration, lavée par 7 fois 10 $cm^3$ d'acétate d'éthyle. Les phases organiques sont rassemblées et évaporées sous pression réduite.

Le résidu obtenu est repris dans l'acétone et, après addition d'une solution d'acide chlorhydrique dans l'éther éthylique, on isole 2,41 g de chlorhydrate de N,N-dipropyl phényl-2 quinoléine-4 propanamide fondant à 130°C.

*Exemple 18*

On opère à l'exemple 16 en partant de 3 g d'acide α-méthyl phényl-2 quinoléine-4 propanoïque dans 30 $cm^3$ de chloroforme, de 0,97 $cm^3$ de chlorure de thionyle, et de 3,2 $cm^3$ de diéthylamine dans 5 $cm^3$ de chloroforme, et en réduisant à 30 minutes le temps de préparation du chlorure d'acide. Le résidu isolé est repris dans l'éthanol et après addition d'une solution d'acide chlorhydrique dans l'éther éthylique et recristallisation dans un mélange éthanol-éther éthylique (1-3 en volume) on isole 2,9 g de chlorhydrate de N,N-diéthyl α-méthyl phényl-2 quinoléine-4 propanamide fondant à 161°C.

L'acide α-méthyl phényl-2 quinoléine-4 propanoïque peut être préparé selon le procédé suivant:

1 — Préparation de la chlorométhyl-4 phényl-2 quinoléine.

A une suspension refroidie à 10°C de 45 g de phényl-2 quinoléine-4 méthanol dans 450 $cm^3$ de chloroforme, on ajoute en 45 minutes 35 $cm^3$ de chlorure de thionyle, puis on agite quatre heures à température ambiante (20°C environ). Le solvant est évaporé sous pression réduite, le résidu repris plusieurs fois au toluène et le toluène évaporé afin d'éliminer le chlorure de thionyle.

Le résidu est repris par 1000 $cm^3$ d'eau, alcalinisé à pH 9 par addition d'une solution concentré d'hydroxyde d'ammonium. La phase aqueuse est extraite par 3 fois 500 $cm^3$ d'éther éthylique, la phase éthérée est lavée par 3 fois 200 $cm^3$ d'eau, séchée sur sulfate de magnésium et évaporée sous pression réduite. Le résidu huileux est repris dans l'éther isopropylique, puis l'éther est évaporé sous pression réduite. On obtient ainsi 45,3 g de chlorométhyl-4 phényl-2 quinoléine fondant à 79°C.

Le phényl-2 quinoléine-4 méthanol peut être préparé selon ROSENMUND et ZYMALKOVSKI, B, 85, 152-159 (1952).

2 — Préparation de l'acide α-méthyl phényl-2 quinoléine-4 propionique.

A 220 $cm^3$ de tétrahydrofuranne sec, sous atmosphère d'azote, on ajoute lentement 14,3 g d'hydrure de sodium en dispersion à 60% dans l'huile. On introduit ensuite lentement en 2 heures une solution de 62,5 g de méthyl malonate de diéthyle dans 220 $cm^3$ de tétrahydrofuranne, puis en une heure une solution de 45,3 g de chlorométhyl-4 phényl-2 quinoléine dans 400 $cm^3$ de tétrahydrofuranne, on agite deux heures à température ambiante (20°C environ), puis on chauffe 1 heure 30 minutes au reflux. On ramène à température ambiante (20°C environ), ajoute goutte à goutte 22 $cm^3$ d'acide acétique glacial, puis 500 $cm^3$ d'eau. Le tétrahydrofuranne est éliminé par évaporation sous pression réduite; on dilue le milieu réactionnel par 500 $cm^3$ d'eau, et extrait par 3 fois 400 $cm^3$ d'éther éthylique. La phase éthérée est lavée à l'eau et concentrée sous pression réduite.

Le résidu obtenu est repris par 500 $cm^3$ d'une solution concentrée d'acide chlorhydrique et 500 $cm^3$ d'acide acétique glacial. On chauffe 3 heures 30 minutes au reflux. On évapore au maximum les acides sous pression réduite, reprend le résidu par 1000 $cm^3$ d'eau, alcalinise à pH 10 par addition d'une solution concentrée d'hydroxyde d'ammonium, ajoute 300 $cm^3$ d'éther éthylique et laisse 15 minures sous agitation. La phase organique est décantée, lavée par 2 fois 200 $cm^3$ d'eau. La phase aqueuse est lavée par 2 fois 300 $cm^3$ d'éther éthylique. Les phases aqueuses sont rassemblées et acidifiées sous agitation à pH 4-5 par de l'acide acétique glacial, on agite encore une heure, puis on essore le précipité, on obtient 47,6 g d'acide α-méthyl phényl-2 quinoléine-4 propanoïque fondant à 211°C.

*Exemple 19*

On opère comme à l'exemple 18 en partant de 3 g d'acide α-méthyl phényl-2 quinoléine-4 propanoïque lévogyre dans 30 $cm^3$ de chloroforme, de 0,97 $cm^3$ de chlorure de thionyle et de 3,2 $cm^3$ de diéthylamine dans 5 $cm^3$ de chloroforme.

Le résidu isolé est repris dans l'éthanol et après addition d'une solution d'acide chlorhydrique dans l'éther éthylique et d'une recristallisation dans un mélange éthanol-éther éthylique (1-3 en volume) puis 2 recristallisations dans un mélange éthanol-éther éthylique (1-2 en volume), on isole 2 g de chlorhydrate de N,N-diéthyl α-méthyl phényl-2 quinoléine-4 propanamide lévogyre fondant à 175°C.

$\alpha_D$ à 0,5% dans EtOH à 21°C = −85,5° ± 2°.

L'acide α-méthyl phényl-2 quinoléine-4 propanoïque lévogyre peut être préparé par dédoublement de l'acide α-méthyl phényl-2 quinoléine-4 propanoïque racémique en opérant de la façon suivante:

1) Préparation des N-(phényl-1 hydroxy-2 éthyl) α-méthyl phényl-2 quinoléine-4 propanamides diastéréoisomères.

On chauffe au reflux pendant une heure 42,5 g d'acide α-méthyl phényl-2 quinoléine-4 propanoïque et 13,8 $cm^3$ de chlorure de thionyle dans 450 $cm^3$ de chloroforme. Le solvant est évaporé sous pression réduite, repris par du chloroforme que l'on évapore à nouveau.

A une solution agitée de 20 g de (−) α-phénylglycinol dans 200 $cm^3$ de chloroforme, on ajoute 41 $cm^3$ de triéthylamine, puis on introduit en 1 heure 30 minutes une solution du chlorure d'acide préparé ci-dessus dans 400 $cm^3$ de chloroforme. On agite deux heures à température ambiante (20°C environ), évapore le chloroforme sous pression réduite, reprend le résidu par 500 $cm^3$ d'eau et 300 $cm^3$ d'acétate d'éthyle. La phase organique est décantée, la phase aqueuse extraite par 100 $cm^3$ d'acétate d'éthyle. Les phases organiques sont rassemblées, lavées à l'eau, séchées sur sulfate de magnésium et évaporées sous pression réduite. 10 g du résidu obtenu sont chromatographiés sur du gel de silice en utilisant comme éluant un mélange chloroforme-acétate d'éthyle (50-50 en volume).

On obtient ainsi 4,6 g de N-(phényl-1 hydroxy-2 éthyl) α-méthyl phényl-2 quinoléine-4 propanamide, forme A, fondant à 134°C qui est élué d'abord, puis 4,6 g de N-(phényl-1 hydroxy-2 éthyl) α-méthyl phényl-2 quinoléine-4 propanamide, forme B que l'on élue ensuite, et qui présente un point de fusion égal à 158°C.

2) Préparation de l'acide α-méthyl phényl-2 quinoléine-4 propanoïque lévogyre.

On chauffe au reflux pendant une heure 30 minutes 5,5 g de N-(phényl-1 hydroxy-2 éthyl) α-méthyl phényl-2 quinoléine-4 propanamide forme A dans 27 $cm^3$ d'acide acétique glacial et 27 $cm^3$ d'une solution concentrée d'acide chlorhydrique.

On évapore les acides sous pression réduite, reprend le résidu par 120 $cm^3$ d'eau, alcalinise à pH 10 au moyen d'une solution concentrée d'hydroxyde d'ammonium, lave la phase aqueuse par 250 $cm^3$ d'éther éthylique. La phase aqueuse est acidifiée à pH 5 par de l'acide acétique cristallisable et extraite par 3 fois 100 $cm^3$ d'acétate d'éthyle. La phase organique est lavée à l'eau, séchée sur sulfate de magnésium et concentrée sous pression réduite.

Après recristallisation dans l'éthanol, on obtient 2,6 g d'acide α-méthyl phényl-2 quinoléine-4 propanoïque lévogyre fondant à 185°C.

$\alpha_D$ à 0,5% dans l'acide acétique glacial = −37,7° ± 2° à 22°C.

*Exemple 20*

On opère comme à l'exemple 18 en partant de 3,4 g d'acide α-méthyl phényl-2 quinoléine-4 propanoïque dextrogyre dans 34 $cm^3$ de chloroforme, de 1,1 $cm^3$ de chlorure de thionyle et 3,6 $cm^3$ de diéthylamine dans 5 $cm^3$ de chloroforme. Le résidu isolé est repris dans l'éthanol et après addition d'une solution d'acide chlorhydrique dans l'éther éthylique, une recristallisation dans un mélange éthanol-éther éthylique (1-3 en volume), puis une autre dans un mélange éthanol-éther éthylique (1-2 en volume), on isole 2,55 g de chlorohydrate de N,N-diéthyl α-méthyl phényl-2 quinoléine-4 propanamide dextrogyre fondant à 175°C.

$\alpha_D$ à 0,5% dans EtOH à 21°C = +81,6° ± 2°.

L'acide α-méthyl phényl-2 quinoléine-4 propanoïque dextrogyre peut être préparé comme son énantiomère lévogyre décrit à l'exemple 19 en partant de 6,6 g de N-(phényl-1 hydroxy-2 éthyl) α-méthyl phényl-2 quinoléine-4 propanamide forme B préparé selon l'exemple 19, de 33 $cm^3$ d'une solution concentrée d'acide chlorhydrique, et de 33 $cm^3$ d'acide acétique.

Le résidu obtenu est recristallisé dans l'éthanol.

On obtient 3,9 g d'acide α-méthyl phényl-2 quinoléine-4 propanoïque dextrogyre fondant à 186°C.

$\alpha_D$ à 0,5% dans l'acide acétique glacial à 24°C = +33,3° ± 2°.

*Exemple 21*

On opère comme à l'exemple 18 en partant de 2,03 g d'acide α-méthyl phényl-2 quinoléine-4 propanoïque dans 20 $cm^3$ de chloroforme, de 0,67 $cm^3$ de chlorure de thionyle et de 7 $cm^3$ d'une solution 3 M de diméthylamine dans du toluène. Le résidu est repris dans l'éthanol et après addition d'une solution d'acide chlorhydrique dans l'éther éthylique et recristallisation dans un mélange éthanol-éther éthylique (1-2 en volume) on isole 1,4 g de chlorhydrate de N,N-diméthyl α-méthyl phényl-2 quinoléine-4 propanamide fondant à 160°C.

*Exemple 22* (pour référence)

A une solution de 3,7 g d'acide phényl-2 quinoléine-4 butanoïque dans 60 $cm^3$ de chloroforme on ajoute 1,02 $cm^3$ de chlorure de thionyle et on chauffe 15 minutes au reflux. Les solvants sont évaporés sous pression réduite. Le résidu obtenu est dissous dans 40 $cm^3$ de chloroforme. A cette solution on ajoute lentement en 20 minutes 6 $cm^3$ de diéthylamine. On agite 3 heures à température ambiante (20°C environ) et reprend par 40 $cm^3$ d'eau. La phase organique est décantée, lavée par 40 $cm^3$ d'eau, séchée sur sulfate de magnésium et évaporée sous pression réduite.

Le résidu est chromatographié sur du gel de silice en utilisant comme éluant un mélange cyclohexane-acétate d'éthyle (50-50 en volume). On obtient ainsi 3,3 g de N,N-diéthyl phényl-2 quinoléine-4 butanamide que l'on transforme au sein de l'acétone en monochlorhydrate. Celui-ci fond à 130°C.

L'acide phényl-2 quinoléine-4 butanoïque peut être préparé de la façon suivante:

1 — Préparation du γ-oxo phényl-2 quinoléine-4 butanoate d'éthyle.

A 500 $cm^3$ de tétrahydrofuranne sec, sous atmosphère d'azote, on ajoute lentement 84 $cm^3$ d'une suspension à 20% d'hydrure de potassium dans l'huile. On introduit ensuite sous agitation 47 g de phényl-2 quinoléine-4 carboxylate-4 d'éthyle, puis on ajoute lentement, en 2 heures, à température ambiante (environ 20°C) une solution de 24,4 g succinate d'éthyle dans 250 $cm^3$ de tétrahydrofuranne. On ajoute ensuite 80 $cm^3$ d'éthanol, puis 800 $cm^3$ d'eau. Le tétrahydrofuranne est éliminé par évaporation, la phase aqueuse est extraite par 2 fois 200 $cm^3$ d'éther éthylique, puis acidifiée à pH 4,5 par addition d'acide acétique glacial, et enfin extraite par 3 fois 200 $cm^3$ d'éther éthylique. La phase éthérée est lavée à l'eau, séchée sur sulfate de magnésium et évaporée sous pression réduite.

Le résidu est repris par 600 $cm^3$ d'une solution 6 N d'acide chlorhydrique et on chauffe au reflux pendant 6 heures. Après concentration sous pression réduite on obtient 57,4 g d'un mélange contenant l'acide phényl-2 quinoléinecarboxylique-4 et l'acide γ-oxo phényl-2 quinoléine-4 butanoïque.

Ce mélange est repris par 600 $cm^3$ d'éthanol absolu et 60 $cm^3$ d'acide sulfurique concentré et chauffée une nuit au reflux. L'éthanol est éliminé par évaporation sous pression réduite, le résidu est coulé sur 600 $cm^3$ d'eau glacée et 220 $cm^3$ d'une solution concentrée d'hydroxyde d'ammonium. Cette phase aqueuse est extrait par 3 fois 700 $cm^3$ d'éther éthylique, la phase organique est lavée à l'eau séchée sur sulfate de magnésium et évaporée sous pression réduite. Le résidu est chromatographié sur du gel de silice en utilisant comme éluant un mélange cyclohexane-acétate d'éthyle (90-10 en volume). On obtient ainsi 24,4 g de γ-oxo phényl-2 quinoléine-4 butanoate d'éthyle fondant à 66°C.

2 — Préparation de l'acide phényl-2 quinoléine-4 butanoïque.

On chauffe à 150°C, pendant 15 minutes, un mélange de 5 g de γ-oxo phényl-2 quinoléine-4 butanoate d'éthyle et de 2,25 $cm^3$ d'hydrate d'hydrazine à 98% dans 15 $cm^3$ de diéthylèneglycol. On refroidit à 120°C, puis on introduit, en 15 minutes, 2,5 g d'hydroxyde de potassium en pastilles et on chauffe à 150°C pendant une heure 30 minutes.

On dilue le mélange réactionnel avec 300 $cm^3$ d'eau, extrait par 3 fois 80 $cm^3$ d'éther éthylique. La phase éthérée est lavée par 10 $cm^3$ d'eau, les phases aqueuses sont rassemblées, acidifiées à pH 4,5 par addition d'acide acétique, et extraites par 3 fois 100 $cm^3$ d'éther éthylique. La phase éthérée est lavée à l'eau, séchée sur sulfate de magnésium et évaporée sous pression réduite. On obtient 3,7 g d'acide phényl-2 quinoléine-4 butanoïque fondant à 125°C.

*Exemple 23*

On opère comme à l'exemple 22 en partant de 1,7 g de chlorhydrate de l'acide α-éthyl phényl-2 quinoléine-4 butanoïque dans 35 $cm^3$ de chloroforme, de 0,38 $cm^3$ de chlorure de thionyle et de 2,2 $cm^3$ de diéthylamine. Après chromatographie du résidu sur du gel de silice en utilisant comme éluant un mélange cyclohexane-acétate d'éthyle (80-20 en volume) et cristallisation dans l'éther de pétrole 40-60°, on obtient 1,1 g de N,N-diéthyl α-éthyl phényl-2 quinoléine-4 propanamide à 81°C.

L'acide α-éthyl phényl-2 quinoléine-4 butanoïque est préparé par hydrolyse de l'ester éthylique correspondant au moyen d'une solution 6 N d'acide chlorhydrique.

L'α-éthyl phényl-2 quinoléine-4 butanoate d'éthyle est préparé de la façon suivante:

A 30 $cm^3$ de tétrahydrofuranne sec, placés sous atmosphère d'azote, on ajoute 2,96 $cm^3$ de diisopropylamine. La solution est agitée et refroidie à −70°C. On introduit alors en 15 minutes 11,3 $cm^3$ d'une solution 1,6 M de butyllithium dans l'hexane, puis après stabilisation de la température à −70°C, on introduit en 5 minutes une solution de 3,8 g de phényl-2 quinoléine-4 butanoate d'éthyle dans 30 $cm^3$ de tétrahydrofuranne. On agite 30 minutes à −70°C puis on introduit en 5 minutes 1,15 $cm^3$ d'iodure d'éthyle et 0,8 $cm^3$ d'hexaméthylphosphoramide dans 20 $cm^3$ de tétrahydrofuranne. On agite 7 heures à −70°C. On ajoute 7 $cm^3$ d'éthanol, puis 2 $cm^3$ d'acide acétique. On laisse remonter la température jusqu'à la température ambiante (20°C

environ), dilue le milieu réactionnel par 300 $cm^3$ d'eau et extrait par 2 fois 100 $cm^3$ d'éther éthylique. La phase éthérée est lavée à l'eau, séchée sur sulfate de magnésium et évaporée sous pression réduite. Le résidu est chromatographié sur du gel de silice au moyen d'un mélange cyclohexane-acétate d'éthyle (90-10 en volume). On isole ainsi 1,8 g d'α-éthyl phényl-2 quinoléine-4 butanoate d'éthyle fondant à 76°C.

Le phényl-2 quinoléine-4 butanoate d'éthyle peut être préparé par estérification de l'acide correspondant dont la préparation est décrite à l'exemple 22 au moyen d'éthanol en présence d'acide sulfurique.

*Exemple 24*

On opère comme à l'exemple 22 en partant de 4 g d'acide phényl-2 quinoléine-5 pentanoïque dans 80 $cm^3$ de chloroforme, de 1,05 $cm^3$ de chlorure de thionyle et de 6 $cm^3$ de diéthylamine.

Le résidu obtenu est chromatographié sur du gel de silice en utilisant comme éluant le mélange cyclohexane-acétate d'éthyle (50-50 en volume). On isole ainsi 2,7 g de N,N-diéthyl phényl-2 quinoléine-4 pentanamide sous forme d'huile jaune. Le spectre de RMN du proton dans le chloroforme deutérié présente les caractéristiques suivantes:

Ar-$\underline{CH_2}$-$CH_2$ δ : 3,13 ppm
-$\underline{CH_2}$-CON δ : 2,32 ppm
$H_3$ δ : 7,68 ppm
Ar-$CH_2$-$\underline{CH_2}$-$\underline{CH_2}$-$CH_2$-CON δ : 1,84 ppm.

L'acide phényl-2 quinoléine-5 pentanoïque peut être préparé selon le procédé suivant:

1) Préparation de l'acide δ-oxophényl-2 quinoléine--4 pentanoïque.

A une suspension d'éthylate de sodium (préparée à partir de 0,92 g de sodium et de 2,34 $cm^3$ d'éthanol) dans 200 $cm^3$ de toluène, on ajoute à 100°C en 5 minutes une solution de γ-oxo phényl-2 quinoléine-4 propionate d'éthyle (préparé selon F. GES, Chim. Ind. Basel. DRP 462 136) dans 50 $cm^3$ de toluène. On chauffe une heure à 100°C puis on ajoute 4,4 $cm^3$ d'acrylate d'éthyle; on chauffe 3 heures 30 minutes à 100°C, ajoute 2,2 $cm^3$ d'acétate d'éthyle et agite à 100°C une nuit. On ramène à température ambiante (environ 20°C), ajoute 35 $cm^3$ d'acide acétique, puis 150 $cm^3$ d'eau et 50 $cm^3$ d'éther éthylique.

La phase organique est décantée, lavée à l'eau, séchée sur sulfate de magnésium et évaporée sous pression réduite. Le résidu est chromatographié sur du gel de silice en utilisant le mélange cyclohexane-acétate d'éthyle (90-10 en volume) comme éluant. On obtient ainsi 6,6 g de β-cétoester, que l'on reprend par 130 $cm^3$ d'une solution 6 N d'acide chlorhydrique et que l'on chauffe au reflux une heure. On refroidit à 0°C, alcalinise à pH 9 par addition de 60 $cm^3$ d'une solution concentrée d'hydroxyde d'ammonium, puis acidifie à pH 4 par addition d'acide acétique glacial.

La phase aqueuse est extraite par 3 fois 150 $cm^3$ d'éther éthylique, la phase organique est séchée sur sulfate de magnésium et évaporée sous pression réduite. On obtient ainsi 4,7 g d'acide δ-oxo phényl-2 quinoléine-4 pentanoïque fondant à 136°C.

2) Préparation de l'acide phényl-2 quinoléine-4 pentanoïque.

On opère de la même façon que pour préparer l'acide phényl-2 quinoléine-4 butanoïque, décrit dans l'exemple 22 mais en partant de 4,6 g d'acide δ-oxo phényl-2 quinoléine-4 pentanoïque, de 2,15 g d'hydrate d'hydrazine à 98%, de 2,4 g d'hydroxyde de potassium en pastilles et de 14 $cm^3$ de diéthylèneglycol. On isole ainsi 4 g d'acide phényl-2 quinoléine-4 pentanoïque dont le spectre RMN du proton dans le chloroforme deutérié présente les caractéristiques suivantes:

Ar-$\underline{CH_2}$ δ : 3,16 ppm
Ar-$CH_2$-$\underline{CH_2}$-$\underline{CH_2}$-$CH_2$-COOH δ : 1,83 ppm
$\underline{CH_2}$-COOH δ : 2,37 ppm $H_3$ δ : 7,59 ppm.

*Exemple 25*

A une suspension de 2,5 g d'acide phényl-2 quinoléine-4 propanoïque dans 100 $cm^3$ de chloroforme, on ajoute 3,78 $cm^3$ de triéthylamine puis, sous azote, et après avoir refroidi à 10°C, 1,24 g de chloroformiate d'éthyle. On agite ensuite pendant 40 minutes à température ambiante (20°C environ), puis on introduit par petites quantités 10,5 g de chlorhydrate de N-méthyl butanamine-2. On agite 20 heures à température ambiante (20°C environ). Après évaporation du solvant sous pression réduite, on reprend le résidu dans l'acétate d'éthyl, lave la phase organique par une solution aqueuse saturée en carbonate de sodium, le sèche sur sulfate de magnésium. Le résidu obtenu après évaporation du solvant sous pression réduite est chromatographié sous pression sur du gel de silice, une première fois avec un mélange cyclohexane-toluène-diéthylamine (80-15-5 en volume) une seconde fois avec un mélange cyclohexane-toluène-diéthylamine (90-7,5-2,5 en volume) une troisième fois avec un mélange hexane-acétate d'éthyle (50-50 en volume). Le résidu obtenu est repris dans l'acétone et après addition d'une solution d'acide chlorhydrique dans l'éther éthylique, on isole le produit sous forme de chlorhydrate brut. Ce dernier est recristallisé dans un mélange isopropanol-éther isopropylique. Après retour à la base à l'aide d'hydroxyde de sodium 2 N, extraction à l'acétate d'éthyle et évaporation sous pression réduite, on isole 0,45 g de N-méthyl N-méthyl-1 propyl phényl-2 quinoléine-4 propanamide sous forme d'huile dont le spectre RMN du proton dans le chloroforme deutérié présente les caractéristiques suivantes:

Ar-$\underline{CH_2}$-$CH_2$ δ : 3,52 ppm
N-$\underline{CH_3}$ δ : 2,65-2,78 ppm
-$\underline{CH_2}$-CO-N δ : 2,78 ppm $H_3$ δ : 7,73 ppm

*Exemple 26*

On porte au reflux pendant 20 heures, 1,71 g d'acide métatrifluorométhylbenzoïque et 20 $cm^3$ de chlorure de thionyle. On évapore l'excès de chlorure de thionyle sous pression réduite et reprend le résidu dans 20 $cm^3$ de chloroforme, 1,9 $cm^3$ de triéthyla-

mine et ajoute 1,11 g de N,N-diéthyl (amino-2 benzoyl)-3 propanamide. Après 1 heure de contact à la température ambiante (20°C environ), on évapore le chloroforme sous pression réduite, reprend le produit résiduel dans l'éther éthylique, filtre et évapore le filtrat à sec. On mélange le résidu avec 5 $cm^3$ d'acide acétique et 5 g d'acétate d'ammonium et porte l'ensemble à 100°C pendant 6 heures. On évapore l'acide acétique sous pression réduite, ajoute 50 $cm^3$ d'eau et extrait avec 3 fois 50 $cm^3$ d'acétate d'éthyle. On sèche la phase organique sur du sulfate de magnésium et l'évapore à sec sous pression réduite. On chromatographie le produit brut sur du gel de silice avec un mélange cyclohexane-acétate d'éthyle (1-1 en volume) comme éluant. On récupère 1,2 g de produit que l'on cristallise dans l'éther isopropylique. On obtient 0,75 g de N,N-diéthyl (trifluoro méthyl-3 phényl)-2 quinazoline-4 propanamide fondant à 115°C.

Le N,N-diéthyl (amino-2 benzoyl)-3 propanamide est préparé selon le procédé suivant:

On place sous léger courant d'air, 0,5 g de chlorure cuivreux et 1,22 $cm^3$ de pyridine dans 20 $cm^3$ de chlorure de méthylène. Après 15 minutes on ajoute, goutte à goutte, une solution de N,N-diéthyl indole-3 propanamide dans 30 $cm^3$ de chlorure de méthylène et on laisse 20 heures sous agitation et sous courant d'air.

On lave la solution organique avec 50 $cm^3$ d'une solution saturée de chlorure d'ammonium, sèche la phase organique sur sulfate de magnésium et l'évapore à sec sous pression réduite. On reprend le résidu dans 40 $cm^3$ d'éthanol et 10 $cm^3$ d'une solution 5 N d'hydroxyde de sodium. On agite à la température ambiante (environ 20°C) 1 heure, ajoute 100 $cm^3$ d'eau et extrait la phase aqueuse avec 3 fois 100 $cm^3$ d'éther éthylique. On évapore l'éther sous pression réduite et chromatographie le produit résiduel sur du gel de silice avec l'acétate d'éthyle comme éluant. On obtient 2,22 g de N,N-diéthyl (amino-2 benzoyl(-3 propanamide sous forme d'huile dont le spectre RMN du proton dans le chloroforme deutérié a les caractéristiques suivantes:

| | |
|---|---|
| $CH_2$-$\underline{CH_3}$ | δ : 1,1 et 1,3 ppm |
| N-$\underline{CH_2}$- | δ : 3,3 ppm |
| Ar-CO-$\underline{CH_2}$ | δ : 3,3 ppm |
| -$\underline{CH_2}$-CO- | δ : 2,8 ppm |
| Aromatiques $H_6$ | δ : 7,9 ppm |
| $H_4$ | δ : 7,3 ppm |
| $H_3$ et $H_5$ | δ : 6,7 ppm. |

Le N,N-diéthyl indole-3 propanamide peut être préparé selon H.E. JOHNSON et Coll.,J. Org. Chem., 28, 2030 (1963).

*Exemple 27*

On opère comme à l'exemple 26 à partir de 1,15 g d'acide thiénoïque-2 et de 10 $cm^3$ de chlorure de thionyle, puis de 1,9 $cm^3$ de triéthylamine, de 1,11 g de N,N-diéthyl (amino-2-benzoyl)-3 propanamide et de 20 $cm^3$ de chloroforme et enfin de 5 g d'acétate d'ammonium et 5 $cm^3$ d'acide acétique. Après chromatographie sur du gel de silice avec un mélange cyclohexane-acétate d'éthyle (1-1 en volume) comme éluant et cristallisation dans l'éther isopropylique, on obtient 0,83 g de N,N-diéthyl thiényl-2 quinazoline-4 propanamide fondant à 106°C.

*Exemple 28*

On opère comme à l'exemple 26 à partir de 1,72 g de N,N-diéthyl (amino-2 bromo-5 benzoyl)-3 propanamide, de 1,98 g de chlorure de benzoyle, de 4,2 $cm^3$ de triéthylamine et de 20 $cm^3$ de chloroforme puis de 5 g d'acétate d'ammonium et 5 $cm^3$ d'acide acétique.

Après chromatographie sur du gel de silice avec un mélange cyclohexane-acétate d'éthyle (1-1 en volume) comme éluant et recristallisation dans l'acétate d'éthyle, on obtient 1,32 g de N,N-diéthyl bromo-6 phényl-2 quinazoline-4 propanamide fondant à 146°C.

Le N,N-diéthyl (amino-2 bromo-5 benzoyl)-3 propanamide est préparé selon le procédé suivant:

On agite 72 heures à la température ambiante (20°C environ) 13,7 g de bromo-5 indole et 15 $cm^3$ d'acide acrylique dans 10 $cm^3$ d'acide acétique et 10 $cm^3$ d'anhydride acétique.

On élimine les solvants sous pression réduite et chromatographie le résidu sur du gel de silice d'abord avec le chloroforme puis avec un mélange chloroforme-méthanol (98-2 en volume) comme éluant. On récupère 13 g de produit que l'on reprend, sous azote, dans 60 $cm^3$ de tétrahydrofuranne sec. On ajoute 12,5 g de carbonyldiimidazole et on agite pendant 1 heure. On ajoute ensuite 15 $cm^3$ de diéthylamine et laisse en contact 20 heures. On élimine le solvant sous pression réduite, ajoute 100 $cm^3$ d'eau et extrait avec 2 fois 100 $cm^3$ d'acétate d'éthyle puis 2 fois 100 $cm^3$ d'éther éthylique. On sèche la phase organique sur du sulfate de magnésium, la filtre et l'évapore à sec sous pression réduite. On cristallise le résidu dans l'éther éthylique, on obtient 7,8 g de N,N-diéthyl bromo-5 indole-3 propanamide fondant à 128°C.

Sous courant d'air, on place 0,5 g de chlorure cuivreux, 1,22 $cm^3$ de pyridine et 30 $cm^3$ de chlorure de méthylène. On ajoute ensuite une solution de 7,5 g de N,N-diéthyl bromo-5 indole-3 propanamide dans 60 $cm^3$ de chlorure de méthylène, agite 24 heures, ajoute de nouveau 0,5 g de chlorure cuivreux et 1,5 $cm^3$ de pyridine et agite encore 24 heures. On ajoute 10 g de silice, filtre et évapore le filtrat à sec sous pression réduite.

Le produit résiduel est repris dans 50 $cm^3$ d'éthanol, 5 $cm^3$ d'eau et 5 $cm^3$ d'une lessive concentrée d'hydroxyde de sodium. On porte à 80°C 15 minutes, élimine l'éthanol par évaporation sous pression réduite, ajoute 50 $cm^3$ d'eau et extrait la phase aqueuse avec 3 fois 100 $cm^3$ d'éther éthylique. On sèche la phase organique sur du sulfate de magnésium et l'évapore à sec sous pression réduite. On chromatographie le produit résiduel sur du gel de silice avec un mélange cyclohexane-acétate d'éthyle (1-1 en volume) puis de l'acétate d'éthyle. On récupère 1,93 g de N,N-diéthyl (amino-2 bromo-5 benzoyl)-3 propanamide fondant à 120°C.

*Exemple 29*

On opère comme à l'exemple 26 à partir de 1,5 g de N,N-diéthyl (amino-2 méthoxy-5 benzoyl)-3 propanamide, de 1,9 g de chlorure de benzoyle, de 2,7 g de triéthylamine et 20 $cm^3$ de chloroforme; puis de 5 g d'acétate d'ammonium et 5 $cm^3$ d'acide acétique.

Après chromatographie sur du gel de silice avec l'acétate d'éthyle comme éluant et cristallisation dans l'éther isopropylique on obtient 0,64 g de N,N-diéthyl méthoxy-6 phényl-2 quinazoline-4 propanamide fondant à 144°C.

Le N,N-diéthyl (amino-2 méthoxy-5 benzoyl)-3 propanamide est préparé selon le procédé suivant:

On agite 72 heures à la température ambiante (20°C environ), 11,2 g de méthoxy-5 indole et 16,4 g d'acide arcylique dans 11 $cm^3$ d'acide acétique et 11 $cm^3$ d'anhydride acétique.

On évapore le solvant sous pression réduite, reprend le résidu dans 200 $cm^3$ d'une solution normale d'hydroxyde de sodium, agite, filtre et lave le produit insoluble avec 100 $cm^3$ d'une solution normale d'hydroxyde de sodium. On acidifie la phase aqueuse à pH 1 et extrait l'insoluble avec 3 fois 100 $cm^3$ de chloroforme. On sèche la phase organique sur du sulfate de magnésium, la filtre et l'évapore à sec sous pression réduite. On chromatographie le produit résiduel sur du gel de silice avec un mélange chloroforme-méthanol (95-5 en volume) comme éluant, on obtient 4,6 g d'acide méthoxy-5 indole-3 propanoïque fondant à 128°C.

On place sous azote 8 g d'acide méthoxy-5 indole-3 propanoïque dans 40 $cm^3$ de tétrahydrofuranne sec. On ajoute, peu à peu, 7,1 g de carbonyldiimidazole et on laisse sous agigation 1 heure à la température ambiante (20°C environ). On ajoute 13,4 g de diéthylamine et on poursuit l'agitation pendant 2 heures. On ajoute 200 $cm^3$ d'eau et extrait avec 3 fois 100 $cm^3$ d'acétate d'éthyle. On sèche la phase organique sur du sulfate de magnésium, la filtre et l'évapore à sec sous pression réduite. On cristallise le produit résiduel avec de l'éther éthylique, on obtient 8 g de N,N-diéthyl méthoxy-5 indole-3 propanamide fondant à 80°C.

On place sous courant d'air 1 g de chlorure cuivreux, 2,5 $cm^3$ de pyridine et 60 $cm^3$ de chlorure de méthylène. Après 15 minutes on ajoute 7,7 g de N,N-diéthyl méthoxy-5 indole-3 propanamide en solution dans 60 $cm^3$ de chlorure de méthylène et on agite 20 heures à la température ambiante (20°C environ). On ajoute 10 g de silice, agite, filtre et évapore le filtrat sous pression réduite.

On chromatographie le produit résiduel sur du gel de silice avec un mélange cyclohexane-acétate d'éthyle (1-1 en volume) puis avec de l'acétate d'éthyle comme éluant. On récupère 3,8 g de produit que l'on traite à 80°C pendant 30 minutes par 25 $cm^3$ d'éthanol, 2,5 $cm^3$ d'eau et 2,5 $cm^3$ d'une solution concentrée d'hydroxyde de sodium. On évapore l'éthanol sous pression réduite, ajoute 100 $cm^3$ d'eau et extrait avec 3 fois 50 $cm^3$ d'éther éthylique. On sèche la phase organique sur du sulfate de magnésium, la filtre et l'évapore à sec sous pression réduite. On obtient 1,5 g de N,N-diéthyl (amino-2 méthoxy-5 benzoyl)-3 propanamide sous forme d'huile que l'on utilise telle que.

*Exemple 30*

On opère comme à l'exemple 26 à partir de 2,62 g de N,N-diéthyl (amino-2 méthyl-3 benzoyl)-3 propanamide, de 2,5 $cm^3$ de chlorure de benzoyle, de 6,6 $cm^3$ de triéthylamine dans 30 $cm^3$ de chloroforme, puis de 10 g d'acétate d'ammonium et 10 $cm^3$ d'acide acétique.

Après chromatographie sur du gel de silice avec un mélange cyclohexane-acétate d'éthyle (1-1 en volume) comme éluant et cristallisation dans l'éther isopropylique, on obtient 1,95 g de N,N-diéthyl méthyl-8 phényl-2 quinazoline-4 propanamide fondant à 80°C.

Le N,N-diéthyl (amino-2 méthyl-3 benzoyl)-3 propanamide est préparé selon le procédé suivant:

On agite 48 heures à la température ambiante (20°C environ) 26,2 g de méthyl-7 indole avec 30 $cm^3$ d'acide acrylique dans 10 $cm^3$ d'anhydride acétique et 20 $cm^3$ d'acide acétique. On évapore les solvants et chromatographie le produit résiduel sur du gel de silice avec le chloroforme comme éluant.

On récupère 14 g d'acide méthyl-7 indole-3 propanoïque fondant à 110°C.

Sous azote, on place 17 g d'acide méthyl-7 indole-3 propanoïque et 100 $cm^3$ de tétrahydrofuranne sec. On ajoute peu à peu 17,2 g de carbonyldiimidazole, laisse sous agitation 1 heure, ajoute 45 $cm^3$ de diéthylamine et agite encore 2 heures. On ajoute 300 $cm^3$ d'eau et extrait avec 300 $cm^3$ puis 2 fois 100 $cm^3$ d'acétate d'éthyle. On sèche la phase organique sur du sulfate de magnésium, la filtre et l'évapore à sec sous pression réduite. On reprend le produit résiduel dans 100 $cm^3$ d'éther éthylique, après filtration et séchage, on récupère 18,8 g de N,N-diéthyl méthyl-7 indole-3 propanamide que l'on redissout dans 350 $cm^3$ de méthanol. On ajoute une solution de 47 g de métapériodate de sodium dans 250 $cm^3$ d'eau et agite pendant 24 heures. On filtre, dilue le filtrat avec 250 $cm^3$ d'eau et l'extrait avec 3 fois 250 $cm^3$ de chlorure de méthylène. On sèche la phase organique sur du sulfate de magnésium, la filtre et l'évapore à sec sous pression réduite. On reprend le produit résiduel dans 200 $cm^3$ d'éthanol et 20 $cm^3$ d'acide chlorhydrique concentré. On laisse en contact 72 heures à la température ambiante (20°C environ), évapore l'éthanol sous pression réduite, ajoute 200 $cm^3$ d'eau, lave la phase aqueuse à l'éther éthylique, l'alcalinise à pH 11 et l'extrait avec 2 fois 100 $cm^3$ d'éther éthylique. On sèche la phase organique sur du sulfate de magnésium, la filtre et l'évapore à sec sous pression réduite.

On recristallise le solide résiduel dans un mélange de 20 $cm^3$ d'acétate d'éthyle et 50 $cm^3$ d'éther isopropylique. On obtient 6,7 g de N,N-diéthyl (amino-2 méthyl-3 benzoyl)-3 propanamide fondant à 104°C.

*Exemple 31*

A une suspension agitée de 2,21 g de phényl-2 quinolinol-4 dans 15 $cm^3$ de diméthylformamide, on ajoute sous azote 2,1 $cm^3$ de triéthylamine puis 2,03 g de chlorure de N,N-diéthylcarbamoyle. On chauffe 13 heures à 70°C, puis coule le mélange réactionnel dans 400 $cm^3$ d'eau glacée et 400 $cm^3$ de chloroforme. L'insoluble est éliminé par filtration,

la phase organique lavée à l'eau, séchée sur sulfate de magnésium et évaporée sous pression réduite. Le résidu est transformé au sein de l'éther éthylique en un chlorhydrate brut (1,6 g) qui est repris par 200 $cm^3$ d'eau et 5 $cm^3$ d'une solution concentrée d'hydroxyde de sodium; cette phase alcaline est extraite par 2 fois 100 $cm^3$ d'éther éthylique. La phase éthérée est lavée par 6 fois 50 $cm^3$ d'eau, séchée sur sulfate de magnésium et évaporée sous pression réduite.

Le résidu obtenu est repris dans l'éther éthylique et après addition d'une solution d'acide chlorhydrique dans l'éther éthylique on isole 1,28 g de chlorhydrate de N,N-diéthylcarbamate de phényl-2 quinolyle-4 fondant à 120°C.

Le phényl-2 quinolinol-4 peut être préparé selon C. HAUSER et A. REYNOLDS, J.A.C.S., 70, 2402-04 (1984).

*Exemple 32*

On procède comme à l'exemple 31 mais partant de 4,42 g de phényl-2 quinolinol-4, de 4,3 $cm^3$ de triéthylamine, de 4,42 g de (chlorocarbonyl)-1 pipéridine dans 30 $cm^3$ de diméthylformamide. Le résidu obtenu est purifié comme à l'exemple 31, repris dans l'éther éthylique et après addition d'une solution d'acide chlorhydrique dans l'éther éthylique, on isole 2,56 g de chlorhydrate de pipéridine-1 carboxylate de phényl-2 quinolyle-4 fondant à 120°C.

*Exemple 33*

On opère comme à l'exemple 31 mais en partant de 2,47 g de phényl-4 quinolinol-2, de 2,33 $cm^3$ de triéthylamine, de 2,47 g de (chlorocarbonyl)-1 pipéridine dans 30 $cm^3$ de diméthylformamide. Le résidu obtenu est purifié par chromatographie sur du gel de silice au moyen d'un éluant cyclohexane-acétate d'éthyle (80-20 en volume). On isole ainsi 0,51 g de pipéridine-1 carboxylate de phényl-4 quinolyle-2 fondant à 85°C.

Le phényl-4 quinolinol-2 peut être préparé selon C. HAUSER et A. REYNOLDS, J.A.C.S., 70, 2402-4 (1948).

*Exemple 34*

On opère comme à l'exemple 31, mais en partant de 3,54 g de phényl-4 quinolinol-2, de 2,2 g de chlorure de N,N-diéthylcarbamoyle, de 3,36 $cm^3$ de triéthylamine dans 60 $cm^3$ de diméthylformamide.

Le résidu obtenu est purifié par chromatographie sur du gel de silice en utilisant comme éluant un mélange cyclohexane-acétate d'éthyle (80-20 en volume). Le résidu obtenu (1,2 g) est recristallisé dans un mélange acétone-eau (1-3 en volume). On obtient ainsi 0,83 g de N,N-diéthylcarbamate de phényl-4 quinolyle-2 fondant à 64°C.

*Exemple 35*

A 10 g de phényl-4 quinazolinol-2 dans 100 $cm^3$ de diméthylformamide à 60°C, on ajoute en 4 fois et à 24 heures d'intervalle 38 $cm^3$ de triéthylamine et 34,5 $cm^3$ de chlorure de diéthylcarbamoyle. Après refroidissement on ajoute 300 $cm^3$ d'eau et extrait la phase aqueuse avec 3 fois 200 $cm^3$ de chlorure de méthylène. Après évaporation du solvant on reprend le solide résiduel dans 100 $cm^3$ d'éther éthylique, filtre et évapore de nouveau. On dissout le résidu dans 100 $cm^3$ d'acétate d'éthyle à chaud, filtre et évapore le solvant.

On obtient 4 g de produit brut que l'on recristallise dans l'isopropanol. On récupère 2,5 g de diéthylcarbamate de phényl-4 quinazolinyle-2 fondant à 112°C.

Le phényl-4 quinazolinol-2 peut être préparé selon GABRIEL, Ber. 29, 1310 (1896).

*Exemple 36*

On opère comme à l'exemple 35 à partir de 10 g de phényl-2 quinazolinol-4, de 50 $cm^3$ de triéthylamine et de 45,5 $cm^3$ de chlorure de diéthylcarbamoyle dans 100 $cm^3$ de diméthylformamide.

La purification du produit brut est effectuée par plusieurs chromatographies sur du gel de silice d'abord avec un mélange cyclohexane-acétate d'éthyle (7-3 en volume) comme éluant, puis chloroforme-méthanol (95-5 en volume) et efin cyclohexane-diéthylamine (95-5 en volume). Une recristallisation finale dans l'éther de pétrole fournit 1,2 g de diéthylcarbamate de phényl-2 quinazolinyle-4 fondant à 54°C.

Le phényl-2 quinazolinol-4 peut être préparé selon H. STEPHEN, J. Chem. Soc., 4420 (1956).

*Exemple 37*

On opère comme à l'exemple 35 à partir de 3,5 g de phényl-1 isoquinolinol-3, de 7,2 g de triéthylamine et de 9,63 g de chlorure de diéthylcarbamoyle dans 35 $cm^3$ de diméthylformamide. On purifie le produit par chromatographie sur du gel de silice avec un mélange cyclohexane-acétate d'éthyle (4-1 en volume) comme éluant. Par cristallisation au moyen d'éther isopropylique, on obtient 0,8 g de diéthylcarbamate de phényl-1 isoquinolyle-3, fondant à 76°C.

Le phényl-1 isoquinolinol-2 peut être préparé selon D.W. JONES, J. Chem. Soc., 1729 (1969).

*Exemple 38*

On opère comme à l'exemple 35 à partir de 4,1 g de phényl-3 isoquinolinol-1, de 7,5 g de triéthylamine et de 10 g de chlorure de diéthylcarbamoyle dans 40 $cm^3$ de diméthylformamide.

Après chromatographie sur du gel de silice avec un mélange cyclohexane-acétate d'éthyle (4-1 en volume) comme éluant et cristallisation dans l'éther isopropylique, on obtient 2,7 g de diéthylcarbamate de phényl-3 isoquinolyle-1 fondant à 81°C.

Le phényl-3 isoquinolinol-1 peut être préparé selon GABRIEL, Chem. Ber. 18, 3471 (1885).

*Exemple 39*

On opère comme à l'exemple 35 à partir de 4,7 g de (méthyl-4 phényl)-3 isoquinolinol-1, de 8,1 g de triéthylamine et de 10,8 g de chlorure de diéthylcarbamoyle dans 50 $cm^3$ de diméthylformamide. Après chromatographie sur du gel de silice avec un mélange cyclohexane-acétate d'éthyle (4-1 en volume) comme éluant et recristallisation dans l'éther éthylique on obtient 0,6 g de diéthylcarbamate de (méthyl-4 phényl)-3 isoquinolyle-1 fondant à 98°C.

Le (méthyl-4 phényl)-3 isoquinolinol-1 peut être

préparé selon A. KASAHARA et Coll., Chem. Ind. (LONDON), (16), 666, (1980).

*Exemple 40*

On opère comme à l'exemple 35 à partir de 10 g de (méthoxy-4 phényl)-3 isoquinolinol-1, de 16 g de triéthylamine et de 21,7 g de chlorure de diéthylcarbamoyle dans 100 $cm^3$ de diméthylformamide. Après chromatographie sur du gel de silice la première fois avec un mélange cyclohexane-acétate d'éthyle (1-1 en volume) la deuxième fois avec un mélange toluène-méthanol (98-2 en volume) comme éluant et cristallisation dans l'éthanol aqueux à 80%, on obtient 0,7 g de diéthylcarbamate de (méthoxy-4 phényl)-3 isoquinolyle-1 fondant à 84°C.

Le (méthoxy-4 phényl)-3 isoquinolinol-1 peut être préparé selon W.T. BOYCE et Coll., J. Org. Chem., 31, 3807 (1966).

*Exemple 41*

On chauffe 8 heures 30 minutes au reflux 2,93 g d'acide [(phényl-2 quinolyl-4) oxy]-2 propionique et 2,2 $cm^3$ de chlorure de thionyle dans 75 $cm^3$ de chloroforme. On élimine les solvants sous pression réduite, et le résidu obtenu est ajouté, en 15 minutes, à une solution préalablement refroidie à 5°C de 10,3 $cm^3$ de diéthylamine dans 100 $cm^3$ de chlorure de méthylène. On agite 1 heure 30 minutes à 5-10°C, lave la phase organique par 6 fois 100 $cm^3$ d'eau, sèche sur sulfate de magnésium et évapore sous pression réduite. Le résidu obtenu est chromatographié sur du gel de silice en utilisant comme éluant le mélange cyclohexane-acétate d'éthyle (50-50 en volume).

Le résidu obtenu est recristallisé dans l'éther isopropylique. On isole ainsi 2,09 g de N,N-diéthyl [(phényl-2 quinolyl-4) oxy]-2 propanamide fondant à 130°C.

L'acide [(phényl-2 quinolyl-4) oxy]-2 propionique peut être préparé par saponification de l'ester éthylique correspondant au moyen d'une solution normale d'hydroxyde de sodium. Il présente un point de fusion égal à 124°C.

Le [(phényl-2 quinolyl-4) oxy]-2 propionate d'éthyle peut être préparé de la façon suivante:

A une suspension agitée de 6,63 g de phényl-2 quinolinol-4 et de 8,3 g de carbonate de potassium dans 200 $cm^3$ de méthyléthylcétone, on ajoute, goutte à goutte, 4,3 $cm^3$ de bromo-2 propionate d'éthyle. On chauffe trois heures au reflux. On ramène à température ambiante (20°C environ), essore l'insoluble et élimine les solvants sous pression réduite. Le résidu est repris par 100 $cm^3$ d'éther de pétrole 40-70°C, et essoré. On isole ainsi 9,2 g de [(phényl-2 quinolyl-4) oxy]-2 propionate d'éthyle fondant à 80°C.

*Exemple 42*

On chauffe 3 heures au reflux 1,1 g d'acide [(phényl-2 quinolyl-4) oxy]-4 butanoïque et 0,53 $cm^3$ de chlorure de thionyle dans 20 $cm^3$ de chloroforme. On élimine les solvants sous pression réduite. On ajoute alors au résidu 20 $cm^3$ de chloroforme et introduit, goutte à goutte, sous agitation, 2,2 $cm^3$ de diéthylamine. On agite 2 heures à température ambiante (20°C environ), évapore le solvant sous pression réduite et reprend le résidu par 50 $cm^3$ d'eau et 50 $cm^3$ d'acétate d'éthyle. La phase aqueuse est extraite par 3 fois 20 $cm^3$ d'acétate d'éthyle, les phases organiques sont rassemblées, lavées par 50 $cm^3$ d'eau, séchées sur sulfate de magnésium et évaporées sous pression réduite. Le résidu obtenu est chromatographié sur du gel de silice en utilisant comme éluant le mélange cyclohexane-acétate d'éthyle (50-50 en volume). Le résidu obtenu est repris dans l'acétone et après addition d'une solution d'acide chlorhydrique dans l'éther éthylique, on isole 0,47 g de chlorhydrate de N,N-diéthyl [(phényl-2 quinolyl-4) oxy]-4 butanamide fondant à 140°C.

L'acide [(phényl-2 quinolyl-4) oxy]-4 butanoïque peut être préparé de la manière suivante:

1 — Préparation du [(phényl-2 quinolyl-4) oxy]-4 butanol.

A 30 $cm^3$ de méthanol, on ajoute sous agitation 0,713 g de sodium en petits morceaux. On agite 10 minutes à température ambiante (20°C environ) puis on ajoute 24,8 $cm^3$ de butanediol-1,4. On chauffe ensuite afin d'éliminer le méthanol par distillation jusqu'à 160°C et on maintient cette température 15 minutes. On refroidit à 100°C, et sous atmosphère d'azote, on ajoute 30 mg de cuivre en poudre puis sous agitation, et par petites quantités 7 g de bromo-4 phényl-2 quinoléine en 30 minutes. On chauffe ensuite 2 heures à 160°C. Après refroidissement à température ambiante (20°C environ), en reprend le milieu réactionnel à l'eau, élimine le cuivre par filtration et extrait le filtrat au chloroforme. La phase organique est lavée à l'eau, séchée sur sulfate de magnésium et évaporée sous pression réduite. Après deux recristallisations dans un mélange cyclohexane-acétate d'éthyle (70-30 en volume), on isole 2,83 g de [(phényl-2 quinolyl-4) oxy-4 butano] fondant à 112°C. La bromo-4 phényl-2 quinoléine peut être préparée selon KASLOW et al., J. Amer. Chem. Soc., 72, 1723 (1950).

2 — Préparation de l'acide [(phényl-2 quinolyl-4) oxy]-4 butanoïque.

A une solution refroidie à 5°C de 2,25 g d'anhydride chromique dans 5 $cm^3$ d'acide acétique à 90%, on ajoute lentement une solution de 2,20 g de [(phényl-2 quinolyl-4) oxy]-4 butanol dans 10 $cm^3$ d'acide acétique glacial. On laisse la température remonter jusqu'à 20°C environ puis agite une heure à cette température. On ajoute ensuite 50 $cm^3$ d'éthanol, évapore les solvants sous pression réduite, reprend le résidu par 100 $cm^3$ d'eau, essore l'insoluble et le lave plusieurs fois à l'eau. Cet insoluble est repris par 15 $cm^3$ d'une acétate normale d'hydroxyde de sodium et est chauffé au reflux pendant deux heures. L'insoluble est éliminé par filtration et le filtrat acidifié par addition d'acide acétique glacial. Le précipité obtenu est essoré. On obtient 0,740 g d'acide [(phényl-2 quinolyl-4) oxy]-4 butanoïque fondant à 264°C.

*Exemple 43*

On opère comme à l'exemple 42 mais en partant de

1,25 g d'acide [(phényl-2 quinolyl-4) oxy]-3 propanoïque, de 1,25 $cm^3$ de chlorure de thionyle dans 100 $cm^3$ de chloroforme et de 2,66 $cm^3$ de diéthylamine. Après chromatographie du résidu sur du gel de silice au moyen de l'éluant cyclohexane-acétate d'éthyle (50-50 en volume), on isole 0,43 g de N,N-diéthyl [(phényl-2 quinolyl-4) oxy]-3 propanamide fondant à 94°C.

L'acide [(phényl-2 quinolyl-4) oxy]-3 propanoïque qui présente un point de fusion égal à 172°C peut être préparé par oxydation du [(phényl-2 quinolyl-4) oxy]-propanol, lui même préparé par réaction de la bromo-4 quinoléine sur le monosel de sodium de propanediol-1,3, en suivant les procédés décrits dans l'exemple 42 pour la préparation de l'acide [(phényl-2 quinolyl-4) oxy]-4 butanoïque.

*Exemple 44*

A une suspension agitée de 4,42 g de phényl-4 quinolinol-2, de 5,52 g de carbonate de potassium et 0,95 g d'iodure cuivreux dans 200 $cm^3$ de butanone-2, on ajoute 3,3 g de N,N-diéthylchloro-2 acétamide. On chauffe 21 heures au reflux. On ramène à température ambiante (20°C environ), filtre l'insoluble sur fritté, et évapore à sec sous pression réduite.

Le résidu obtenu est chromatographié sur du gel de silice en utilisant un mélange cyclohexane-acétate d'éthyle (50-50 en volume) comme éluant. On isole ainsi 3,9 g de N,N-diéthyl (phényl-4 quinolyl-2) oxy acétamide qui présente après recristallisation dans l'éther isopropylique un point de fusion égal à 100 °C.

*Exemple 45*

On porte 22 heures à ébullition un mélange de 4,45 g de phényl-2 quinazolinol-4, de 3,3 g de N,N-diéthyl chloroacétamide, de 4,25 g de carbonate de sodium et de 1 g d'iodure cuivreux dans 70 $cm^3$ de butanone-2. On évapore le solvant sous pression réduite, ajoute 100 $cm^3$ d'eau, alcalinise avec une solution d'ammoniaque et extrait la phase aqueuse avec 3 fois 100 $cm^3$ de chlorure de méthylène. On sèche la phase organique sur du sulfate de magnésium et l'évapore à sec sous pression réduite. On chromatographie le résidu sur du gel de silice avec un mélange cyclohexane-acétate d'éthyle (1-1 en volume) comme éluant. Après cristallisation dans l'éther isopropylique on obtient 4 g de N,N-diéthyl (phényl-2 quinazolinyl-4 )oxy acétamide fondant à 113°C.

*Exemple 46*

On opère comme à l'exemple 45 à partir de 4,45 g de phényl-4 quinazolinol-2, de 6,6 g de N,N-diéthyl chloroacétamide, de 4,25 g de carbonate de sodium et de 1 g de d'iodure cuivreux dans 70 $cm^3$ de butanone-2.

Après chromatographie sur du gel de silice avec l'acétate d'éthyle comme éluant et recristallisation dans l'éther isopropylique, on obtient 1 g de N,N-diéthyl (phényl-2 quinazolinyl-2) oxy acétamide fondant à 88°C.

*Exemple 47*

On opère comme à l'exemple 45 à partir de 5,1 g de phényl-3 isoquinolinol-1, de 3,8 g de N,N-diéthyl chloroacétamide, de 4,8 g de carbonate de sodium et de 1,15 g d'iodure cuivreux dans 100 $cm^3$ de butanone-2. Après 2 chromatographies sur du gel de silice avec du chloroforme comme éluant et cristallisation du produit dans l'éther de pétrole on obtient 0,5 g de N,N-diéthyl (phényl-3 isoquinolyl-1) oxyacétamide fondant à 102°C.

*Exemple 48*

On opère comme à l'exemple 45 à partir de 4,4 g de phényl-1 isoquinolinol-3, de 3,3 g de N,N-diéthyl chloro-2 acétamide, de 4,25 g de carbonate de sodium et de 1 g d'iodure cuivreux dans 90 $cm^3$ de butanone-2. Après chromatographie sur du gel de silice avec un mélange chloroforme-méthanol (98-2 en volume) comme éluant et cristallisation du produit dans l'éther isopropylique on obtient 2,6 g de N,N-diéthyl (phényl-1 isoquinolyl-3) oxyacétamide fondant à 105°C.

*Exemple 49*

On opère comme à l'exemple 7 à partir de 0,78 g d'acide [(phényl-3 isoquinolyl)-1 oxy]-2 propanoïque, de 0,52 g carbonyldiimidazole et de 0,82 $cm^3$ de diéthylamine dans 20 $cm^3$ de tétrahydrofuranne anhydre.

Après chromatographie sur du gel de silice avec un mélange cyclohexane-acétate d'éthyle (1-1 en volume) comme éluant et recristallisation dans l'éther isopropylique on obtient 0,53 g de N,N-diéthyl [(phényl-3 isoquinolyl-1) oxy]-2 propanamide fondant à 117°C.

L'acide [(phényl-3 isoquinolyl)-1 oxy]-2 propanoïque est préparé selon le procédé suivant:

On porte 96 heures à ébullition un mélange de 2,42 g de phényl-3 isoquinolinol-1, de 8 $cm^3$ d'α-bromopropionate d'éthyle et de 5,4 g de carbonate de sodium dans 20 $cm^3$ de butanone-2.

On évapore le solvant sous pression réduite. On ajoute 50 $cm^3$ d'eau et extrait la phase aqueuse par 3 fois 50 $cm^3$ de chloroforme. On sèche la phase organique sur du sulfate de magnésium et évapore le solvant sous pression réduite. On chromatographie le résidu sur du gel de silice avec un mélange cyclohexane-acétate d'éthyle (1-1 en volume) comme éluant. Les premières fractions fournissent, après évaporation 1 g de produit que l'on traite pendant 1 heure, par 2 $cm^3$ d'une lessive d'hydroxyde de sodium et 20 $cm^3$ d'éthanol à la température ambiante (environ 20°C). On élimine l'éthanol sous pression réduite, ajoute 50 $cm^3$ d'eau, lave la phase aqueuse avec 50 $cm^3$. d'éther éthylique, acidifie la phase aqueuse avec de l'acide chlorhydrique concentré et extrait avec 3 fois 50 $cm^3$ de chloroforme. On sèche la phase organique sur du sulfate de magnésium, la filtre et l'évapore à sec sous pression réduite. On obtient 0,78 g d'acide [(phényl-3 isoquinolyl-1) oxy]-2 propanoïque.

*Exemple 50*

On porte, 9 heures, à ébullition un mélange de 4,45 g de phényl-2 quinazolinol-4, de 4,2 g de N,N-diéthyl bromo-2 propanamide et de 4,24 g de carbonate de sodium dans 50 $cm^3$ de butanone-2. Après refroidissement, on filtre et évapore le filtrat à sec sous pression réduite. On extrait le résidu avec 50 $cm^3$ d'un mélange cyclohexane-acétate d'éthyle (7-3 en volume) à 60°C, filtre l'insoluble et évapore le filtrat à sec sous pression réduite. On chromatographie le solide résiduel sur du gel de silice avec un mélange cyclohexane-acétate d'éthyle (7-3 en volume) comme éluant. Après trois recristallisations dans un mélange éthanol-eau (2-1 en volume) on obtient 0,96 g de N,N-diéthyl [(phényl-2 quinazolinyl-4) oxy]-2 propanamide fondant à 160°C.

*Exemple 51*

La séparation des 2 énantiomères est effectuée à partir de 1 g de N,N-diéthyl [(phényl-2 quinazolinyl)-4 oxy]-2 propanamide obtenue selon l'exemple 50.

La chromatographie «HPLC» est faite sur une colonne chirale DNBPG covalente (dinitrobenzoyle phénylglycine) de J.T. BAKER, en utilisant comme éluant un mélange hexane-alcoolisopropylique (95-5 en volume). Le débit est de 0,7 $cm^3$/min et le nombre d'injections a été de cent.

On récupère, après évaporation des meilleures fractions et cristallisation dans de l'éthanol aqueux à 80%, 200 mg de N,N-diéthyl [(phényl-2 quinazolinyl-4) oxy]-2 propanamide dextrogyre, fondant à 160°C, $\alpha_D^{24}$ = +48,7° (0,5% dans HCl N) et 200 mg de N,N-diéthyl [(phényl-2 quinazolinyl-4) oxy]-2 propanamide lévogyre, fondant à 160°C, $\alpha_D^{24}$ = −52,0° (0,5% dans HCl N).

*Exemple 52*

A une suspension de 2,4 g d'acide [(phényl-2 quinolyl-4) thio] acétique dans 24 $cm^3$ de chloroforme on ajoute 0,65 $cm^3$ de chlorure de thionyle et on agite deux heures à température ambiante (20°C environ). On refroidit le mélange réactionnel à 5°C, puis on ajoute sous agitation 2,5 $cm^3$ de diéthylamine. On agite ensuite une heure à température ambiante (20°C environ), évapore les solvants sous pression réduite, reprend le résidu par 50 $cm^3$ d'eau et 50 $cm^3$ d'acétate d'éthyle; la phase organique est décantée, la phase aqueuse extraite par 50 $cm^3$ d'acétate d'éthyle. Les phases organiques sont rassemblées, lavées par 2 fois 20 $cm^3$ d'eau, puis par 20 $cm^3$ d'une solution normale d'hydroxyde d'ammonium, et enfin par 2 fois 10 $cm^3$ d'eau, séchées sur sulfate de magnésium et évaporées sous pression réduite. Le résidu obtenu est chromatographié sur du gel de silice avec un mélange cyclohexane-acétate d'éthyle (50-50 en volume). L'huile obtenue (1 g) est reprise dans l'acétone et après addition d'une solution d'acide chlorhydrique dans l'éther éthylique et recristallisation du chlorhydrate brut dans l'éthanol, on obtient 0,68 g de chlorhydrate de N,N-diéthyl [(phényl-2 quinolyl-4) thio] acétamide fondant à 150°C.

L'acide [(phényl-2 quinolyl-4) thio] acétique est préparé de la façon suivante:

On chauffe au reflux pendant 4 heures 3 g de chloro-4 phényl-2 quinoléine et 1,38 g d'acide thioglycolique dans 40 $cm^3$ de pyridine. Après évaporation de la pyridine sous pression réduite on reprend le résidu par 125 $cm^3$ d'eau et 40 $cm^3$ d'une solution aqueuse normale d'hydroxyde de sodium. La phase aqueuse est lavée par 2 fois 50 $cm^3$ d'éther éthylique, acidifiée à pH 5 par de l'acide acétique glacial et extraite par 3 fois 50 $cm^3$ d'éther éthylique. La phase éthérée est lavée à l'eau, séchée sur sulfate de magnésium et évaporée sous pression réduite. On obtient ainsi 3,2 g d'acide [(phényl-2 quinolyl-4) thio] acétique fondant à 138°C.

La chloro-4 phényl-2 quinoléine peut être préparée selon BANGDIWALA et al., J. Indian. Chem. Soc., 31, 43 (1954).

*Exemple 53*

A une suspension de 4,8 g d'acide [phényl-2 quinolyl-4) thio]-2 propionique dans 48 $cm^3$ de chloroforme, on ajoute 1,24 $cm^3$ de chlorure de thionyle. On agite quinze minutes à température ambiante (20°C environ) puis on chauffe deux heures trente minutes au reflux. On ramène la température à 5°C puis on introduit en 20 minutes 4,9 $cm^3$ de diéthylamine. On agite 30 minutes à 5°C, puis 30 minutes à température ambiante (20°C environ). On évapore les solvants sous pression réduite, reprend le résidu par 50 $cm^3$ d'eau et 80 $cm^3$ d'éther éthylique. La phase organique est décantée et la phase aqueuse est extraite par 2 fois 50 $cm^3$ d'éther éthylique. Les phases organiques sont rassemblées, lavées par 2 fois 20 $cm^3$ d'eau, 1 fois 20 $cm^3$ d'une solution 0,1 N d'hydroxyde de sodium, et 2 fois 10 $cm^3$ d'eau, séchées sur sulfate de sodium et évaporées sous pression réduite. Le résidu obtenu est repris dans l'éthanol et après addition d'une solution d'acide chlorhydrique dans l'éther éthylique, et deux recristallisations dans l'éthanol on isole 1,95 g de chlorhydrate de N,N-diéthyl [(phényl-1 quinolyl-4) thio]-2 propanamide fondant à 155°C.

L'acide [(phényl-2 quinolyl-4) thio]-2 propionique peut être préparé de façon analogue à l'acide [(phényl-2 quinolyl-4) thio]-2 acétique décrit dans l'exemple 52 en partant de 4,8 g de chloro-4 phényl-2 quinoléine, 2,54 g d'acide thiolactique et 50 $cm^3$ de pyridine sur tamis. On obtient ainsi 5,3 g d'acide [(phényl-2 quinolyl-4) thio]-2 propionique dont le spectre RMN du proton, dans le chloroforme deutérié a les caractéristiques suivantes:

| | |
|---|---|
| S-<u>CH</u>($CH_3$)-COOH | δ : 4,70 ppm |
| S-CH(<u>$CH_3$</u>)-COOH | δ : 1,65 ppm |
| $H_3$ | δ : 8,14 ppm |
| $H_{5,8}$ | δ : 8,22 ppm |

*Exemple 54*

On opère comme à l'exemple 52 mais en partant de 3,4 g d'acide [(phényl-1 quinolyl-4) thio]propionique, de 0,88 $cm^3$ de chlorure de thionyle, et de 3,5 $cm^3$ de diéthylamine.

Le résidu obtenu est repris dans l'éthanol et après addition d'une solution d'acide chlorhydrique dans l'éther éthylique et recristallisation dans l'éthanol, on isole 2,3 g de chlorhydrate de N,N-diéthyl [(phényl-2 quinolyl-4) thio]-3 propanamide fondant à 145°C.

L'acide [(phényl-2 quinolyl-4) thio]-3 propionique est préparé de la façon suivante:

On chauffe au reflux pendant 11 heures 4,8 g de chloro-4 phényl-2 quinoléine et 2,54 g d'acide mercapto-3 propionique dans 50 $cm^3$ de pyridine. Après un traitement analogue à celui qui est décrit à l'exemple 52 pour l'acide [(phényl-2 quinolyl-4) thio]-2

acétique on isole 3,4 g d'acide [(phényl-2 quinolyl-4) thio]-3 propionique dont le spectre RMN du proton dans le chloroforme deutérié a les caractéristiques suivantes:

| | | | |
|---|---|---|---|
| S-$\underline{CH_2}$ | δ : 3,42 ppm | $H_3$ | δ : 7,62 ppm |
| -$\underline{CH_2}$-COOH | δ : 2,80 ppm | $H_{5,8}$ | δ : 8 ppm |

*Exemple 55*

A une solution de 3,3 g de N,N-diéthyl bromo-2 acétamide dans 120 $cm^3$ de méthyléthylcétone on ajoute 4,65 g de carbonate de potassium puis 4 g de phényl-4 quinoléine-2 thiol. On agite 10 minutes à température ambiante (20°C environ) puis on chauffe 15 minutes au reflux. Les sels minéraux sont éliminés par filtration et lavés avec 3 fois 10 $cm^3$ de méthyléthylcétone. Les filtrats sont rassemblés, évaporés sous pression réduite; le résidu est repris par 200 $cm^3$ d'éther éthylique, la phase organique lavée par 2 fois 50 $cm^3$ d'eau, séchée sur sulfate de magnésium et évaporée sous pression réduite.

Le résidu obtenu est repris dans l'acétone et après addition d'une solution d'acide chlorhydrique dans l'éther éthylique et recristallisation dans l'éthanol, on obtient 2,6 g de chlorhydrate de N,N-diéthyl [(phényl-4 quinolyl-2) thio] acétamide.

Le phényl-4 quinoléine-2-thiol peut être préparé par chauffage de la chloro-2 phényl-4 quinoléine avec l'hydrogénosulfure de sodium à 170°C dans un mélange glycol-dowtherm.

*Exemple 56*

A une solution de 4,7 g de N,N-diéthyl [(phényl-2 quinolyl-4) thio]-2 propanamide preparé selon l'exemple 54 dans 76 $cm^3$ d'éthanol, on ajoute en 10 minutes une solution de 5,5 g de périodate de sodium dans 38 $cm^3$ d'eau préalablement tiédie à 50°C. On agite 3 jours à température ambiante (20°C environ). L'éthanol est évaporé sous pression réduite, le résidu repris par 100 $cm^3$ d'eau et 200 $cm^3$ d'acétate d'éthyle. La phase organique est décantée, lavée par 2 fois 50 $cm^3$ d'eau, séchée sur sulfate de magnésium et évaporée sous pression réduite.

Le résidu est chromatographié sur du gel de silice au moyen du mélange chloroforme-acétate d'éthyle (70-30 en volume). Après recristallisation du résidu obtenu dans l'acétate d'éthyle, on obtient 2,8 g de N,N-diéthyl [(phényl-2 quinolyl-4) sulfinyl]-3 propanamide fondant à 130°C.

*Exemple 57*

A une solution de 1,8 g de N,N-diéthyl [(phényl-2 quinolyl-4) sulfinyl]-3 propanamide préparé selon l'exemple 56 dans 18 $cm^3$ d'acide acétique glacial, on ajoute 0,31 $cm^3$ d'acide méthanesulfonique et 0,53 $cm^3$ d'une solution à 30% d'eau oxygénée. On chauffe trois heures à 80°C sous agitation. On refroidit à température ambiante (20°C environ), ajoute 1 $cm^3$ d'une solution concentrée d'hydroxyde d'ammonium, puis 180 $cm^3$ d'eau et extrait au moyen de 3 fois 100 $cm^3$ d'éther éthylique. La phase éthérée est lavée par 50 $cm^3$ d'une solution 0,1 N d'hydroxyde d'ammonium, puis par 50 $cm^3$ d'eau et séchée sur sulfate de magnésium. Après avoir détruit les éventuels péroxydes par addition de sulfite de sodium, et concentré les solvants sous pression réduite, on obtient un résidu (1,8 g) qui est chromatographié sur du gel de silice en utilisant comme éluant le mélange cyclohexane-acétate d'éthyle (50-50 en volume). Après recristallisation du résidu dans l'acétate d'éthyle, on obtient 0,82 g de N,N-diéthyl [(phényl- quinolyl-4) sulfonyl]-3 propanamide fondant à 100°C.

*Exemple 58*

On chauffe à 140°C pendant 3 heures 3 g de bromo-phényl-2 quinoléine, 1,97 g de phénol et 1,66 g de N,N-diéthyl éthylamino-2 acétamide, ajoute ensuite à nouveau 1,66 g de N,N-diéthyl éthylamino-2 acétamide, et chauffe encore 2 heures à 140°C. Le milieu réactionel est repris par un mélange eau-éther éthylique, puis on acidifie par addition d'une solution décinormale d'acide chlorhydrique; la phase aqueuse acide est lavée à l'éther éthylique, alcalinisée à pH par addition d'une solution concentrée d'hydroxyde d'ammonium et extraite par 3 fois 100 $cm^3$ d'éther éthylique. La phase organique est séchée sur sulfate de magnésium et évaporée sous pression réduite. On obtient ainsi un résidu (2,5 g) qui est chromatographié sur du gel de silice en utilisant comme éluant un mélange cyclohexane-acétate d'éthyle (50-50 en volume). Le résidu est repris dans l'acétone et après addition d'une solution d'acide chlorhydrique dans l'éther éthylique, on obtient 2,45 g de chlorhydrate de N,N-diéthyl [éthyl(phényl-2 quinolyl-4) amino] acétamide fondant à 150°C.

Le N,N-diéthyl éthylamino-2 acétamide peut être préparé selon A.G. GEIGY, demandes de brevet allemand 2 411 662, 2 447 587 (1944).

*Exemple 59*

On opére comme à l'exemple 58 mais en partant de 7,7 g de bromo-4 phényl-2 quinoléine, de 4,7 g de N,N-diéthyl éthylamino-3 propanamide et de 5 g de phénol. Après une première chromatographie du résidu sur du gel de silice au moyen d'un mélange cyclohexane-acétate d'éthyle (50-50 en volume), puis une seconde chromatographie avec un mélange cyclohexane-acétate d'éthyle (70-30 en volume), on isole un résidu (1,4 g) que l'on transforme en dichlorhydrate au sein de l'acétone par addition d'une solution d'acide chlorhydrique dans l'éther éthylique. Après recristallisation dans un mélange acétone-éther éthylique, on obtient 0,79 g de dichlorhydrate de N,N-diéthyl [éthyl(phényl-2 quinolyl-4) amino]-3 propanamide fondant à 154°C.

La N,N-diéthyl éthylamino-3 propanamide peut être préparée par analogie avec des méthodes connues de la littérature, MARINI, BETTOLO, CAVALLA, Gazetta Chim. Ital., 84, 896, 906 (1954).

*Exemple 60*

A une suspension agitée de 5,2 g de phényl-3 naphtol-1 et de 6,6 g de carbonate de potassium dans 150 $cm^3$ de butanone-2 on ajoute 4,65 g de N,N-diéthyl bromoacétamide.

On chauffe au reflux 2 heures, refroidit à température ambiante (20°C environ), élimine l'insoluble par filtration et évapore le solvant sous pression réduite.

Après chromatographie du résidu sur du gel de silice en utilisant comme éluants successifs le chlorure de méthylène puis un mélange chlorure de méthylène-acétate d'éthyle (98-2 en volume) et cristallisation du résidu obtenu dans l'éther isopropylique, on isole 2 g de N,N-diéthyl (phényl-3 naphtyl-1) oxyacétamide fondant à 82°C.

Le phényl-3 naphtol-1 peut être préparé selon C. KIPPING et al., J. Prakt. Chem., 315(5), 887-94 (1973).

*Exemple 61*

On chauffe au reflux pendant 4 heures, 3,15 g de phényl-3 naphtol-1, 1,95 g de chlorure de N,N-diéthyl carbamoyle, 1,45 g de triéthylamine et 0,035 g de diméthylamino-4 pyridine dans 37 $cm^3$ de tétrahydrofuranne. On ajoute 0,4 g de chlorure de N,N-diéthylcarbamoyle, chauffe encore 2 heures puis refroidit à température ambiante (20°C environ), élimine le précipité par filtration et évapore le filtrat sous pression réduite. Le résidu obtenu est chromatographié sur du gel de silice en utilisant comme éluant un mélange cyclohexane-acétate d'éthyle (80-20 en volume). Après recristallisation du résidu dans un mélange éther isopropylique-éther de pétrole (1-1 en volume), on isole 2,94 g de N,N-diéthylcarbamate de phényl-3 naphtyle-1 fondant à 74°C.

*Exemple 62*

On opère comme à l'exemple 60, à partir de 3 g de phényl-3 naphtol-1, de 3,75 g de carbonate de potassium et de 2,83 g de N,N-diéthyl bromo-2 propanamide dans 90 $cm^3$ de butanone-2, en ajoutant 2,2 g d'iodure de potassium au bout de 8 heures de reflux puis 0,56 g de N,N-diéthyl bromo-2 propanamide au bout de 10 heures de reflux.

Après recristallisation du résidu dans l'éther isopropylique, on obtient 3,7 g de N,N-diéthyl (phényl-3 naphtyl-1) oxy-2 propanamide fondant à 109°C.

*Exemple 63*

On chauffe 3 heures au reflux 2,5 g d'acide (phényl-2 quinolyl-4) oxy-2 propanoïque et de 1,85 $cm^3$ de chlorure de thionyle dans 50 $cm^3$ de chloroforme. On éliminé le solvant sous pression réduite et le résidu obtenu est mis en suspension dans 40 $cm^3$ de chloroforme.

A cette suspension, on ajoute lentement, sous agitation, 1,05 $cm^3$ de diallylamine et 2,63 $cm^3$ de triéthylamine dans 75 $cm^3$ de chloroforme, en maintenant la température à 10°C. On agite 15 minutes à température ambiante (environ 20°C), évapore le solvant sous pression réduite et reprend le résidu par 50 $cm^3$ d'acétate d'éthyle et 20 $cm^3$ d'eau. La phase organique est décantée, lavée par 2 fois 10 $cm^3$ d'eau puis par 10 $cm^3$ d'une solution normale d'acide chlorhydrique et enfin par 10 $cm^3$ d'eau.

Après évaporation du solvant sous pression réduite, le résidu est dissous dans l'acétonitrile et cristallise par addition lente d'éther isopropylique. Après recristallisation du résidu obtenu dans l'éther isopropylique en présence de noir acticarbone, on obtient 1,9 g de N,N-dipropen-2 yl [(phényl-2 quinolyl-4) oxy]-2 propanamide fondant à 110°C.

*Exemple 64*

On procède comme à l'exemple 18 en partant de 1,8 g d'acide α-méthyl phényl-2 quinoléine-4 propanoïque dans 20 $cm^3$ de chloroforme, de 0,58 $cm^3$ de chlorure de thionyle, de 0,81 $cm^3$ de N-méthyl cyclohexylamine et de 1,88 $cm^3$ de triéthylamine dans 20 $cm^3$ de chloroforme.

Le résidu obtenu est recristallisé dans l'acétate d'éthyle. On isole ainsi 1,45 g de N-cyclohexyl N-méthyl α-méthylphényl-2 quinoléine-4 propanamide fondant à 160°C.

*Exemple 65*

On procède comme à l'exemple 18 en partant de 1,8 g d'acide α-méthyl phényl-2 quinoléine-4 propanoïque dans 20 $cm^3$ de chloroforme, de 0,58 $cm^3$ de chlorure de thionyle, de 1,44 $cm^3$ de dihexylamine et de 1,88 $cm^3$ de triéthylamine dans 20 $cm^3$ de chloroforme.

Le résidu obtenu est chromatographié sur du gel de silice en utilisant comme éluant un mélange cyclohexane-acétate d'éthyle (80-20 en volume). Le résidu est repris dans l'acétone et après addition d'une solution d'acide chlorhydrique dans l'éther éthylique et recristallisation du chlorhydrate brut obtenu dans un mélange éthanol-éther éthylique (1-3 en volume) on isole 0,36 g de chlorhydrate de N,N-dihexyl α-méthyl phényl-2 quinoléine-4 propanamide fondant à 102°C.

*Exemple 66*

On procède comme à l'exemple 18 en partant de 1,1 g d'acide α-méthyl phényl-3 naphtalène-1 propanoïque, de 0,37 $cm^3$ de chlorure de thionyle dans 20 $cm^3$ de chloroforme et de 2 $cm^3$ de diéthylamine dans 20 $cm^3$ de chloroforme.

Le résidu est chromatographié sur du gel de silice en utilisant comme éluant un mélange cyclohexane-acétate d'éthyle (70-30 en volume). On isole ainsi 1 g de N,N-diéthyl α-méthyl phényl-3 naphtalène-1 propanamide fondant à 70°C.

L'acide α-méthyl phényl-3 naphtalène-1 propanoïque peut être préparé de la manière suivante:

1 — Préparation du phényl-3 naphtalène-1 méthanol.

A une solution agitée de 5,6 g de phényl-3 naphtalène-1 carboxylate d'éthyle dans 80 $cm^3$ de tertiobutanol, on ajoute 1,9 g de borohydrure de sodium. On porte au reflux, puis on coule en 2 heures 10 minutes 16 $cm^3$ de méthanol. On continue le chauffage au reflux pendant encore 2 heures, puis refroidit à température ambiante (20°C environ), ajoute 40 $cm^3$ d'eau puis 4 $cm^3$ d'acide acétique et évapore sous pression réduite. Le résidu est repris par 100 $cm^3$ de chloroforme et 100 $cm^3$ d'eau. La phase organique est décantée, lavée par 100 $cm^3$ d'eau et évaporée sous pression réduite. Le résidu est chromatographié sur du gel de silice en utilisant comme éluant du chloroforme. Les fractions contenant le phényl-3 naphtalène-1 méthanol sont rassemblées. Après évaporation sous pression réduite, on obtient un résidu que l'on reprend par du chlorure de méthylène, lave par une solution d'hydroxyde d'ammonium. La phase organique est séchée sur sulfate de magnésium et évaporée sous pression réduite.

On isole 3,6 g de phényl-3 naphtalène-1 méthanol dont le spectre RMN du proton dans le chloroforme deutérié a les caractéristiques suivantes:

Ar-$\underline{CH_2}$OH δ : 5,20 ppm

| | | |
|---|---|---|
| protons naphtaléniques | $H_2$ | δ : 7,48 ppm |
| | $H_4$ | δ : 7,67 ppm |
| protons du noyau phényle | Hoo' | δ : 7,71 ppm |
| | Hmm'p | δ : 7,45 ppm. |

Le phényl-3 naphtalène-1 carboxylate d'éthyle peut être préparé par estérification de l'acide phényl-3 naphtalène-1 carboxylique au moyen d'éthanol en présence d'acide sulfurique.

L'acide phényl-3 naphtalène-1 carboxylique peut être préparé selon F.G. BADDAR et Coll., J. Chem. Soc., 1009 (1959).

2 — Préparation du chlorométhyl-1 phényl-3 naphtalène.

A une solution agitée de 1 g de phényl-3 naphtalène-1 méthanol dans 20 $cm^3$ de chloroforme, préalablement refroidie à 0°C, on ajoute en 10 minutes 0,78 $cm^3$ de chlorure de thionyle puis on laisse remonter la température jusqu'à la température ambiante (20°C environ). On agite 15 heures à cette température et évapore le solvant sous pression réduite. On obtient 1,1 g de chlorométhyl-1 phényl-3 naphtalène dont le spectre RMN du proton dans le chloroforme deutérié présente les caractéristiques suivantes:

Ar-$\underline{CH_2}$Cl δ : 5,08 ppm

| | | |
|---|---|---|
| protons du naphtalène | $H_2$ | δ : 7,65 ppm |
| | $H_4$ | δ : 7,78 ppm |
| protons du phényle | Hmm'p | δ : 7,44 ppm. |

3 — Préparation de l'acide α-méthyl phényl-3 naphtalène-1 propanoïque.

A 15 $cm^3$ de tétrahydrofuranne sec, sous atomosphère d'azote, on ajoute lentement 0,62 g d'hydrure de sodium en dispersion à 60% dans l'huile. On introduit ensuite, goutte à goutte, une solution de 2,69 g de méthylmalonate de diéthyle dans 15 $cm^3$ de tétrahydrofuranne, puis en 50 minutes, une solution de 1,95 g de chlorométhyl-1 phényl-3 naphtalène dans 30 $cm^3$ de tétrahydrofuranne. On agite une heure à température ambiante (20°C environ) puis 3 heures au reflux du tétrahydrofuranne. On ramène à température ambiante (20°C environ), ajoute goutte à goutte 2 $cm^3$ d'acide acétique glacial. Le tétrahydrofuranne est évaporé sous pression réduite. Le résidu repris par 100 $cm^3$ d'eau et extrait par 2 fois 50 $cm^3$ d'éther éthylique. La phase éthérée est lavée à l'eau, séchée sur sulfate de magnésium et évaporée sous pression réduite. Le résidu obtenu est repris par 25 $cm^3$ d'une solution concentrée d'acide chlorhydrique et 25 $cm^3$ d'acide acétique glacial. On chauffe 4 heures au reflux puis on évapore au maximum les acides sous pression réduite, reprend le résidu par 100 $cm^3$ d'eau, alcalinise à pH 10 par addition d'une solution concentrée d'hydroxyde d'ammonium et extrait par 2 fois 50 $cm^3$ d'éther éthylique.

La phase aqueuse est acidifiée à pH 6 par addition d'acide acétique et extraite par 2 fois 50 $cm^3$ d'acétate d'éthyle. La phase organique est séchée sur sulfate de magnésium et évaporée sous pression réduite. Le résidu obtenu est chromatographié sur du gel de silice en utilisant comme éluant un mélange chloroforme-acide acétique (9-1 en volume). On isole 1 g d'acide α-méthyl phényl-3 naphtalène-1 propanoïque dont le spectre de RMN du proton dans le chloroforme deutérié présente les caractéristiques suivantes:

| | |
|---|---|
| Protons aromatiques | δ : 7,3 à 8,2 ppm |
| Ar-$\underline{CH_2}$, Ar-$CH_2$-$\underline{CH}$- | δ : 2,7 à 3,5 ppm |
| Ar-CH($\underline{CH_3}$)- | δ : 1,2 ppm. |

*Exemple 67*

On chauffe au reflux pendant 1 heure 45 minutes 3 g de phényl-4 quinolinethiol-2, 3,43 g de chlorure de N,N-diéthyl carbamoyle, 2,55 g de triéthylamine et 0,68 g de diméthylamino-4 pyridine dans 30 $cm^3$ de tétrahydrofuranne. On refroidit à température ambiante, évapore le tétrahydrofuranne sous pression réduite, reprend le résidu par 20 $cm^3$ d'eau et 50 $cm^3$ d'éther. La phase organique est décantée, la phase aqueuse lavée par 3 fois 50 $cm^3$ d'éther. Les phases organiques sont rassemblées, lavées à l'eau, séchées sur sulfate de magnésium et évaporées sous pression réduite. Le résidu obtenu est repris dans l'acétone et après addition d'une solution d'acide chlorhydrique dans l'éther et recristallisation dans un mélange acétone-éther, on isole 1,05 de chlorhydrate de S-diéthylcarbamothioate de phényl-4 quinolyle-2 fondant à 84°C.

Le phényl-4 quinoléinethiol-2 peut être préparé selon KUENZLE, F.M. et Coll., Helv. Chim. Acta 1970, 53 (4) 798-804.

*Exemple 68*

On chauffe au reflux pendant deux heures 45 minutes 4 g de phényl-2 quinolinethiol-4, 4,57 g de chlorure de N,N-diéthylcarbamoyle, 3,4 g de triéthylamine et 0,9 g de diméthylamino-4 pyridine dans 40 $cm^3$ de tétrahydrofuranne. On refroidit à température ambiante, élimine le précipité par filtration et évapore le filtrat sous pression réduite. Le résidu est repris par 20 $cm^3$ d'eau et 50 $cm^3$ d'acétate d'éthyle.

La phase organique est décantée, la phase acqueuse lavée par 2 fois 50 $cm^3$ d'acétate d'éthyle. Les phases organiques sont rassemblées, séchées sur sulfate de magnésium et évaporées sous pression réduite. Le résidu est chromatographié sur du gel de silice une première fois au moyen d'un éluant toluène-diéthylamine, puis une seconde fois au moyen d'un éluant cyclohexane-acétate d'éthyle (80-20 en volume).

Le résidu obtenu est repris dans l'acétone et après addition d'une solution d'acide chlorhydrique dans l'éther on isole 3,2 g de chlorhydrate de S-diéthylcarbamothioate de phényl-2 quinolyle-4 fondant à 122°C.

Le phényl-2 quinoléinethiol-4 peut être préparé selon JOHN, J. Prakt Chem. (2), 119, 49.

*Exemple 69*

On opère comme à l'exemple 18 en partant de 1,2 g d'acide [(phényl-2 quinolyl-4 oxy]-2 propanoïque dans 40 $cm^3$ de chloroforme, de 0,89 $cm^3$ de chlorure de thionyle, de 0,41 g de thiomorpholine et de 1,15 $cm^3$ de triéthylamine dans 20 $cm^3$ de chloroforme.

Le résidu est chromatographié sur du gel de silice en utilisant comme éluants successifs le chlorure de méthylène puis un mélange chlorure de méthylène acétate d'éthyle (95-5 en volume). On obtient 0,7 g de [(phényl-2 quinolyl-4) oxy]-2 propionyl-4 thiomorpholine fondant à 198°C.

*Exemple 70*

On opère comme à l'exemple 68 à partir de 6 g de nitro-6 phényl-2 hydroxy-4 quinoléine dans 60 $cm^3$ de tétrahydrofuranne, de 6,1 g de chlorure de N,N-diéthylcarbamoyle, de 6,3 $cm^3$ de triéthylamine et de 1,3 g de diméthylamino-4 pyridine. Le résidu est recristallisé deux fois dans l'acétate d'éthyle. On obtient ainsi 6,4 g de diéthylcarbamate de (nitro-6 phényl-2 quinolyle)-4 fondant à 140°C.

*Exemple 71*

On opère comme à l'exemple 68 à partir de trifluorométhyl-8 phényl-2 hydroxy-4 quinoléine dans 50 $cm^3$ de tétrahydrofuranne, de 4,7 g de chlorure de N,N-diéthylcarbamoyle, de 4,8 $cm^3$ de triéthylamine et de 1,1 g de diméthylamino-4 pyridine.

Après chromatographie du résidu sur du gel de silice en utilisant comme éluant un mélange cyclohexane-acétate d'éthyle (80-20 en volume) et recristallisation dans un mélange éther isopropylique-éther de pétrole, on obtient 1,38 g de diéthylcarbamate de (trifluorométhyl-8 phényl-2 quinolyle)-4 fondant à 79°C.

La trifluorométhyl-8 phényl-2 hydroxy-4 quinoléine peut être préparée par action à 140°C du benzoylacétate d'éthyle (0,12 mole) sur la trifluorométhyl-2 aniline (0,12 mole) en présence d'acide polyphosphorique (86 g). Elle présente un point de fusion de 136°C.

*Exemple 72*

On opère comme à l'exemple 61, à partir de 2,1 g de phényl-4 naphtol-2, de 2,6 g de chlorure de N,N-diéthylcarbamoyle, de 2,7 $cm^3$ de triéthylamine et de 0,05 g de diméthylamino-4 pyridine dans 20 $cm^3$ de tétrahydrofuranne.

Après trois chromatographies successives du résidu dans un mélange cyclohexane-acétate d'éthyle (80-20 en volume), on isole 0,5 g de diéthylcarbamate de (phényl-2 naphtyle)-2 dont le spectre de RMN du proton dans le chloroforme deutérié a les caractéristiques suivantes:

$H_1$ : $\delta$ = 7,6 ppm
$H_3$ : $\delta$ = 7,25 ppm
$H_5$ et $H_8$ : $\delta$ = 7,85 ppm.

Les autres protons aromatiques : $\delta$ entre 7,3 et 7,6 ppm.

Le phényl-4 naphtol-2 peut être préparé selon KOPTYUG et ANDREEVA, Zh. Org. Khim. 1971, 7 (II) 2398-403.

*Exemple 73*

On opère comme à l'exemple 31, à partir de 2,7 g de (méthoxy-4 phényl)-2 quinolinol-4, de 3 $cm^3$ de triéthylamine et de 3 g de chlorure de N,N-diéthylcarbamoyle dans 15 $cm^3$ de diméthylformamide.

Le résidu est purifié comme dans l'exemple 31; le chlorhydrate brut est recristallisé dans l'acétate d'éthyle. On isole ainsi 0,8 g de chlorhydrate de diéthylcarbamate de [(méthoxy-4 phényl)-2 quinolyle]-4 fondant à 107°C.

La (méthoxy-4 phényl)-2 hydroxy-4 quinoléine peut être préparée selon SORM, Chem. Listy 49, 901 (1954).

*Exemple 74*

On opère comme à l'exemple 68, à partir de 3,3 g de (trifluorométhyl-3 phényl)-2 hydroxy-4 quinoléine, de 3,1 g chlorure de N,N-diéthylcarbamoyle, de 3,2 $cm^3$ de triéthylamine, et de 0,9 g de diméthylamino-4 pyridine dans 33 $cm^3$ de tétrahydrofuranne.

Après chromatographie du résidu sur du gel de silice en utilisant comme éluant un mélange cyclohexane-acétate d'éthyle (80-20 en volume), on isole 1,35 g de diéthylcarbamate de (trifluorométhyl-3 phényl)-2 quinolyle-4 fondant à 96°C.

La (trifluorométhyl-3 phényl)-2 hydroxy-4 quinoléine peut être préparée par action à 160°C du trifluorométhyl-3 benzoyl acétate d'éthyle (0,245 mole) sur l'aniline (0,245 mole) en présence d'acide polyphosphorique (156 g).

*Exemple 75*

On opère comme à l'exemple 68, à partir de 5 g de (méthyl-4 phényl)-2 hydroxy-4 quinoléine, de 5,8 g de chlorure de N,N-diéthylcarbamoyle, de 6 $cm^3$ de triéthylamine, de 1,35 g de diméthylamino-4 pyridine dans 50 $cm^3$ de tétrahydrofuranne.

Le résidu est chromatographié sur du gel de silice en utilisant un mélange cyclohexane-acétate d'éthyle (80-20 en volume) comme éluant.

Le résidu est repris dans l'éther éthylique et, après addition d'une solution d'acide chlorhydrique dans isopropanol, on isole 6,5 g de chlorhydrate de diéthylcarbamate de [(méthyl-4 phényl)-2 quinolyle]-4 fondant à 116°C.

La (méthyl-4 phényl)-2 hydroxy-4 quinoléine peut être préparée par action à 140°C du méthyl-4 benzoylacétate d'éthyle (0,294 mole) sur l'aniline (0,294 mole) en présence d'acide polyphosphorique (168). Elle présente un point de fusion supérieur à 268°C.

*Exemple 76*

On opère comme à l'exemple 68, à partir de 2 g de (fluoro-2 phényl)-2 hydroxy-4 quinoléine, de 2,27 g de chlorure de N,N-diéthylcarbamoyle, de 2,35 $cm^3$ de triéthylamine, de 0,55 g de diméthylamino-4 pyridine dans 20 $cm^3$ de tétrahydrofuranne.

Le résidu est chromatographié sur du gel de silice

en utilisant un mélange cyclohexane-acétate d'éthyle (70-30 en volume) comme éluant.

Le résidu est repris dans l'éther éthylique, et après addition d'une solution d'acide chlorhydrique dans l'isopropanol, on isole 2,4 g de chlorhydrate de diéthylcarbamate de [(fluoro-2 phényl)-2 quinolyle]-4 fondant à 123°C.

La (fluoro-2 phényl)-2 hydroxy-4 quinoléine peut être préparée par action à 160°C du fluoro-2 benzoylacétate d'éthyle (0,05 mole) sur l'aniline (0,05 mole) en présence d'acide polyphosphorique (25 g). Elle présente un point de fusion égal à 224°C.

*Exemple 77*

On opère comme à l'exemple 68 à partir de 3 g de (thiényl-2)-2 hydroxy-4 quinoléine, de 3,58 g de chlorure de N,N-diéthylcarbamoyle, de 3,7 $cm^3$ de triéthylamine, de 0,5 g de diméthylamino-4 pyridine dans 30 $cm^3$ de tétrahydrofuranne.

Le résidu est repris dans l'éther éthylique et après addition d'une solution d'acide chlorhydrique dans l'isopropanol, on isole un chlorhydrate brut. Ce dernier est repris par 150 $cm^3$ d'acétate d'éthyle, 150 $cm^3$ d'eau et 40 $cm^3$ d'une solution normale d'hydroxyde de sodium. La phase organique est décantée, lavée à l'eau, séchée sur sulfate de magnésium et évaporée sous pression réduite. Le résidu obtenu est cristallisé dans l'éther de pétrole. On isole ainsi 2,3 g de diéthylcarbamate de (thiényl-2)-2 quinolyle-4 fondant à 72°C.

La (thiényl-2)-2 hydroxy-4 quinoléine peut être préparée par action à 160°C du thénoyl-2 acétate d'éthyle (0,103 mole) sur l'aniline (0,103 mole) en présence d'acide polyphosphorique (45,8 g). Elle présente un point de fusion supérieur à 268°C.

*Exemple 78*

On opère comme à l'exemple 68, à partir de 4,44 g de (pyridyl-4)-2 hydroxy-4 quinoléine, de 5,42 g de chlorure de N,N-diéthylcarbamoyle, de 5,6 $cm^3$ de triéthylamine, de 0,5 g de diméthylamino-4 pyridine dans 45 $cm^3$ de tétrahydrofuranne.

Après chromatographie du résidu sur du gel de silice en utilisant un mélange cyclohexane-acétate d'éthyle (20-80 en volume), on isole un résidu que l'on purifie par l'intermédiaire d'un chlorhydrate brut comme à l'exemple 77.

On isole ainsi 1,15 g de diéthylcarbamate de (pyridyl-4)-2 quinolyle-4 fondant à 76°C.

La (pyridyl-4)-2 hydroxy-4 quinoléine peut être préparée par action, à 160°C du pyridinoyl-4 acétate d'éthyle (0,08 mole) sur l'aniline (0,08 mole) en présence d'acide polyphosphorique (36 g). Elle présente un point de fusion égal à 246°C.

*Exemple 79*

On opère comme à l'exemple 68, à partir de 1,8 g de (chloro-3 phényl)-2 hydroxy-4 quinoléine de 1,8 $cm^3$ de chlorure de N,N-diéthylcarbamoyle, de 2 $cm^3$ de triéthylamine et de 0,2 g de diméthylamino-4 pyridine dans 20 $cm^3$ tétrahydrofuranne anhydre.

Après deux chromatographies successives du résidu sur du gel de silice, la première au moyen d'un mélange cyclohexane-acétate d'éthyle (50-50 en volume), la deuxième en utilisant un mélange cyclohexane-acétate d'éthyle (90-10 en volume), et cristallisation dans l'éther de pétrole 40-60°, on isole 1,15 g de diéthylcarbamate de [(chloro-3 phényl)-2 quinolyle]-4 fondant à 83°C.

La (chloro-3 phényl)-2 hydroxy-4 quinoléine peut être préparée par action à 160°C du chloro-3 benzoylacétate d'éthyle (0,025 mole) sur l'aniline (0,025 mole) en présence d'acide polyphosphorique (11 g). Elle présente un point de fusion de 210°C.

*Exemple 80*

On opère comme à l'exemple 68, à partir de 2,8 g (pyridyl-2)-2 hydroxy-4 quinoléine, de 3,42 g de chlorure de N,N-diéthylcarbamoyle, de 3,5 $cm^3$ de triéthylamine, de 0,5 g de diméthylamino-4 pyridine dans 23 $cm^3$ de tétrahydrofuranne anhydre.

Après chromatographie du résidu sur du gel de silice en utilisant l'acétate d'éthyle comme éluant et cristallisation dans l'éthanol on isole 2,25 g de diéthylcarbamate de (pyridyl-2)-2 quinolyle-4 fondant à 100°C.

La (pyridyl-2)-2 hydroxy-4 quinoléine peut être préparée par action à 160°C du pyridinoylacétate d'éthyle (0,05 mole) sur l'aniline (0,05 mole) en présence d'acide polyphosphorique (58 g). Elle présente un point de fusion égal à 228°C.

*Exemple 81*

On opère comme à l'exemple 50, en partant de 6 g de phényl-2 trifluorométhyl-8 quinolinol-4, de 4,76 g de N,N-diéthyl bromo-2 propanamide, de 6 g de carbonate de potassium dans 400 $cm^3$ de méthyléthylcétone.

Après recristallisation du résidu dans un mélange acétate d'éthyle-éther isopropylique (1-4 en volume), on isole 3 g de N,N-diéthyl [(phényl-2 trifluorométhyl-8 quinolyl-4) oxy]-2 propanamide fondant à 146°C.

La phényl-2 trifluorométhyl-8 hydroxy-4 quinoléine peut être préparée par action 140°C de benzoylacétate d'éthyle (0,12 mole) sur la trifluorométhyl-2 aniline (0,12 mole) en présence d'acide polyphosphorique (86 g). Elle présente un point de fusion égal à 136°C.

*Exemple 82*

A une solution agitée de 2 g d'acide α-méthyl phényl-2 quinazoline-4 propanoïque dans 20 $cm^3$ de tétrahydrofuranne, on ajoute 1,65 g de carbonyldiimidazole. On agite pendant une vingtaine de minutes jusqu'à la fin du dégagement gazeux, puis encore une heure et on ajoute 1,05 $cm^3$ de diéthylamine. On agite 5 jours à température ambiante (20°C environ) puis chauffe deux heures au reflux. On évapore le solvant sous pression réduite et reprend le résidu par 100 $cm^3$ d'éther éthylique. La phase organique est lavée par 2 fois 10 $cm^3$ d'eau, puis 2 fois 5 $cm^3$ d'une solution normale d'acide chlorhydrique puis 2 fois 5 $cm^3$ d'une solution normale d'hydroxyde de sodium et enfin par 2 fois 5 $cm^3$ d'eau. Elle est ensuite séchée sur sulfate de magnésium et évaporée sous pression réduite. Le résidu est recristallisé deux fois dans l'éther isopropylique. On isole ainsi 1,1 g de N,N-diéthyl α-méthyl phényl-2 quinazoline

propanamide-4 lévogyre fondant à 93,5°C, $\alpha_D$ à 0,5% dans EtOH à 23°C = −17,9° ± 2°.

L'acide α-méthyl phényl-2 quinazoline-4 propanoïque dextrogyre peut être préparé par dédoublement de l'acide α-méthyl phényl-2 quinazoline-4 propanoïque racémique en opérant de la façon suivante:

1) Préparation des N-(phényl-1 hydroxy-2 éthyl) α-méthyl phényl-2 quinazoline-4 propanamides diastéréomères.

Sous atmosphère d'azote, à une solution agitée de 59,1 g d'acide α-méthyl phényl-2 quinazoline-4 propanoïque dans 1800 $cm^3$ de chlorure de méthylène on ajoute 44,5 g de 2,2' dipyridyldisulfure et 28 g de (−)α-phényl glycinol. On refroidit vers 0°C puis introduit en 15 minutes par spatulées 53 g de triphénylphosphine. On agite 21 heures à température ambiante (20°C environ), élimine le solvant sous pression réduite, reprend le résidu par 1800 $cm^3$ d'acétate d'éthyle. La phase organique est lavée successivement par 450 $cm^3$ puis 200 $cm^3$ puis 100 $cm^3$ d'une solution normale d'hydroxyde de sodium, ensuite par 2 fois 100 $cm^3$ d'eau et enfin par 200 $cm^3$ d'une solution à 10% de dithionnite de sodium, 100 $cm^3$ d'eau et 100 $cm^3$ d'une solution saturée de chlorure de sodium. La phase organique est finalement séchée sur sulfate de magnésium et évaporée sous pression réduite. Le résidu est chromatographié sous pression sur du gel de silice en utilisant comme éluant un mélange chloroforme-toluène diéthylamine (50-44-6 en volume).

Après recristallisation des deux amides diastéréomères dans l'acétonitrile, on obtient 18,1 g de N-(phényl-1 hydroxy-2 éthyl) α-méthyl phényl-2 quinazoline-4 propanamide forme A, fondant à 199°C, qui est élué d'abord, puis 15,4 g de N-(phényl-1 hydroxy-2 éthyl) α-méthyl phényl-2 quinazoline-4 propanamide forme B que l'on élue ensuite et qui présente un point de fusion égal à 204°C.

2) Préparation de l'acide α-méthyl phényl-2 quinazoline-4 propanoïque dextrogyre.

On chauffe au reflux pendant une heure 30 minutes 19 g de N-(phényl-1 hydroxy-2 éthyl) α-méthyl phényl-2 quinazoline-4 propanamide forme A dans 90 $cm^3$ d'acide acétique glacial et 90 $cm^3$ d'une solution concentrée d'acide chlorhydrique.

On évapore les acides sous pression réduite, reprend le résidu par 600 $cm^3$ d'eau, alcalinise à pH 10 au moyen d'une solution concentrée d'hydroxyde d'ammonium et lave par 100 $cm^3$ d'éther éthylique. La phase aqueuse est acidifiée à pH 5 par de l'acide acétique cristallisable. Le précipité est filtré, lavé à l'eau et séché. On obtient ainsi 13,5 g d'acide α-méthyl phényl-2 quinazoline-4 propanoïque dextrogyre fondant à 179,1°C, $\alpha_D$ à 0,5% dans l'acide acétique glacial = + 4° ± 2° à 21°C.

*Exemple 83*

On opère comme à l'exemple 82 en partant de 2 g d'acide α-méthyl phényl-2 quinazoline-4 propanoïque lévogyre dans 20 $cm^3$ de tétrahydrofuranne, de 1,65 g de carbonyldiimidazole et de 1,05 $cm^3$ de diéthylamine. Après deux recristallisations dans l'éther isopropylique on isole 1,3 g de N,N-diéthyl α-méthyl phényl-2 quinazoline propanamide dextrogyre fondant à 93,8°C.

$\alpha_D$ à 0,5% dans EtOH à 23°C = + 17,8° ± 2°.

L'acide α-méthyl phényl-2 quinazoline-4 propanoïque lévogyre peut être préparé comme son énantiomère dextrogyre décrit à l'exemple 82 en partant de 16,4 g de N-(phényl-1 hydroxy-2 éthyl) α-méthyl phényl-2 quinazoline-4 propanamide forme B préparé selon l'exemple 82, de 80 $cm^3$ d'acide acétique glacial et de 80 $cm^3$ d'une solution concentrée d'acide chlorhydrique.

On obtient 11,4 g d'acide α-méthyl phényl-2 quinazoline-4 propanoïque lévogyre fondant à 179,3°C.'

$\alpha_D$ à 0,5% dans l'acide acétique glacial = −4° ± 2° à 21°C.

*Exemple 84*

A une solution agitée de 2 g d'acide α-méthyl phényl-2 quinazoline-4 propanoïque dextragyre dans 20 $cm^3$ de tétrahydrofuranne, on ajoute 1,65 g de carbonyldiimidazole. On agite une heure à température ambiante (jusqu'à fin du dégagement gazeux) puis on ajoute 1,03 $cm^3$ de N-méthyl isopropylamine. On agite trois jours à température ambiante (20°C environ), chauffe ensuite au reflux 6 heures, ajoute 1,03 $cm^3$ de N-méthyl isopropylamine puis chauffe encore 12 heures au reflux. Le traitement est ensuite analogue à celui décrit à l'exemple 85. Après deux recristallisations dans l'acétonitrile, on isole 19 g de N-isopropyl N-méthyl α-méthyl phényl-2 quinazoline propanamide-4 lévogyre fondant à 171,2°C, $\alpha_D$ à 0,5% dans EtOH à 23°C = −12,1° ± 2°.

*Exemple 85*

On opère comme à l'exemple 84, en partant de 2 g d'acide α-méthyl phényl-2 quinazoline-4 propanoïque lévogyre dans 20 $cm^3$ de tétrahydrofuranne, de 1,65 g de carbonyldiimidazole et de 1,03 $cm^3$ de N-méthyl isopropylamine.

Après deux recristallisations dans l'acétonitrile, on isole 1,1 g de N-isopropyl N-méthyl α-méthyl phényl-2 quinazoline propanamide-4 dextrogyre fondant à 171,0°C.

$\alpha_D$ à 0,5% dans EtOH à 23°C = + 16,3° ± 2°.

Les médicaments selon l'invention sont constitués par un composé de formule I, un mélange de composés stéréoisomères de formule I, ou lorsqu'il peut exister un sel d'un tel composé ou mélange, à l'état pur ou sous forme d'une composition dans laquelle il est associé à tout autre produit pharmaceutiquement compatible, pouvant être inerte ou physiologiquement actif. Ces médicaments selon l'invention peuvent être utilisés par voie orale, parentérale, rectale ou topique.

Comme compositions solides pour administration orale peuvent être utilisés des comprimés, pilules, poudres (capsules de gélatine, cachets) ou granulés. Dans ces compositions, le principe actif selon l'invention est mélangé à un ou plusieurs diluants inertes, tels que amidon, cellulose, saccharose, lactose

ou silice. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale peuvent être de préférence des solutions non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans un milieu stérile injectable.

Les compositions pour administation rectale sont les suppositoires ou les capsules rectales, qui contiennent outre le produit actif des excipients tels que le beurre de cacao, des glycérides semi-synthétiques ou des polyéthylèneglycols.

Les compositions pour administration topique peuvent être par exemple des crèmes, pommades, lotions, collyres, collutoires, gouttes nasales ou aérosols.

En thérapeutiques humaine, les composés selon l'invention sont particulièrement utiles comme anxiolytiques, anticonvulsivants, antiangoreux et pour le traitement des états d'immunodépression.

Les doses dépendent de l'effet recherché, de la durée du traitement et de la voie d'administration utilisée; elles sont généralement comprises entre 20 et 1000 mg par jour par voie orale pour un adulte avec des doses unitaires allant de 5 à 200 mg de substance active.

D'une façon générale, le médicin déterminera la posologie appropriée en fonction de l'âge, du poids et de tous les autres facteurs propres au sujet à traiter.

Les exemples suivants illustrent des compositions selon l'invention:

*Exemple A*

On prépare, selon la technique habituelle, des gélules dosées à 50 mg de produit actif ayant la composition suivante:

| | |
|---|---|
| - N,N-diéthyl [(phényl-2 quinolyl-4) oxy]-2 propanamide | 50 mg |
| - Cellulose | 18 mg |
| - Lactose | 55 mg |
| - Silice celloïdale | 1 mg |
| - Carboxyméthylamidon sodique | 10 mg |
| - Talc | 10 mg |
| - Stéarate de magnésium | 1 mg |

*Exemple B*

On prépare selon la technique habituelle, des comprimés dosés à 50 mg de produit actif ayant la composition suivante:

| | |
|---|---|
| - N,N-diéthyl α-méthyl phényl-2 quinazoline-4 propanamide | 50 mg |
| - Lactose | 104 mg |
| - Cellulose | 40 mg |
| - Polyvidone | 10 mg |
| - Carboxyméthylamidon sodique | 22 mg |
| - Talc | 10 mg |
| - Stéarate de magnésium | 2 mg |
| Silice colloïdale | 2 mg |
| - Mélange d'hydroxyméthylcellulose, glycérine, oxyde de titane (72-3,5-24,5) | q.s.p 1 comprimé pelliculé terminé à 245 mg. |

*Exemple C*

On prépare une solution injectable contenant 10 mg de produit actif ayant la composition suivante:

| | | | |
|---|---|---|---|
| - Diéthylcarbamate de phényl-3 isoquinolyle-1 | | 10 | mg |
| - Acide benzoïque | | 80 | mg |
| - Alcool benzylique | | 0,06 | $cm^3$ |
| - Benzoate de sodium | | 80 | mg |
| - Ethanol à 95% | | 0,4 | $cm^3$ |
| - Hydroxyde de sodium | | 24 | mg |
| - Propylène glycol | | 1,6 | $cm^3$ |
| - Eau | q.s.p | 4 | $cm^3$. |

**Revendications pour les Etats Contractants:**
BE, CH, DE, FR, GB, IT, LI, LU, NL, SE

1. Composé racémique ou stéréoisomère de formule I

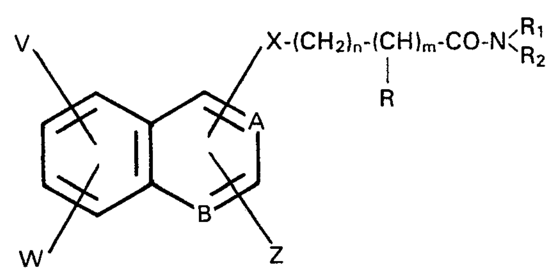

(I)

dans laquelle A représente un atome d'azote ou un groupe CH,

B représente un atome d'azote ou un groupe CH,

V et W, identiques ou différents, représentent des atomes d'hydrogène ou d'halogène, des groupes alkyle ou alcoxy comportant 1 à 3 atomes de carbone, des groupes nitro ou trifluorométhyle,

Z est fixé en position ortho ou para par rapport à B

et représente un radical phényle, thiényle, pyridyle ou phényle substitué par un ou deux substituants pris parmi les atomes d'halogène, les groupes alkyle et alcoxy comportant 1 à 4 atomes de carbone, les groupes trifluorométhyle ou nitro,

la chaîne $X-(CH_2)_n-(CHR)_m-CO-NR_1R_2$ est fixée en position ortho ou para par rapport à B,

E représente un atome d'hydrogène ou un groupe alkyle comportant 1 à 3 atomes de carbone,

$R_1$ et $R_2$, identiques ou différents, représentent un groupe alkyle linéaire ou ramifié comportant 1 à 6 atomes de carbone, cycloalkyle comportant 3 à 6 atomes de carbone, phényle, phénylalkyle ou cycloalkylalkyle dont la partie alkyle comporte 1 à 3 atomes de carbone et la partie cycloalkyle comporte 3 à 6 atomes de carbone, alcényle comportant 3 à 6 atomes de carbone à condition que la double liaison ne soit pas située en position 1, 2 par rapport à l'atome d'azote,

$R_1$ et $R_2$ peuvent également former ensemble avec l'atome d'azote auquel ils sont rattachés un cycle pipéridine ou thiomorpholine,

X représente un groupe $CH-R_3$, $N-R_4$, SO, $SO_2$ ou un atome d'oxygène ou de soufre,

$R_3$ représente un atome d'hydrogène ou un groupe alkyle comportant 1 à 3 atomes de carbone,

$R_4$ représente un groupe alkyle comportant 1 à 3 atomes de carbone

m est égal à 0 ou 1,

n est égal à 0, 1 ou 2,

étant entendu que lorsque X représente un groupe SO, $SO_2$ ou $N-R_4$, la somme m + n est au moins égale à 1, étant entendu que lorsque A et B représentent chacun un atome d'azote et Z est en position para par rapport à B, X ne peut pas représenter le groupe $CH-R_3$, étant entendu que lorsque A représente un groupe CH, B représente un atome d'azote, Z est en position ortho par rapport à B, X représente un atome d'oxygène et R représente un atome d'hydrogène, la somme m + n est différente de 1; étant entendu que lorsque A représente un groupe CH, B représente un atome d'azote, X représente un groupe $CH_2$ et R représente un atome d'hydrogène, la somme m + n est différente de 2, et à l'exception du N,N-diméthylcarbamate de phényl-2 quinolyle-4 ainsi que lorsqu'il peut exister un sel d'un tel composé avec un acide.

2. Composé racémique ou stéréoisomere selon la revendication 1 pour lesquels V et W, identiques ou différents, représentent des atomes d'hydrogène ou des groupes alkyle comportant 1 à 3 atomes de carbone,

Z est fixé en position ortho par rapport à B et représente un radical phényle, éventuellement substitué par un groupe alkyle, alcoxy comportant 1 à 4 atomes de carbone, un groupe nitro ou trifluorométhyle ou un radical thiènyle,

la chaîne $X-(CH_2)_n-(CHR)_m-CO-NR_1R_2$ est fixée en position para par rapport à B,

E représente un atome d'hydrogène ou un groupe alkyle comportant 1 à 3 atomes de carbone,

$R_1$ et $R_2$, identiques ou différents, représentent un groupe alkyle linéaire ou ramifié comportant 1 à 6 atomes de carbone ou un groupe phényle,

$R_1$ et $R_2$ peuvent également former ensemble avec l'atome d'azote auquel ils sont rattachés un cycle pipéridine,

X représente un groupe $CH-R_3$, ou un atome d'oxygène ou de soufre, $R_3$ représente un atome d'hydrogène,

m est égal à 0 ou 1,

n est égal à 0, 1 ou 2,

et soit A représente un groupe CH ou B un atome d'azote, soit A représente un atome d'azote et B un groupe CH, soit A et B représentent un atome d'azote, étant entendu que lorsque X représente un groupe SO, $SO_2$ ou $N-R_4$, la somme m + n est au moins égale à 1, étant entendu que lorsque A et B représentent chacun un atome d'azote et Z est en position para par rapport à B, X ne peut pas représenter le groupe $CH-R_3$, étant entendu que lorsque A représente un groupe CH, B représente un atome d'azote, Z est en position ortho par rapport à B, X représente un atome d'oxygène et R représente un atome d'hydrogène, la somme m + n est différente de 1, étant entendu que lorsque A représente un groupe CH, B représente un atome d'azote, X représente un groupe $CH_2$ et R représente un atome d'hydrogène, la somme m + n est différente de 2, et à l'exception du N,N-diméthylcarbamate de phényl-2 quinolyle-4 ainsi que lorsqu'il peut exister un sel d'un tel composé avec un acide.

3. Procédé de préparation d'un composé selon la revendication 1 dans la formule duquel X représente un atome d'oxygène ou de soufre, n et m sont égaux à 0, A, B, V, W, Z, $R_1$, $R_2$ sont définis comme à la revendication 1 caractérisé en ce que l'on fait réagir un composé de formule:

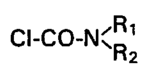 (II)

dans laquelle $R_1$ et $R_2$ ont les mêmes significations que ci-dessus avec un dérivé de formule:

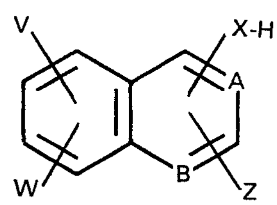 (III)

dans laquelle A, B, V, W, Z ont les mêmes significations que ci-dessus et X représente un atome d'oxygène ou de soufre, isole le produit et le transforme éventuellement en sel d'addition avec un acide.

4. Procédé de préparation d'un composé selon la revendication 1 dans la formule duquel X représente un atome d'oxygène ou de soufre, n est égal à 0, m est égal à 1, A, B, V, W, Z, R, $R_1$ et $R_2$ sont définis comme à la revendication 1 caractérisé en ce que l'on fait réagir un composé de formule:

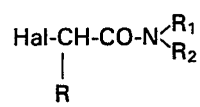 (IV)

sur un dérivé de formule:

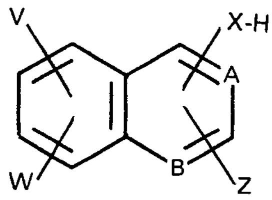

(III)

dans lesquelles Hal représente un atome d'halogène, X représente un atome d'oxygène ou de soufre, A, B, V, W, Z, R, $R_1$ et $R_2$ ont les mêmes significations que ci-dessus, isole le produit et le transforme éventuellement en sel d'addition avec un acide.

5. Procédé de préparation d'un composé selon la revendication 1 dans la formule duquel soit X représente un atome d'oxygène ou de soufre, n est égal à 0, 1 ou 2 et m est égal à 1, soit X représente un groupe $CH-R_3$, n est égal à 0,1 ou 2 et m est égal à 0 ou 1, A, B, V, W, Z, R, $R_1$, $R_2$ et $R_3$ sont définis comme à la revendication 1 caractérisé en ce que l'on traite un composé de formule:

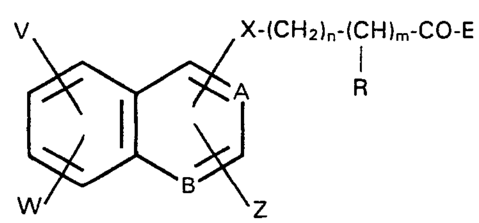

(VI)

avec une amine de formule:

$$HN{<}^{R_1}_{R_2} \quad (V)$$

dans lesquelles E représente un groupe alcoxy comportant 1 à 4 atomes de carbone, alcoxycarbonyloxy comportant 2 à 5 atomes de carbone, un atome de chlore, ou un reste N-imidazolyle X est un atome d'oxygène ou de soufre ou un groue $CH-R_3$, A, B, V, W, Z, R, $R_1$, $R_2$, m et n ont les mêmes significations que ci-dessus, isole le produit et le transforme éventuellement en sel d'addition avec un acide.

6. Procédé de préparation d'un composé selon la revendication 1 dans la forme duquel X représente un groupe sulfinyle ou sulfonyle, m est égal à 1, A, B, V, W, Z, R, $R_1$, $R_2$ et n sont définis comme à la revendication 1 caractérisé en ce que l'on oxyde les composés correspondants pour lesquels X représente l'atome de soufre, isole le produit et le transforme éventuellement en sel d'addition avec un acide.

7. Procédé de préparation d'un composé selon la revendication 1 dans la formule duquel A est un groupe CH, B est un atome d'azote, Z est fixé en position ortho par rapport à B, la chaîne $X-(CH_2)_n-(CHR)_m-CONR_1R_2$ est fixée en position para par rapport à B, m est égal à 0, n est égal à 0, X représente le groupe $CH-R_3$, $R_3$ représente un groupe alkyle comportant 1 à 3 atomes de carbone, V, W, Z, $R_T$ et $R_2$ sont définis comme à la revendication 1 caractérisé en ce que l'on alkyle un composé de formule:

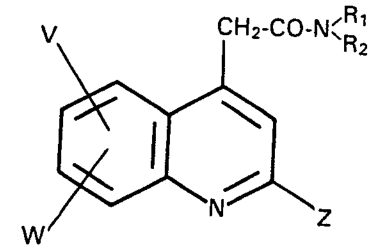

(VIII)

avec un composé de formule $R_3$-Hal

dans lesquelles V, W, Z, $R_1$, $R_2$ et $R_3$ ont les mêmes significations que ci-dessus et Hal représente un atome d'halogène, isole le produit et le transforme éventuellement en sel d'addition avec un acide.

8. Procédé de préparation d'un composé selon la revendication 1 dans la formule duquel X représente un groupe $N-R_4$, m est égal à 1, A, B, V, W, Z, R, $R_1$, $R_2$, $R_4$ et n sont définis comme dans la revendication 1 caractérisé en ce que l'on fait réagir un compose de formule:

$$R_4\text{-}NH\text{-}(CH_2)_n\text{-}\underset{\displaystyle R}{\underset{|}{CH}}\text{-}CO\text{-}N{<}^{R_1}_{R_2} \quad (IX)$$

sur un dérivé de formule:

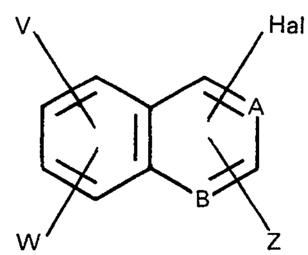

(X)

dans lesquelles Hal représente un atome d'halogène, A, B, V, W, Z, R, $R_1$, $R_2$, $R_4$ et n ont les mêmes significations que ci-dessus, isole le produit et éventuellement transforme en sel d'addition avec un acide.

9. Procédé de préparation d'un composé selon la revendication 1 dans la formule duquel A et B représentent chacun un atome d'azote, Z est fixé en position ortho par rapport à B, la chaîne $X-(CH_2)_n-(CHR)_m-CO-NR_1R_2$ est fixée en position para par rapport à B, X est un groupe $CH_2$, n est égal à 1, m est égal à 0, V, W, $R_1$, $R_2$ sont définis comme dans la revendication 1 caractérisé en ce que l'on fait agir un chlorure de formule ZCOCl sur un composé de formule:

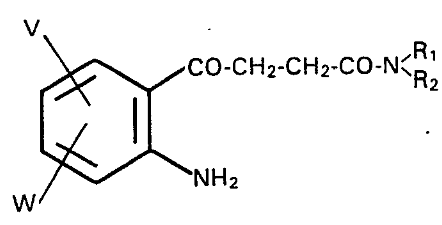

(XI)

dans lequelles V, W, Z, $R_1$ et $R_2$ ont les mêmes significations que ci-dessus, puis cyclise l'intermédiaire ainsi obtenu, isole le produit et le transforme éventuellement en sel d'addition avec un acide.

10. Médicament contenant au moins une substance active et éventuellement un ou plusieurs diluants ou adjuvants compatibles et pharmaceutiquement acceptables caractérisé en ce que la substance active est un composé de formule I

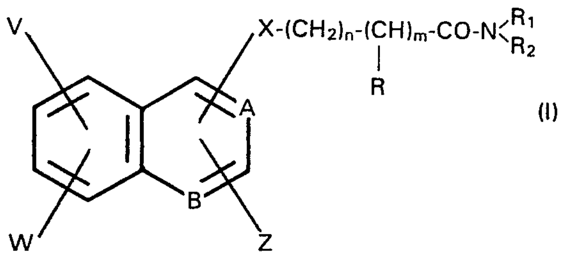

(I)

dans laquelle A représente un atome d'azote ou un groupe CH,

B représente un atome d'azote ou un groupe CH,

V et W, identiques ou différents, représentent des atomes d'hydrogène ou d'halogène, des groupes alkyle ou alcoxy comportant 1 à 3 atomes de carbone, des groupes nitro ou trifluorométhyle,

Z est fixé en position ortho ou para par rapport à B et représente un radical phényle, thiényle, pyridyle ou phényle substitué par un ou deux substituants pris parmi les atomes d'halogène, les groupes alkyle et alcoxy comportant 1 à 4 atomes de carbone, les groupes trifluorométhyle ou nitro,

la chaîne $X-(CH_2)_n-(CHR)_m-CO-NR_1R_2$ est fixée en position ortho ou para par rapport à B,

R représente un atome d'hydrogène ou un groupe alkyle comportant 1 à 3 atomes de carbone,

$R_1$ et $R_2$, identiques ou différents, représentent un groupe alkyle linéaire ou ramifié comportant 1 à 6 atomes de carbone, cycloalkyle comportant 3 à 6 atomes de carbone, phényle, phénylalkyle ou cycloalkylalkyle dont la partie alkyle comporte 1 à 3 atomes de carbone et la partie cycloalkyle comporte 3 à 6 atomes de carbone, alcényle comportant 3 à 6 atomes de carbone à condition que la double liaison ne soit pas située en position 1,2 par rapport à l'atome d'azote,

$R_1$ et $R_2$ peuvent également former ensemble avec l'atome d'azote auquel ils sont rattachés un cycle pipéridine ou thiomorpholine,

X représente un groupe $CH-R_3$, $N-R_4$, SO ou $SO_2$ ou un atome d'oxygène ou de soufre,

$R_3$ représente un atome d'hydrogène ou un groupe alkyle comportant 1 à 3 atomes de carbone,

$R_4$ représente un groupe alkyle comportant 1 à 3 atomes de carbone

m est égal à 0 ou 1,

n est égal à 0, 1 ou 2,

étant entendu que lorsque X représente un groupe SO, $SO_2$ ou $N-R_4$, la somme m + n est au moins égale à 1, étant entendu que lorsque A et B représentent chacun un atome d'azote et Z est en position para par rapport à B, X ne peut pas représenter le groupe $CH-R_3$, étant entendu que lorsque A représente un groupe CH, B représente un atome d'azote, Z est en position ortho par rapport à B, X représente un atome d'oxygène et R représente un atome d'hydrogène, la somme m + n est différente de 1; étant entendu que lorsque A représente un groupe CH, B représente un atome d'azote, X représente un groupe $CH_2$ et R représente un atome d'hydrogène, la somme m + n est différente de 2, et à l'exception du N,N-diméthylcarbamate de phényl-2 quinolyle-4, ou un mélange de composés stéréoisomères de formule I ou lorsqu'il peut exister un sel d'un tel composé ou d'un mélange de composés stéréoisomères avec un acide pharmaceutiquement acceptable.

11. Médicament selon la revendication 10 contenant un composé de formule I dans laquelle V et W, identiques ou différents, représentent des atomes d'hydrogène ou des groupes alkyle comportant 1 à 3 atomes d'hydrogène,

Z est fixé en position ortho par rapport à B et représente un radical phényle éventuellement substitué par un groupe alkyle, alcoxy comportant 1 à 4 atomes de carbone, un groupe nitro ou trifluorométhyle ou un radical thiényle,

la chaîne $X-(CH_2)_n-(CHR)_m-CO-NR_1R_2$ est fixée en position para par rapport à B,

R représente un atome d'hydrogène ou un groupe alkyle comportant 1 à 3 atomes de carbone,

$R_1$ et $R_2$, identiques ou différents, représentent un groupe alkyle linéaire ou ramifié comportant 1 à 6 atomes de carbone ou un groupe phényle,

$R_1$ et $R_2$ peuvent également former ensemble avec l'atome d'azote auquel ils sont rattachés un cycle pipéridine,

X représente un groupe $CH-R_3$ ou un atome d'oxygène ou de soufre,

$R_3$ représente un atome d'hydrogène,

m est égal à 0 ou 1,

n est égal à 0, 1 ou 2,

et soit A représente un groupe CH et B un atome d'azote, soit A représente un atome d'azote et B un groupe CH, soit A et B représentent un atome d'azote, étant entendu que lorsque X représente un groupe SO, $SO_2$ ou $N-R_4$ la somme m+n est au moins égale à 1, étant entendu que lorsque A et B représentent chacun un atome d'azote et Z est en position para par rapport à B, X ne peut pas représenter le groupe $CH-R_3$, étant entendu que lorsque A représente un groupe CH, B représente un atome d'azote, Z est en position ortho par rapport à B, X représente un atome d'oxygène et R représente un atome d'hydrogène, la somme m+n est différente de 1 étant entendu que lorsque A représente un groupe CH, B représente un atome d'azote, X représente un groupe $CH_2$ et R représente un atome d'hydrogène, la somme m+n est différente de 2, et à l'exception du N,N-diméthylcarbamate de phényl-2 quinolyle-4, ou un mélange de composés stéréoisomères de formule I ou lorsqu'il peut exister un sel d'un tel composé ou d'un mélange de composés stéréoisomères avec un acide pharmaceutiquement acceptable.

**Revendication par l'Etat Contractant:** AT

Procédé de préparation d'un composé de formule I:

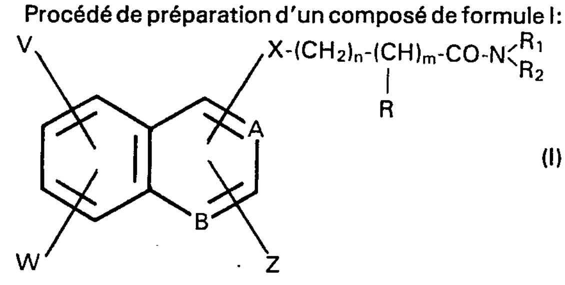

(I)

dans laquelle A représente un atome d'azote ou un groupe CH,

B représente un atome d'azote ou un groupe CH,

V et W, identiques ou différents, représentent des atomes d'hydrogène ou d'halogène, des groupes alkyle ou alcoxy comportant 1 à 3 atomes de carbone, des groupes nitro ou trifluorométhyle,

Z est fixé en position ortho ou para par rapport à B et représente un radical phényle, thiényle, pyridyle ou phényle substitué par un ou deux substituants pris parmi les atomes d'halogène, les groupes alkyle et alcoxy comportant 1 à 4 atomes de carbone, les groupes trifluorométhyle ou nitro,

la chaîne $X-(CH_2)_n-(CHR)_m-CO-NR_1R_2$ est fixée en position ortho ou para par rapport à B,

E représente un atome d'hydrogène ou un groupe alkyle comportant 1 à 3 atomes de carbone,

$R_1$ et $R_2$, identiques ou différents, représentent un groupe alkyle linéaire ou ramifié comportant 1 à 6 atomes de carbone, cycloalkyle comportant 3 à 6 atomes de carbone, phényle, phénylalkyle ou cycloalkylalkyle dont la partie alkyle comporte 1 à 3 atomes de carbone et la partie cycloalkyle comporte 3 à 6 atomes de carbone, alcényle comportant 3 à 6 atomes de carbone à condition que la double liaison ne soit pas située en position 1, 2 par rapport à l'atome d'azote,

$R_1$ et $R_2$ peuvent également former ensemble avec l'atome d'azote auquel ils sont rattachés un cycle pipéridine ou thiomorpholine,

X représente un groupe $CH-R_3$, $N-R_4$, SO, $SO_2$ ou un atome d'oxygène ou de soufre,

$R_3$ représente un atome d'hydrogène ou un groupe alkyle comportant 1 à 3 atomes de carbone,

$R_4$ représente un groupe alkyle comportant 1 à 3 atomes de carbone

m est égal à 0 ou 1,

n est égal à 0, 1 ou 2,

étant entendu que lorsque X représente un groupe SO, $SO_2$ ou $N-R_4$, la somme m + n est au moins égale à 1, étant entendu que lorsque A et B représentent chacun un atome d'azote et Z est en position para par rapport à B, X ne peut pas représenter le groupe $CH-R_3$, étant entendu que lorsque A représente un groupe CH, B représente un atome d'azote, Z est en position ortho par rapport à B, X représente un atome d'oxygène et R représente un atome d'hydrogène, la somme m + n est différente de 1; étant entendu que lorsque A représente un groupe CH, B représente un atome d'azote, X représente un groupe $CH_2$ et R représente un atome d'hydrogène, la somme m + n est différente de 2, et à l'exception du N,N-diméthylcarbamate de phényl-2 quinolyle-4, ses diastéréoisomères, racémiques et énantiomères et lorsqu'il peut exister, son sel d'addition avec un acide caractérisé en ce que:

A - Pour la préparation d'un composé de formule générale I dans laquelle X représente un atome d'oxygène ou de soufre, n et m sont égaux à 0 et A, B, V, W, Z, $R_1$ et $R_2$ sont définis comme précédemment, on fait réagir un composé de formule:

$$Cl-CO-N<^{R_1}_{R_2} \qquad (II)$$

avec un dérivé de formule:

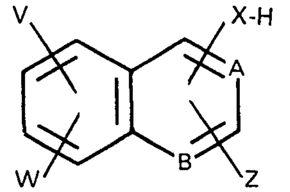

(III)

dans laquelle A, B, V, W, Z ont les mêmes significations que ci-dessus et X représente un atome d'oxygène ou de soufre, isole le produit et le transforme éventuellement en sel d'addition avec un acide,

B - Pour la préparation d'un composé de formule I dans laquelle X représente un atome d'oxygène ou de soufre, n est égal à 0, m est égal à 1, A, B, V, W, Z, R, $R_1$ et $R_2$ sont définis comme précédemment, on fait réagir un composé de formule:

$$Hal-\underset{\displaystyle R}{\underset{|}{CH}}-CO-N<^{R_1}_{R_2} \qquad (IV)$$

sur un dérivé de formule:

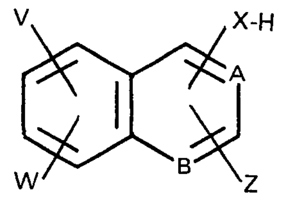

(III)

dans lesquelles Hal représente un atome d'halogène, X représente un atome d'oxygène ou de soufre, A, B, V, W, Z, R, $R_1$ et $R_2$ ont les mêmes significations que ci-dessus, isole le produit et le transforme éventuellement en sel d'addition avec un acide,

C - Pour la préparation d'un composé de formule I dans laquelle X représente un atome d'oxygène ou de soufre ou un groupe $CH-R_3$, n est égal à 0, 1 ou 2 et m est égal à 1, A, B, V, W, Z, R, $R_1$, $R_2$ et $R_3$ sont définis comme précédemment, on traite un composé de formule:

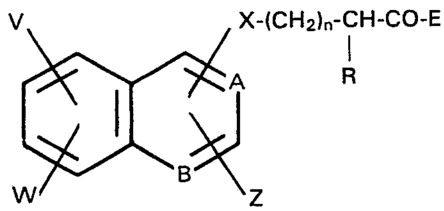

(VI)

avec une amine de formule:

$$HN<^{R_1}_{R_2} \qquad (V)$$

dans lesquelles E représente un groupe alcoxy comportant 1 à 4 atomes de carbone, alcoxycarbonyloxy comportant 2 à 5 atomes de carbone ou un atome de chlore, X est un atome d'oxygène ou de soufre ou un groupe $CH-R_3$, A, B, V, W, Z, R, $R_1$, $R_2$ et n ont les mêmes significations que ci-dessus, isole le produit et le transforme éventuellement en sel d'addition avec un acide,

D - Pour la préparation d'un composé de formule I dans laquelle X représente un groupe sulfinyle ou sulfonyle, m est égal à 1, A, B, V, W, Z, R, $R_1$, $R_2$

et n sont définis comme précédemment, on oxyde les composés correspondants dans lesquels X représente l'atome de soufre, isole le produit et le transforme éventuellement en sel d'addition avec un acide,

E - Pour la préparation d'un composé de formule I dans laquelle A est un groupe CH, B est un atome d'azote, Z est fixé en ortho par rapport à B, la chaîne $X-(CH_2)_n-(CHR)_m-CONR_1R_2$ est fixée en position para par rapport à B, m est égal à 0, n est égal à 0, X représente le groupe $CH-R_3$, $R_3$ représente un groupe alkyle comportant 1 à 3 atomes de carbone, V, W, Z, $R_1$ et $R_2$ sont définis comme précédemment, on alkyle un composé de formule:

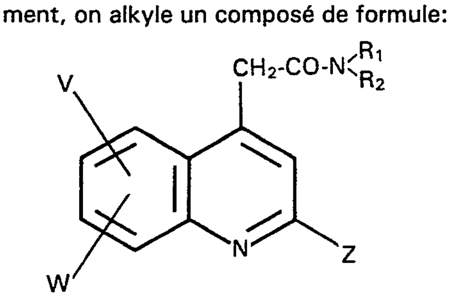

(VIII)

avec un composé de formule $R_3$-Hal

dans lesquelles V, W, Z, $R_1$, $R_2$ et $R_3$ ont les mêmes significations que ci-dessus, et Hal représente un atome d'halogène, isole le produit et le transforme éventuellement en sel d'addition avec un acide,

F - Pour la préparation d'un composé de formule I dans laquelle X représente un groupe $N-R_4$, m est égal à 1, A, B, V, W, Z, R, $R_1$, $R_2$, $R_4$ et n sont définis comme précédemment, on fait réagir un composé de formule:

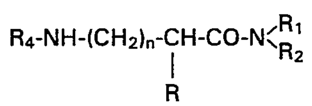

(IX)

sur un dérivé de formule:

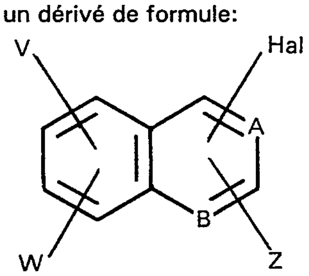

(X)

dans lequelles Hal représente un atome d'halogène, A, B, V, W, X, Z, R, $R_1$, $R_2$, $R_4$ et n ont les mêmes significations que ci-dessus, isole le produit et éventuellement le transforme en sel d'addition avec un acide,

G - Pour la préparation d'un composé de formule I dans laquelle A et B représentent chacun un atome d'azote, Z est fixé en position ortho par rapport à B, la chaîne $X-(CH_2)_n-(CHR)_m-CO-NR_1R_2$ est fixée en position para par rapport à B, X est un groupe $CH_2$, n est égal à 1, m est égal à 0, V, W, $R_1$ et $R_2$ sont définis comme précédemment, on fait agir un chlorure de formule ZCOCl sur un composé de formule:

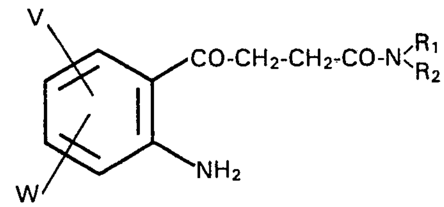

(XI)

dans lesquelles V, W, Z, $R_1$ et $R_2$ ont les mêmes significations que ci-dessus, puis cyclise l'intermédiaire obtenu, isole le produit et le transforme éventuellement en sel d'addition avec un acide.

**Patentansprüche für die Vertragsstaaten:**
BE, CH, DE, FR, GB, IT, LI, LU, NL, SE

1. Racemische oder stereoisomere Verbindung der Formel I

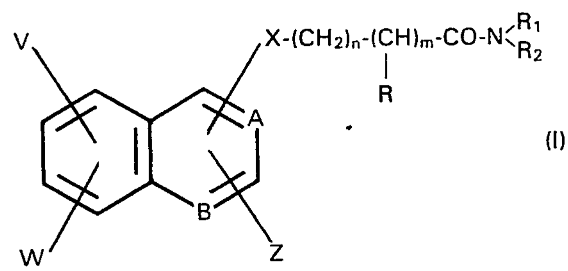

(I)

worin A ein Stickstoffatom oder eine CH-Gruppe bedeutet,

B ein Stickstoffatom oder eine CH-Gruppe bedeutet,

V und W, die identisch oder verschieden sind, Wasserstoff- oder Halogenatome, Alkyl- oder Alkoxygruppen mit 1 bis 3 Kohlenstoffatomen, Nitrogruppen oder Trifluormethyl bedeuten,

Z in ortho- oder para-Stellung in bezug auf B fixiert ist und einen Phenyl-, Thienyl-, Pyridylrest bedeutet oder Phenyl, substituiert durch einen oder zwei Substituenten, ausgewählt unter den Halogenatomen, den Alkyl- und Alkoxygruppen mit 1 bis 4 Kohlenstoffatomen, den Gruppen Trifluormethyl oder Nitro,

die Kette $X-(CH_2)_n-(CHR)_m-CO-NR_1R_2$ in ortho- oder para-Stellung in bezug auf B fixiert ist,

R ein Wasserstoffatom oder eine Alkylgruppe mit 1 bis 3 Kohlenstoffatomen bedeutet,

$R_1$ und $R_2$, die identisch oder verschieden sind, bedeuten eine garade oder verzweigte Alkylgruppe mit 1 bis 6 Kohlenstoffatomen, Cycloalkyl mit 3 bis 6 Kohlenstoffatomen, Phenyl, Phenylalkyl oder Cycloalkylalkyl, dessen Alkylteil 1 bis 3 Kohlenstoffatome trägt, und der Cycloalkylteil trägt 3 bis 6 Kohlenstoffatome, Alkenyl mit 3 bis 6 Kohlenstoffatomen, unter der Bedingung, dass die Doppelbindung nicht in 1,2-Stellung in bezug auf das Stickstoffatom ist,

$R_1$ und $R_2$ können auch zusammen mit dem Stickstoffatom, an das sie gebunden sind, einen Piperidin- oder Thiomorpholinring bilden,

X bedeutet eine Gruppe $CH-R_3$, $N-R_4$, SO oder $SO_2$ oder ein Sauerstoff- oder Schwefelatom,

$R_3$ bedeutet ein Wasserstoffatom oder eine Alkylgruppe mit 1 bis 3 Kohlenstoffatomen,

$R_4$ bedeutet eine Alkylgruppe mit 1 bis 3 Kohlenstoffatomen,

m = 0 oder 1,

n = 0, 1 oder 2,

wobei, falls X eine Gruppe SO, $SO_2$ oder $N-R_4$ bedeutet, die Summe von m + n mindestens gleich 1 ist, wobei, falls A und B jeweils ein Stickstoffatom bedeuten und Z in para-Stellung in bezug auf B ist, X nicht die Gruppe $CH-R_3$ bedeuten kann, wobei, falls A eine Gruppe CH bedeutet, B ein Stickstoffatom darstellt, Z in ortho-Stellung in bezug auf B ist, X ein Sauerstoffatom darstellt und R ein Wasserstoffatom bedeutet, die Summe m + n verschieden von 1 ist; wobei, falls A eine Gruppe CH bedeutet, B ein Stickstoffatom darstellt, X eine Gruppe $CH_2$ bedeutet und R ein Wasserstoffatom darstellt, die Summe m + n verschieden von 2 ist, und mit Ausnahme des 2-Phenylchinolyl-4-N,N-dimethylcarbamats sowie falls ein Salz einer solchen Verbindung mit einer Säure existieren kann.

2. Racemische oder stereoisomere Verbindung gemäss Anspruch 1, wofür V und W, die identisch oder verschieden sind, Wasserstoffatome oder Alkylgruppen mit 1 bis 3 Kohlenstoffatomen bedeuten,

Z in ortho-Stellung in bezug auf B fixiert ist und einen Phenyl-, Thienylrest bedeutet oder Phenyl, substituiert durch einen Substituenten, ausgewählt unter den Alkyl- und Alkoxygruppen mit 1 bis 4 Kohlenstoffatomen, den Gruppen Trifluormethyl oder Nitro,

die Kette $X-(CH_2)_n-(CHR)_m-CO-NR_1R_2$ in para-Stellung in bezug auf B fixiert ist,

R ein Wasserstoffatom oder eine Alkylgruppe mit 1 bis 3 Kohlenstoffatomen bedeutet,

$R_1$ und $R_2$, die identisch oder verschieden sind, eine lineare oder verzweigte Alkylgruppe mit 1 bis 6 Kohlenstoffatomen oder eine Phenylgruppe bedeuten,

$R_1$ und $R_2$ können auch zusammen mit dem Stickstoffatom, an das sie gebunden sind, einen Piperidinring bilden,

X bedeutet eine Gruppe $CH-R_3$ oder ein Sauerstoff- oder Schwefelatom, $R_3$ bedeutet ein Wasserstoffatom,

m = 0 oder 1,

n = 0, 1 oder 2,

und entweder A bedeutet eine Gruppe CH und B ein Stickstoffatom, oder A bedeutet ein Stickstoffatom und B eine Gruppe CH, oder A und B bedeuten ein Stickstoffatom, wobei, falls X eine Gruppe SO, $SO_2$ oder $N-R_4$ bedeutet, die Summe von m + n mindestens gleich 1 ist, wobei, falls A und B jeweils ein Stickstoffatom bedeuten und Z in para-Stellung in bezug auf B ist, X nicht die Gruppe $CH-R_3$ bedeuten kann, wobei, falls A eine Gruppe CH bedeutet, B ein Stickstoffatom bedeutet, Z in ortho-Stellung in bezug auf B steht, X ein Sauerstoffatom bedeutet und R ein Wasserstoffatom darstellt, die Summe m + n verschieden von 1 ist, wobei, falls A eine Gruppe CH bedeutet, B ein Stickstoffatom darstellt, X eine Gruppe $CH_2$ bedeutet und R ein Wasserstoffatom darstellt, die Summe m + n verschieden von 2 ist, und mit Ausnahme des 2-Phenylchinolyl-4-N,N-dimethylcarbamats sowie falls ein Salz einer solchen Verbindung mit einer Säure existieren kann.

3. Verfahren zur Herstellung einer Verbindung gemäss Anspruch 1, in deren Formel X ein Sauerstoff- oder Schwefelatom bedeutet, n und m gleich 0 sind, A, B, V, W, Z, $R_1$, $R_2$ wie in Anspruch 1 definiert sind, dadurch gekennzeichnet, dass man eine Verbindung der Formel II

$$Cl-CO-N<^{R_1}_{R_2} \quad (II)$$

worin $R_1$ und $R_2$ die oben angegebenen Bedeutungen haben, mit einem Derivat der Formel III

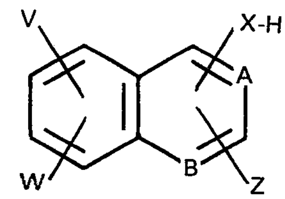

(III)

worin A, B, V, W, Z dieselben Bedeutungen wie vorstehend haben und X ein Sauerstoff- oder Schwefelatom darstellt, zur Umsetzung bringt, das Produkt isoliert und es gegebenenfalls in ein Additionssalz mit einer Säure überführt.

4. Verfahren zur Herstellung einer Verbindung gemäss Anspruch 1, in deren Formel X ein Sauerstoff- oder Schwefelatom bedeutet, n gleich 0 ist, m gleich 1 ist, A, B, V, W, Z, R, $R_1$ und $R_2$ wie in Anspruch 1 definiert sind, dadurch gekennzeichnet, dass man eine Verbindung der Formel IV

$$Hal-\underset{\displaystyle R}{\underset{|}{CH}}-CO-N<^{R_1}_{R_2} \quad (IV)$$

mit einem Derivat der Formel III

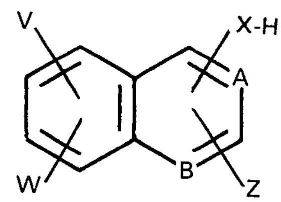

(III)

worin Hal ein Halogenatom bedeutet, X ein Sauerstoff- oder Schwefelatom darstellt, A, B, V, W, Z, R, $R_1$ und $R_2$ dieselben Bedeutungen wir vorstehend haben, zur Umsetzung bringt, das Produkt isoliert und es gegebenenfalls in ein Additionssalz mit einer Säure überführt.

5. Verfahren zur Herstellung einer Verbindung gemäss Anspruch 1, in deren Formel entweder X ein Sauerstoff- oder Schwefelatom darstellt, n gleich 0, 1 oder 2 ist und m gleich 1 ist, oder X eine Gruppe $CH-R_3$ bedeutet, n gleich 0, 1 oder 2 ist und m gleich 0 oder 1 ist, A, B, V, W, Z, R, $R_1$, $R_2$ und $R_3$ wie in Anspruch 1 definiert sind, dadurch gekennzeichnet, dass man eine Verbindung der Formel VI

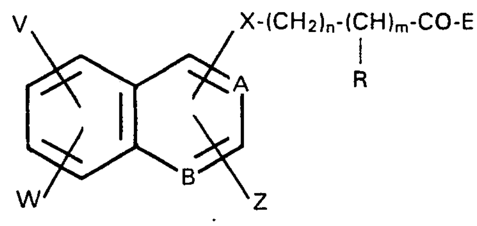

(VI)

mit einem Amin der Formel V

$HN\langle^{R_1}_{R_2}$ (V)

behandelt, worin E eine Alkoxygruppe ist mit 1 bis 4 Kohlenstoffatomen, Alkoxycarbonyloxy mit 2 bis 5 Kohlenstoffatomen, ein Chloratom oder einen N-Imidazolylrest bedeutet, X ein Sauerstoff- oder Schwefelatom oder eine Gruppe $CH-R_3$ ist, A, B, V, W, Z, R, $R_1$, $R_2$, $R_3$, m und n dieselben Bedeutungen wie vorstehend haben, das Produkt isoliert und es gegebenenfalls in ein Additionssalz mit einer Säure überführt.

6. Verfahren zur Herstellung einer Verbindung gemäss Anspruch 1, in deren Formel X eine Sulfinyl- oder Sulfonylgruppe bedeutet, m gleich 1 ist, A, B, V, W, Z, R, $R_1$, $R_2$ und n wie in Anspruch 1 definiert sind, dadurch gekennzeichnet, dass man die entsprechenden Verbindungen, für die X das Schwefelatom darstellt, oxidiert, das Produkt isoliert und es gegebenenfalls in ein Additionssalz mit einer Säure überführt.

7. Verfahren zur Herstellung einer Verbindung gemäss Anspruch 1, in deren Formel A eine Gruppe CH ist, B ein Stickstoffatom darstellt, Z in ortho-Stellung in bezug auf B fixiert ist, die Kette $X-(CH_2)_n-(CHR)_m-CONR_1R_2$ in para-Stellung in bezug auf B fixiert ist, m gleich 0 ist, n gleich 0 ist, X die Gruppe $CH-R_3$ bedeutet, $R_3$ eine Alkylgruppe mit 1 bis 3 Kohlenstoffatomen darstellt, V, W, Z, $R_1$ und $R_2$ wie in Anspruch 1 definiert sind, dadurch gekennzeichnet, dass man eine Verbindung der Formel VIII

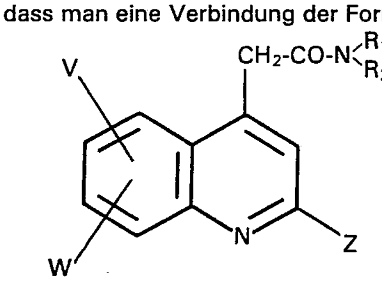

(VIII)

mit einer Verbindung der Formel $R_3$-Hal alkyliert, wobei V, W, Z, $R_1$, $R_2$ und $R_3$ dieselben Bedeutungen wie vorstehend haben und Hal ein Halogenatom bedeutet, das Produkt isoliert und es gegebenenfalls in ein Additionssalz mit einer Säure überführt.

8. Verfahren zur Herstellung einer Verbindung gemäss Anspruch 1, in deren Formel X eine Gruppe $N-R_4$ bedeutet, m gleich 1 ist, A, B, V, W, Z, R, $R_1$, $R_2$, $R_4$ und n wie in Anspruch 1 definiert sind, dadurch gekennzeichnet, dass man eine Verbindung der Formel IX

$$R_4-NH-(CH_2)_n-\underset{\displaystyle R}{\underset{|}{CH}}-CO-N\langle^{R_1}_{R_2} \quad (IX)$$

auf ein Derivat der Formel X

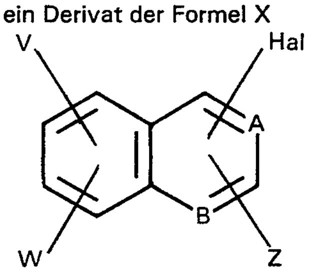

(X)

einwirken lässt, wobei Hal ein Halogenatom bedeutet, A, B, V, W, Z, R, $R_1$, $R_2$, $R_4$ und n dieselben Bedeutungen wie vorstehend haben, dass man das Produkt isoliert und es gegebenenfalls in ein Additionssalz mit einer Säure überführt.

9. Verfahren zur Herstellung einer Verbindung gemäss Anspruch 1, in deren Formel A und B jeweils ein Stickstoffatom darstellen, Z in ortho-Stellung in bezug auf B fixiert ist, die Kette $X-(CH_2)_n-(CHR)_m-CO-NR_1R_2$ in para-Stellung in bezug auf B fixiert ist, X eine $CH_2$-Gruppe ist, n gleich 1 ist, m gleich 0 ist, V, W, $R_1$, $R_2$ wie in Anspruch 1 definiert sind, dadurch gekennzeichnet, dass man ein Chlorid der Formel ZCOCl auf eine Verbindung der Formel XI

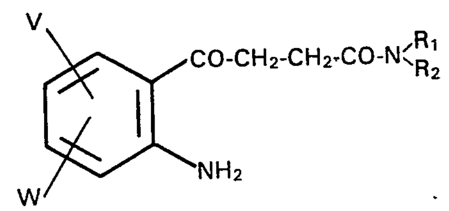

(XI)

einwirkeen lässt, wobei V, W, Z, $R_1$ und $R_2$ dieselben Bedeutungen wie vorstehend haben, dass man das so erhaltenen Zwischenprodukt dann cyclisiert, das Produkt isoliert und es gegebenenfalls in ein Additionssalz mit einer Säure überführt.

10. Arzneimittel, enthaltend mindestens eine aktive Substanz und gegebenenfalls ein oder mehrere verträgliche und pharmazeutisch annehmbare Verdünnungs- oder Hilfsmittel, dadurch gekennzeichnet, dass die aktive Substanz eine Verbindung der Formel I

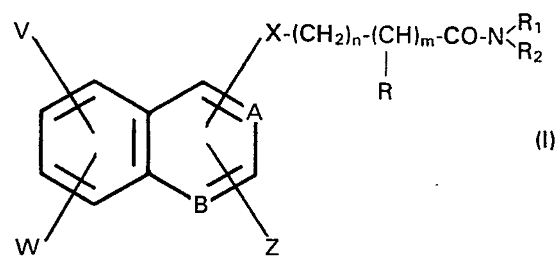

(I)

ist, worin A ein Stickstoffatom oder eine Gruppe CH ist,

B ein Stickstoffatom oder eine Gruppe CH bedeutet,

V und W, identisch oder verschieden sind, Wasserstoff- oder Halogenatome, Alkyl- oder Alkoxygruppen mit 1 bis 3 Kohlenstoffatomen, Nitrogruppen oder Trifluormethyl bedeuten,

Z in ortho- oder para-Stellung in bezug auf B fixiert ist und einen Phenyl-, Thienyl-, Pyridylrest oder Phenyl, substituiert durch einen oder zwei Substituenten, ausgewählt unter den Halogenatomen, den Alkyl- oder Alkoxygruppen mit 1 bis 4 Kohlenstoffatomen, den Gruppen Trifluormethyl oder Nitro bedeutet,

die Kette $X-(CH_2)_n-(CHR)_m-CO-NR_1R_2$ in ortho- oder para-Stellung in bezug auf B fixiert ist,

R ein Wasserstoffatom oder eine Alkylgruppe mit 1 bis 3 Kohlenstoffatomen. bedeutet,

$R_1$ und $R_2$, die identisch oder verschieden sind,

eine gerade oder verzweigte Alkylgruppe mit 1 bis 6 Kohlenstoffatomen bedeuten, Cycloalkyl mit 3 bis 6 Kohlenstoffatomen, Phenyl, Phenylalkyl oder Cycloalkylalkyl, dessen Alkylteil 1 bis 3 Kohlenstoffatome trägt und der Cycloalkylteil 3 bis 6 Kohlenstoffatome hat, Alkenyl mit 3 bis 6 Kohlenstoffatomen, unter der Bedingung, dass die Doppelbindung nicht in 1,2-Stellung in bezug auf das Stickstoffatom steht,

$R_1$ und $R_2$ können auch zusammen mit dem Stickstoffatom, an das sie gebunden sind, einen Piperidin- oder Thiomorpholinring bilden,

X bedeutet eine Gruppe $CH-R_3$, $N-R_4$, SO oder $SO_2$ oder ein Sauerstoff- oder Schwefelatom,

$R_3$ bedeutet ein Wasserstoffatom oder eine Alkylgruppe mit 1 bis 3 Kohlenstoffatomen,

$R_4$ bedeutet eine Alkylgruppe mit 1 bis 3 Kohlenstoffatomen,

m = 0 oder 1,

n = 0, 1 oder 2,

wobei, falls X eine Gruppe SO, $SO_2$ oder $N-R_4$ bedeutet, die Summe m+n mindestens gleich 1 ist, wobei falls A und B jeweils ein Stickstoffatom bedeuten und Z in para-Stellung in bezug auf B steht, X nicht die Gruppe $CH-R_3$ darstellen kann, wobei, falls A eine Gruppe CH bedeutet, B ein Stickstoffatom darstellt, Z in ortho-Stellung in bezug auf B ist, X ein Sauerstoffatom darstellt und R ein Wasserstoffatom darstellt, die Summe m+n verschieden von 1 ist; wobei, falls A eine Gruppe CH bedeutet, B ein Stickstoffatom darstellt, X eine Gruppe $CH_2$ bedeutet und R ein Wasserstoffatom darstellt, die Summe m+n verschieden von 2 ist, mit der Ausnahme von 2-Phenylchinolyl-4-N,N-dimethylcarbamat, oder ein Gemisch der stereoisomeren Verbindungen der Formel I oder, falls ein Salz einer solchen Verbindung existieren kann oder ein Gemisch der stereoisomeren Verbindungen mit einer pharmazeutisch annehmbaren Säure.

11. Arzneimittel gemäss Anspruch 10, enthaltend eine Verbindung der Formel I, worin V und W, die identisch oder verschieden sind, Wasserstoffatome oder Alkylgruppen mit 1 bis 3 Kohlenstoffatomen bedeuten,

Z in ortho-Stellung in bezug auf B fixiert ist und einen Phenyl-, Thienylrest bedeutet oder Phenyl, substituiert durch einen Substituenten, ausgewählt unter den Alkyl- und Alkoxygruppen mit 1 bis 4 Kohlenstoffatomen, den Gruppen Trifluormethyl oder Nitro,

die Kette $X-(CH_2)_n-(CHR)_m-CO-NR_1R_2$ in para-Stellung in bezug auf B fixiert ist,

R ein Wasserstoffatom oder eine Alkylgruppe mit 1 bis 3 Kohlenstoffatomen bedeutet,

$R_1$ und $R_2$, die identisch oder verschieden sind, eine gerade oder verzweigte Alkylgruppe mit 1 bis 6 Kohlenstoffatomen oder eine Phenylgruppe darstellt,

$R_1$ und $R_2$ können auch zusammen mit dem Stickstoffatom, an das sie gebunden sind, einen Piperidinring bilden,

X bedeutet eine Gruppe $CH-R_3$ oder ein Sauerstoff- oder Schwefelatom,

$R_3$ bedeutet ein Wasserstoffatom,

m = 0 oder 1,

n = 0, 1 oder 2,

und entweder A bedeutet eine Gruppe CH und B ein Stickstoffatom, oder A bedeutet ein Stickstoffatom und B eine Gruppe CH, oder A und B bedeuten ein Stickstoffatom, wobei falls X eine Gruppe SO, $SO_2$ oder $N-R_4$ bedeutet, die Summe m+n mindestens gleich 1 ist, wobei, falls A und B jeweils ein Stickstoffatom bedeuten und Z in para-Stellung in bezug auf B ist, X nicht die Gruppe $CH-R_3$ bedeuten kann, wobei, falls A eine Gruppe CH bedeutet, B ein Stickstoffatom darstellt, Z in ortho-Stellung in bezug auf B ist, X ein Stickstoffatom und R ein Wasserstoffatom darstellt, die Summe m+n verschieden von 1 ist, wobei, falls A eine Gruppe CH bedeutet, B ein Stickstoffatom bedeutet, X eine Gruppe $CH_2$ darstellt und R ein Wasserstoffatom bedeutet, die Summe m+n verschieden von 2 ist, und mit Ausnahme des 2-Phenylchinolyl-4-N,N-dimethylcarbamats, oder ein Gemisch der stereoisomeren Verbindungen der Formel I, oder falls ein Salz einer solchen Verbindung oder eines Gemisches der stereosiomeren Verbindungen mit einer pharmazeutisch annehmbaren Säure existieren kann.

**Patentanspruch für den Vertragsstaat:** AT

Verfahren zur Herstellung einer Verbindung der Formel I

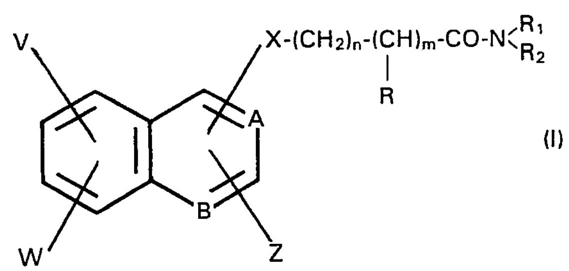

(I)

in welcher A ein Stickstoffatom oder eine CH-Gruppe darstellt,

B ein Stickstoffatom oder eine CH-Gruppe darstellt,

V und W unabhängig voneinander Wasserstoff- oder Halogenatome, Alkyl- oder Alkoxygruppen mit 1 bis 3 Kohlenstoffatomen, Nitro- oder Trifluormethylgruppen darstellen,

Z in ortho- oder para-Stellung in Bezug auf B gebunden ist und einen Phenyl-, Thienyl-, Pyridyl- oder einen durch einen oder zwei Substituenten, ausgewählt aus den Halogenatomen, den Alkyl- und Alkoxygruppen mit 1 bis 4 Kohlenstoffatomen, den Trifluormethyl- oder Nitrogruppen, substituierten Phenylrest darstellt,

die Kette $X-(CH_2)_n-(CHR)_m-CO-NR_1R_2$ in ortho- oder para-Stellung in bezug auf B gebunden ist,

R ein Wasserstoffatom oder eine Alkylgruppe mit 1 bis 3 Kohlenstoffatomen darstellt,

$R_1$ und $R_2$ unabhängig voneinander eine geradkettige oder verzweigte Alkylgruppe mit 1 bis 6 Kohlenstoffatomen, eine Cycloalkylgruppe mit 3 bis 6 Kohlenstoffatomen, eine Phenyl-, Phenylalkyl- oder Cycloalkylalkylgruppe, deren Alkylteil 1 bis 3 Kohlenstoffatome und deren Cycloalkylteil 3 bis 6 Kohlenstoffatome enthält, eine Alkenylgruppe mit 3 bis 6

Kohlenstoffatomen, vorausgesetzt, die Doppelbindung befindet sich nicht in 1,2-Stellung in bezug auf das Stickstoffatom, darstellen,

$R_1$ und $R_2$ können auch zusammenmit dem Stickstoffatom, an das sie gebunden sind, einen Piperidin- oder Thiomorpholinring darstellen,

X eine Gruppe $CH-R_3$, $N-R_4$, SO oder $SO_2$ oder ein Sauerstoff- oder Schwefelatom darstellt,

$R_3$ ein Wasserstoffatom oder eine Alkylgruppe mit 1 bis 3 Kohlenstoffatomen darstellt,

$R_4$ eine Alkylgruppe mit 1 bis 3 Kohlenstoffatomen darstellt,

m gleich 0 oder 1 ist,

n gleich 0, 1 oder 2 ist,

wobei die Summe m + n selbstverständlich zumindest gleich 1 ist, wenn X eine Gruppe SO, $SO_2$ oder $N-R_4$ darstellt, wobei X selbstverständlich nicht die Gruppe $CH-R_3$ darstellen kann, wenn A und B jeweils ein Stickstoffatom darstellen und Z sich in para-Stellung in bezug auf B befindet, wobei die Summe m + n selbstverständlich von 1 verschieden ist, wenn A eine Gruppe darstellt, B ein Stickstoffatom darstellt, Z in ortho-Stellung zu B gebunden ist, X ein Sauerstoffatom darstellt und R ein Wasserstoffatom darstellt; wobei die Summe m + n selbstverständlich von 2 verschieden ist, wenn A eine CH-Gruppe darstellt, B ein Stickstoffatom darstellt, X eine $CH_2$-Gruppe darstellt und R ein Wasserstoffatom darstellt, ausgenommen 2-Phenyl-4-chinolyl-N,N-dimethylcarbamat, ihrer Diastereoisomeren, racemischen Formen und Enantiomeren und, falls existent, ihres Säureadditionssalzes, dadurch gekennzeichnet, dass man:

A - zur Herstellung einer Verbindung der allgemeinen Formel I, in welcher X ein Stickstoff- oder Schwefelatom darstellt, n und m gleich 0 sind und A, B, V, W, Z, $R_1$ und $R_2$ die obige Bedeutung haben, eine Verbindung der Formel

$$Cl-CO-N\begin{matrix} R_1 \\ R_2 \end{matrix} \quad (II)$$

mit einer Verbindung der Formel

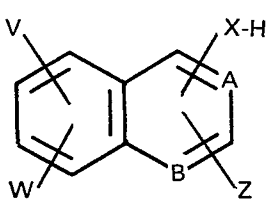

(III)

in welcher A, B, V, W, Z die obige Bedeutung haben und X ein Sauerstoff- oder Schwefelatom darstellt, reagieren lässt, das Produkt isoliert und es gegebenenfalls in ein Säureadditionssalz umwandelt,

B - zur Herstellung einer Verbindung der Formel I, in welcher X ein Sauerstoff- oder Schwefelatom darstellt, n gleich 0 ist, m gleich 1 ist, A, B, V, W, Z, R, $R_1$ und $R_2$ die obige Bedeutung haben, eine Verbindung der Formel

$$Hal-\underset{R}{\underset{|}{CH}}-CO-N\begin{matrix} R_1 \\ R_2 \end{matrix} \quad (IV)$$

mit einer Verbindung der Formel

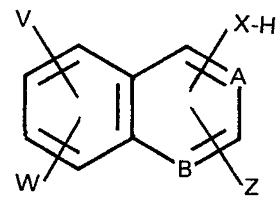

(III)

in welchen Hal ein Halogenatom darstellt, X ein Sauerstoff- oder Schwefelatom darstellt, A, B, V, W, Z, R, $R_1$ und $R_2$ die obigen Bedeutungen haben, umsetzt, das Produkt isoliert und es gegebenenfalls in ein Säureadditionssalz umwandelt,

C - zur Herstellung einer Verbindung der Formel I, in welcher X ein Sauerstoff- oder Schwefelatom oder eine Gruppe $CH-R_3$ darstellt, n gleich 0, 1 oder 2 ist und m gleich 1 ist, A, B, V, W, Z, R, $R_1$, $R_2$ und $R_3$ die obige Bedeutung haben, eine Verbindung der Formel

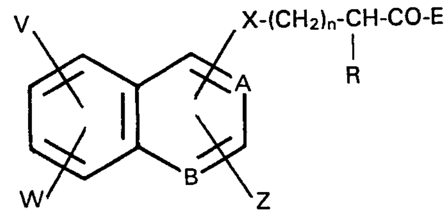

(VI)

mit einem Amin der Formel

$$HN\begin{matrix} R_1 \\ R_2 \end{matrix} \quad (V)$$

in welchen E eine Alkoxygruppe mit 1 bis 4 Kohlenstoffatomen, Alkoxycarbonyloxygruppe mit 2 bis 5 Kohlenstoffatomen oder ein Chloratom darstellt, X ein Sauerstoff- oder Schwefelatom oder eine Gruppe $CH-R_3$ ist, A, B, V, W, Z, R, $R_1$, $R_2$ und n die obigen Bedeutungen haben, behandelt, das Produkt isoliert und es gegebenenfalls in ein Säureadditionssalz umwandelt,

D - zur Herstellung einer Verbindung der Formel I, in welcher X eine Sulfinyl- oder Sulfonylgruppe darstellt, m gleich 1 ist, A, B, V, W, Z, R, $R_1$, $R_2$ und n die obige Bedeutung haben, die entsprechenden Verbindungen, in welchen W das Schwefelatom darstellt, oxidiert, das Produkt isoliert und es gegebenenfalls in ein Säureadditionssalz umwandelt,

E - zur Herstellung einer Verbindung der Formel I, in welcher A eine CH-Gruppe ist, B ein Stickstoffatom ist, Z in ortho-Stellung in bezug auf B gebunden ist, die Kette $X-(CH_2)_n-(CHR)_m-CO-NR_1R_2$ in para-Stellung in bezug auf B gebunden ist, m gleich 0 ist, n gleich 0 ist, X die Gruppe $CH-R_3$ darstellt, $R_3$ eine Alkylgruppe mit 1 bis 3 Kohlenstoffatomen darstellt, V, W, Z, $R_1$ und $R_2$ die obige Bedeutung haben, eine Verbindung der Formel

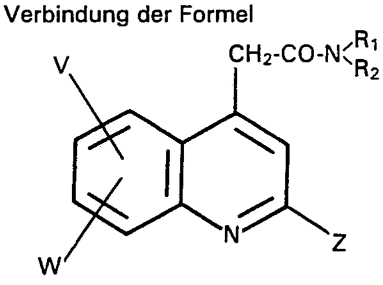

(VIII)

mit einer Verbindung der Formel $R_3$-Hal, in welchen V, W, Z, $R_1$, $R_2$ und $R_3$ die obigen Bedeutungen haben und Hal ein Halogenatom darstellt, alkyliert, das Produkt isoliert und es gegebenenfalls in ein Säureadditionssalz umwandelt,

F - zur Herstellung einer Verbindung der Formel I, in welcher X eine Gruppe N-$R_4$ darstellt, m gleich 1 ist, A, B, V, W, Z, R, $R_1$, $R_2$, $R_4$ und n die obige Bedeutung haben, eine Verbindung der Formel

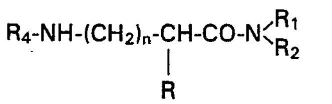

(IX)

mit einer Verbindung der Formel

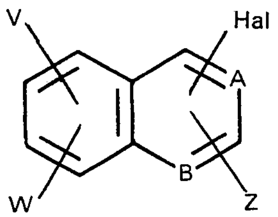

(X)

in welchen Hal ein Halogenatom darstellt, A, B, V, W, Z, R, $R_1$, $R_2$, $R_4$ und n die obigen Bedeutungen haben, reagieren lässt, das Produkt isoliert und es gegebenenfalls in ein Säureadditionssalz umwandelt,

G - zur Herstellung einer Verbindung der Formel I, in welcher A und B jeweils ein Stickstoffatom darstellen, Z in ortho-Stellung in bezug auf B gebunden ist, die Kette X-$(CH_2)_n$-$(CHR)_m$-CO-$NR_1R_2$ in para-Stellung in bezug auf B gebunden ist, X eine CH-2-Gruppe ist, n gleich 1 ist, m gleich 0 ist, V, W, $R_1$ und $R_2$ die obige Bedeutung haben, ein Chlorid der Formel ZCOCl mit einer Verbindung der Formel

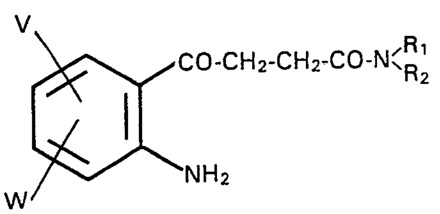

(XI)

in welchen V, W, Z, $R_1$ und $R_2$ die obigen Bedeutungen haben, reagieren lässt, dann das erhaltene Zwischenprodukt cyclisiert, das Produkt isoliert und es gegebenenfalls in ein Säureadditionssalz umwandelt.

**Claims for the Contracting States:**
BE, CH, DE, FR, GB, IT, LI, LU, NL, SE

1. A racemic or stereoisomeric compound of formula I

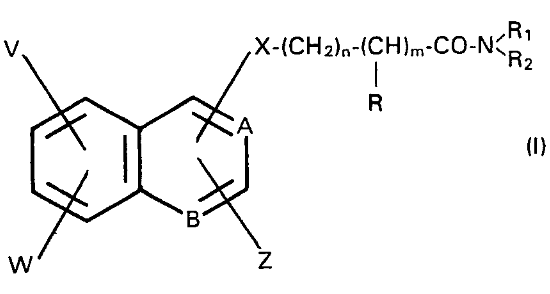

(I)

in which A denotes a nitrogen atom or a CH group,

B denotes a nitrogen atom or a CH group,

V and W, which may be identical or different, denote hydrogen or halogen atoms, alkyl or alkoxy groups containing 1 to 3 carbon atoms, or nitro or trifluoromethyl groups,

Z is bound in the ortho or para position with respect to B and denotes a phenyl, thienyl or pyridyl radical or a phenyl radical substituted with one or two substituents chosen from halogen atoms, alkyl and alkoxy groups containing 1 to 4 carbon atoms and trifluoromethyl or nitro groups,

the chain X-$(CH_2)_n$-$(CHR)_m$-CO-$NR_1R_2$ is bound in the ortho or para position with respect to B,

R denotes a hydrogen atom or an alkyl group containing 1 to 3 carbon atoms,

$R_1$ and $R_2$, which may be identical or different, denote a linear or branched alkyl group containing 1 to 6 carbon atoms, a cycloalkyl group containing 3 to 6 carbon atoms, a phenyl group, a phenylalkyl or cycloalkylalkyl group in which the alkyl portion contains 1 to 3 carbon atoms and the cycloalkyl portion contains 3 to 6 carbon atoms or an alkenyl group containing 3 to 6 carbon atoms on condition that the double bond is not situated in the 1,2 position with respect to the nitrogen atom,

$R_1$ and $R_2$ can also form, together with the nitrogen atom to which they are attached, a piperidine or thiomorpholine ring,

X denotes a group CH-$R_3$, N-$R_4$, SO or $SO_2$, or an oxygen or sulphur atom,

$R_3$ denotes a hydrogen atom or an alkyl group containing 1 to 3 carbon atoms,

$R_4$ denotes an alkyl group containing 1 to 3 carbon atoms,

m equals 0 or 1, and

n equals 0, 1 or 2,

with the proviso that, when X denotes a group SO, $SO_2$ or N-$R_4$, the sum m+n is equal to at least 1, with the proviso that, when A and B each denote a nitrogen atom and Z is in the para position with respect to B, X cannot denote the group CH-$R_3$, with the proviso that, when A denotes a CH group, B denotes a nitrogen atom, Z is in the ortho position with respect to B, X denotes an oxygen atom and R denotes a hydrogen atom, the sum m+n is other than 1; with the proviso that, when A denotes a CH group, B denotes a nitrogen atom, X denotes a $CH_2$ group and R denotes a hydrogen atom, the sum m+n is other than 2, and with the exception of 2-phenyl-4-quinolyl N,N-dimethylcarbamate, and also, where it can exist, a salt of such compound with an acid.

2. A racemic or stereoisomeric compound according to Claim 1 for which V and W, which may be identical or different, denote hydrogen atoms or alkyl groups containing 1 to 3 carbon atoms,

Z is bound in the ortho position with respect to B and denotes a phenyl radical optionally substituted with an alkyl group, an alkoxy group containing 1 to 4 carbon atoms, a nitro or trifluoromethyl group or a thienyl radical,

the chain X-$(CH_2)_n$-$(CHR)_m$-CO-$NR_1R_2$ is bound in the para position with respect to B,

R denotes a hydrogen atom or an alkyl group containing 1 to 3 carbon atoms,

$R_1$ and $R_2$, which may be identical or different, denote a linear or branched alkyl group containing 1 to 6 carbon atoms or a phenyl group,

$R_1$ and $R_2$ can also form, together with the nitrogen atom to which they are attached, a piperidine ring,

X denotes a group $CH\text{-}R_3$ or an oxygen or sulphur atom, $R_3$ denotes a hydrogen atom,

m equals 0 or 1,

n equals 0, 1 or 2,

and either A denotes a CH group and B a nitrogen atom, or A denotes a nitrogen atom and B a CH group, or A and B denote a nitrogen atom, with the proviso that, when X denotes a group SO, $SO_2$ or $N\text{-}R_4$, the sum m+n is equal to at least 1, with the proviso that, when A and B each denote a nitrogen atom and Z is in the para position with respect to B, X cannot denote the group $CH\text{-}R_3$, with the proviso that, when A denotes a CH group, B denotes a nitrogen atom, Z is in the ortho position with respect to B, X denotes an oxygen atom and R denotes a hydrogen atom, the sum m+n is other than 1, with the proviso that when A denotes a CH group, B denotes a hydrogen atom, the sum m+n is other than 2, and with the exception of 2-phenyl-4-quinolyl N,N-dimethylcarbamate, and also, where it can exist, a salt of such a compound with an acid.

3. A process for preparing a compound according to Claim 1 in the formula of which X denotes an oxygen or sulphur atom, n and m are equal to 0 and A, B, V, W, Z, $R_1$ and $R_2$ are defined as in Claim 1, characterized in that a compound of formula

$$Cl\text{-}CO\text{-}N\begin{matrix}R_1\\R_2\end{matrix} \qquad (II)$$

in which $R_1$ and $R_2$ have the same meanings as above, is reacted with a derivative of formula

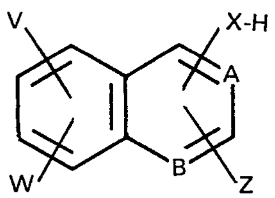

(III)

in which A, B, V, W and Z have the same meanings as above and X denotes on oxygen or sulphur atom, and the product is isolated and optionally converted to an addition salt with an acid.

4. A process for preparing a compound according to Claim 1 in the formula of which X denotes an oxygen or sulphur atom, n equals 0, m equals 1 and A, B, V, W, Z, R, $R_1$ and $R_2$ are defined as in Claim 1, characterized in that a compound of formula

$$Hal\text{-}\underset{\displaystyle R}{\underset{|}{CH}}\text{-}CO\text{-}N\begin{matrix}R_1\\R_2\end{matrix} \qquad (IV)$$

is reacted with a derivative of formula

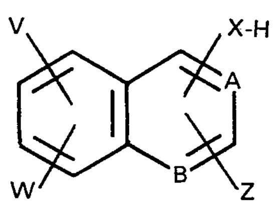

(III)

in which Hal denotes a halogen atom, X denotes an oxygen or sulphur atom and A, B, V, W, Z, R, $R_1$ and $R_2$ have the same meanings as above, and the product is isolated and optionally converted to an addition salt with an acid.

5. A process for preparing a compound according to Claim 1 in the formula of which either X denotes an oxygen or sulphur atom, n equals 0, 1 or 2 and m equals 1, or X denotes a group $CH\text{-}R_3$, n equals 0, 1 or 2 and m equals 0 or 1, and A, B, V, W, Z, R, $R_1$, $R_2$ and $R_3$ are defined as in Claim 1, characterized in that a compound of formula

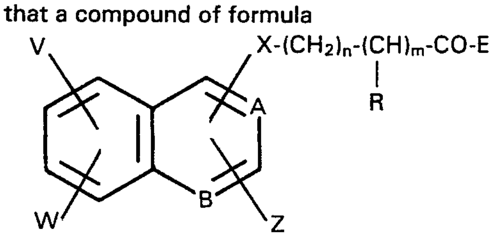

(VI)

is treated with an amine of formula

$$HN\begin{matrix}R_1\\R_2\end{matrix} \qquad (V)$$

in which E denotes an alkoxy group containing 1 to 4 carbon atoms, an alkoxycarbonyloxy group containing 2 to 5 carbon atoms, a chlorine atom, or an N-imidazolyl residue, X is an oxygen or sulphur atom or a group $CH\text{-}R_3$, and A, B, V, W, Z, R, $R_1$, $R_2$, $R_3$, m and n have the same meanings as above, and the product is isolated and optionally converted to an addition salt with an acid.

6. A process for preparing a compound according to Claim 1 in the formula of which X denotes a sulphinyl or sulphonyl group, m equals 1, and A, B, V, W, Z, R, $R_1$, $R_2$ and n are defined as in Claim 1, characterized in that the corresponding compounds for which X denotes a sulphur atom are oxidized, and the product is isolated and optionally converted to an addition salt with an acid.

7. A process for preparing a compound according to Claim 1 in the formula of which A is a CH group, B is a nitrogen atom, Z is bound in the ortho position with respect to B, the chain $X\text{-}(CH_2)_n\text{-}(CHR)_m\text{-}CONR_1R_2$ is bound in the para position with respect to B, m equals 0, n equals 0, X denotes a group $CH\text{-}R_3$, $R_3$ denotes an alkyl group containing 1 to 3 carbon atoms and V, W, Z, $R_1$ and $R_2$ are defined as in Claim 1, characterized in that a compound of formula

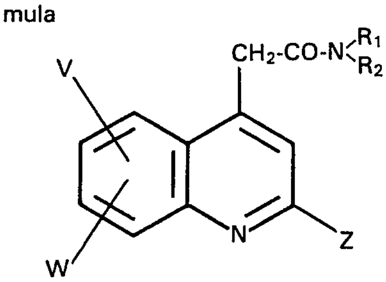

(VIII)

is alkylated with a compound of formula $R_3\text{-}Hal$ in which V, W, Z, $R_1$, $R_2$ and $R_3$ have the same meanings as above and Hal denotes a halogen atom, and the product is isolated and optionally converted to an addition salt with an acid.

8. A process for preparing a compound according to Claim 1 in the formula of which X denotes a group $N-R_4$, m equals 1, and A, B, V, W, Z, R, $R_1$, $R_2$, $R_4$ and n are defined as in Claim 1, characterized in that a compound of formula

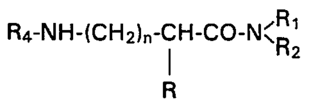

(IX)

is reacted with a derivative of formula

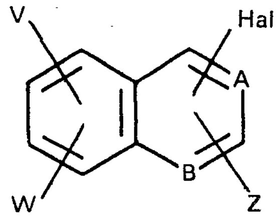

(X)

in which Hal denotes a halogen atom and A, B, V, W, Z, R, $R_1$, $R_2$, $R_4$ and n have the same meanings as above, and the product is isolated and optionally converted to an addition salt with an acid.

9. A process for preparing a compound according to Claim 1 in the formula of which A and B each denote a nitrogen atom, Z is bound in the ortho position with respect to B, the chain $X-(CH_2)_n-(CHR)_m-CO-NR_1R_2$ is bound in the para position with respect to B, X is a $CH_2$ group, n equals 1, m equals 0, and V, W, $R_1$ and $R_2$ are defined as in Claim 1, characterized in that a chloride of formula ZCOCl is reacted with a compound of formula

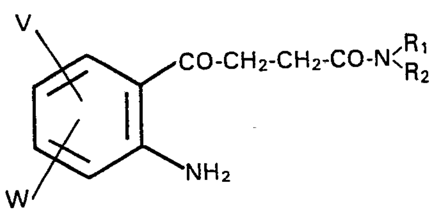

(XI)

in which V, W, Z, $R_1$ and $R_2$ have the same meanings as above, the intermediate thereby obtained is cyclized and the product is isolated and optionally converted to an addition salt with an acid.

10. A medicament containing at least one acitve substance and optionally one or more diluents or adjuvants which are compatible and pharmaceutically acceptable, in which the active substance is a compound of formula I

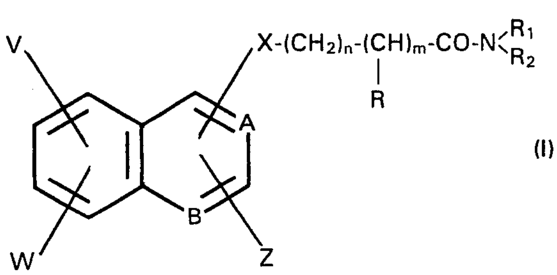

(I)

in which A denotes a nitrogen atom or a CH group,

B denotes a nitrogen atom or a CH group,

V and W, which may be identical or different, denote hydrogen or halogen atoms, alkyl or alkoxy groups containing 1 to 3 carbon atoms, or nitro or trifluoromethyl groups,

Z is bound in the ortho or para position with respect to B and denotes a phenyl, thienyl or pyridyl radical or a phenyl radical substituted with one or two substituents chosen from halogen atoms, alkyl and alkoxy groups containing 1 to 4 carbon atoms and trifluoromethyl or nitro groups,

the chain $X-(CH_2)_n-(CHR)_m-CO-NR_1R_2$ is bound in the ortho position or para position with respect to B,

R denotes a hydrogen atom or an alkyl group containing 1 to 3 carbon atoms,

$R_1$ and $R_2$, which may be identical or different, denote a linear or branched alkyl group containing 1 to 6 carbon atoms, a cycloalkyl group containing 3 to 6 carbon atoms, a phenyl group, a phenylalkyl or cycloalkylalkyl group in which the alkyl portion contains 1 to 3 carbon atoms and the cycloalkyl portion contains 3 to 6 carbon atoms or an alkenyl group containing 3 to 6 carbon atoms on condition that the double bond is not situated in the 1,2 position with respect to the nitrogen atom,

$R_1$ and $R_2$ can also form, together with the nitrogen atom to which they are attached, a piperidine or thiomorpholine ring,

X denotes a group $CH-R_3$, $N-R_4$, SO or $SO_2$, or an oxygen or sulphur atom,

$R_3$ denotes a hydrogen atom or an alkyl group containing 1 to 3 carbon atoms,

$R_4$ denotes an alkyl group containing 1 to 3 carbon atoms,

m equals 0 or 1, and

n equals 0, 1 or 2,

with the proviso that, when X denotes a group SO, $SO_2$ or $N-R_4$, the sum $m+n$ is equal to at least 1, with the proviso that, when A and B each denote a nitrogen atom and Z is in the para position with respect to B, X cannot denote the group $CH-R_3$, with the proviso that, when A denotes a CH group, B denotes a nitrogen atom, Z is the ortho position with respect to B, X denotes an oxygen atom and R denotes a hydrogen atom, the sum $m+n$ is other than 1; with the proviso that, when A denotes a CH group, B denotes a nitrogen atom, X denotes a $CH_2$ group and R denotes a hydrogen atom, the sum $m+n$ is other than 2, and with the exception of 2-phenyl-4-quinolyl N,N-dimethylcarbamate, or a mixture of stereoisomeric compounds of formula I or, where it can exist, a salt of such a compound or of a mixture of stereoisomeric compounds with a pharmaceutically acceptable acid.

11. A medicament according to Claim 10 containing a compound of the formula I in which V and W, which may be identical or different, denote hydrogen atoms or alkyl groups containing 1 to 3 carbon atoms,

Z is bounded in the ortho position with respect to B and denotes a phenyl radical optionally with an alkyl group, an alkoxy group containing 1 to 4 carbon atoms, a nitro or trifluoromethyl group or a thienyl radical,

the chain $X-(CH_2)_n-(CHR)_m-CO-NR_1R_2$ is bound in the para position with respect to B,

R denotes a hydrogen atom or an alkyl group containing 1 to 3 carbon atoms,

$R_1$ and $R_2$, which may be identical or different, denote a linear or branched alkyl group containing 1 to 6 carbon atoms or a phenyl group,

$R_1$ and $R_2$ can also form, together with the nitrogen atom to which they are attached, a piperidine ring,

X denotes a group $CH-R_3$ or an oxygen or sulphur atom,

$R_3$ denotes a hydrogen atom,

m equals 0 or 1,

n equals 0, 1 or 2,

and either A denotes a CH group and B a nitrogen atom, or A denotes a nitrogen atom and B a CH group, or A and B denote a nitrogen atom, with the proviso that, when X denotes a group SO, $SO_2$ or $N-R_4$, the sum m+n is equal to at least 1, with the proviso that, when A and B each denote a nitrogen atom and Z is in the para position with respect to B, X cannot denote the group $CH-R_3$, with the proviso that, when A denotes a CH group, B denotes a nitrogen atom, Z is in the ortho position with respect to B, X denotes an oxygen atom and R denotes a hydrogen atom, the sum m+n is other than 1, with the proviso that when A denotes a CH group, B denotes a nitrogen atom, X denotes a $CH_2$ group and R denotes a hydrogen atom, the sum m+n is other than 2, and with the exception of 2-phenyl-4-quinolyl N,N-dimethylcarbamate, or a mixture of stereosiomeric compounds of formula I or, where it can exist, a salt of such a compound or of a mixture of stereosiomeric compounds with a pharmaceutically acceptable acid.

**Claim for the Contracting State:** AT

Process for the praparation of a compound of formula I

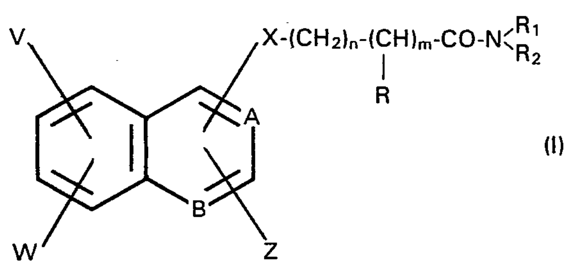

(I)

in which A denotes a nitrogen atom or a CH group,

B denotes a nitrogen atom or a CH group,

V and W, which may be identical or different, denote hydrogen or halogen atoms, alkyl or alkoxy groups containing 1 to 3 carbon atoms, or nitro or trifluoromethyl groups,

Z is bound in the ortho or para position with respect to B and denotes a phenyl, thienyl or pyridyl radical or a phenyl radical substituted with one or two substituents chosen from halogen atoms, alkyl and alkoxy groups containing 1 to 4 carbon atoms and trifluoromethyl or nitro groups,

the chain $X-(CH_2)_n-(CHR)_m-CO-NR_1R_2$ is bound in the ortho or para position with respect to B,

R denotes a hydrogen atom or an alkyl group containing 1 to 3 carbon atoms,

$R_1$ and $R_2$, which may be identical or different, denote a linear or branched alkyl group containing 1 to 6 carbon atoms, a cycloalkyl group containing 3 to 6 carbon atoms, a phenyl group, a phenylalkyl or cycloalkylalkyl group in which the alkyl portion contains 1 to 3 carbon atoms and the cycloalkyl portion contains 3 to 6 carbon atoms or an alkenyl group containing 3 to 6 carbon atoms on condition that the double bond is not situated in the 1,2 position with respect to the nitrogen atom,

$R_1$ and $R_2$ can also form, together with the nitrogen atom to which they are attached, a piperidine or thiomorpholine ring,

X denotes a group $CH-R_3$, $N-R_4$, SO or $SO_2$, or an oxygen or sulphur atom,

$R_3$ denotes a hydrogen atom or an alkyl group containing 1 to 3 carbon atoms,

$R_4$ denotes an alkyl group containing 1 to 3 carbon atoms,

m equals 0 or 1, and

n equals 0, 1 or 2,

with the proviso that, when X denotes a group SO, $SO_2$ or $N-R_4$, the sum m+n is equal to at least 1, with the proviso that, when A and B each denote a nitrogen atom and Z is in the para position with respect to B, X cannot denote the group $CH-R_3$, with the proviso that, when A denotes a CH group, B denotes a nitrogen atom, Z is in the ortho position with respect to B, X denotes an oxygen atom and R denotes a hydrogen atom, the sum m+n is other than 1; with the proviso that, when A denotes a CH group, B denotes a nitrogen atom, X denotes a $CH_2$ group and R denotes a hydrogen atom, the sum m+n is other than 2, and with the exception of 2-phenyl--4-quinolyl N,N-dimethylcarbamate, its diastereoisomers, racemics and enantiomers and, where it can exist, its salt of addition with an acid, characterized in that:

A - For preapring a compound of the general formula I in which X denotes an oxygen or sulphur atom, n and m are equal to 0 and A, B, V, W, Z, $R_1$ and $R_2$ are defined as above, a compound of formula

$$Cl-CO-N{<}^{R_1}_{R_2} \quad (II)$$

is reacted with a derivative of formula

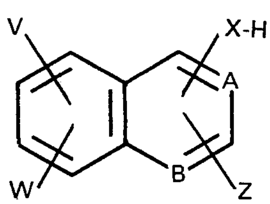

(III)

in which A, B, V, W and Z have the same meanings as above and X denotes an oxygen or sulphur atom, and the product is isolated and optionally converted to an addition salt with an acid,

B - For preparing a compound of the formula I in which X denotes an oxygen or sulphur atom, n equals 0, m equals 1 and A, B, V, W, Z, R, $R_1$ and $R_2$ are defined as above, a compound of formula

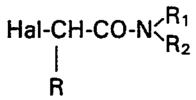

(IV)

is reacted with a derivative of formula

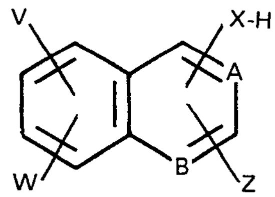

(III)

in which Hal denotes a halogen atom, X denotes an oxygen or sulphur atom and A, B, V, W, Z, R, $R_1$ and $R_2$ have the same meanings as abobe, and the product is isolated and optionally converted to an addition salt with an acid,

C - For preparing a compound of the formula I in which X denotes an oxygen or sulphur atom or denotes a group $CH\text{-}R_3$, n equals 0, 1 or 2 and m equals 1, and A, B, V, W, Z, R, $R_1$, $R_2$ and $R_3$ are defined as above, a compound of formula

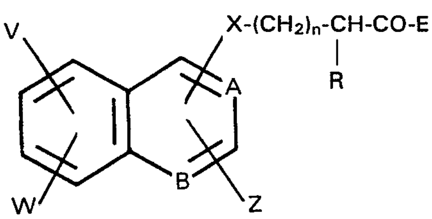

(VI)

is treated with an amine of formula

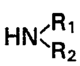

(V)

in which E denotes an alkoxy group containing 1 to 4 carbon atoms, an alkoxycarbonyloxy group containing 2 to 5 carbon atoms, or a chlorine atom, X is an oxygen or sulphur atom or a group $CH\text{-}R_3$, and A, B, V, W, Z, R, $R_1$, $R_2$ and n have the same meanings as above, and the product is isolated and optionally converted to an addition salt with an acid,

D - For preparing a compound of the formula I in which X denotes a sulphinyl or sulphonyl group, m equals 1, and A, B, V, W, Z, R, $R_1$, $R_2$ and n are defined as above, the corresponding compounds in which X denotes a sulphur atom are oxidized, and the product is isolated and optionally converted to an addition salt with an acid,

E - For preparing a compound of the formula I in which A is a CH group, B is a nitrogen atom, Z is bound in the ortho position with respect to B, the chain $X\text{-}(CH_2)_n\text{-}(CHR)_m\text{-}CONR_1R_2$ is bound in the para position with respect to B, m equals 0, n equals 0, X denotes a group $CH\text{-}R_3$, $R_3$ denotes an alkyl group containing 1 to 3 carbon atoms and V, W, Z, $R_1$ and $R_2$ are defined as above, a compound of formula

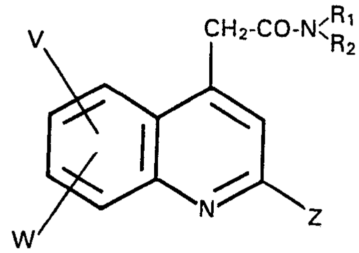

(VIII)

is alkylated with a compound of formula $R_3\text{-}Hal$

in which V,W, Z, $R_1$, $R_2$ and $R_3$ have the same meanings as above and Hal denotes a halogen atom, and the product is isolated and optionally converted to an addition salt with an acid,

F - For preparing a compound of the formula I in which X denotes a group $N\text{-}R_4$, m equals 1, and A, B, V, W, Z, R, $R_1$, $R_2$, $R_4$ and n are defined as above, a compound of formula

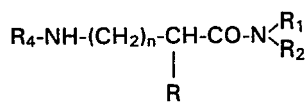

(IX)

is reacted with a derivative of formula

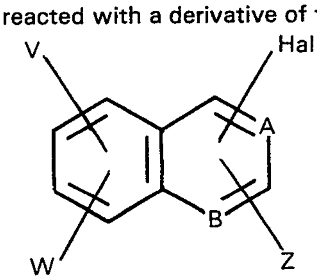

(X)

in which Hal denotes a halogen atom and A, B, V, W, Z, R, $R_1$, $R_2$, $R_2$, $R_4$ and n have the same meanings as above, and the product is isolated and optionally converted to an addition salt with an acid,

G - For preparing a compound of the formula I in which A and B each denote a nitrogen atom, Z is bound in the ortho position with respect to B, the chain $X\text{-}(CH_2)_n\text{-}(CHR)_m\text{-}CO\text{-}NR_1R_2$ is bound in the para position with respect to B, X is a $CH_2$ group, n equals 1, m equals 0, and V, W, $R_1$ and $R_2$ are defined as above, a chloride of formula ZCOCl is reacted with a compound of formula

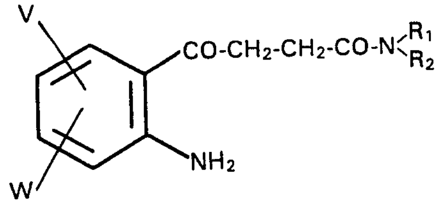

(XI)

in which V, W, Z, $R_1$ and $R_2$ have the same meanings as above, the intermediate thereby obtained is cyclized and the product is isolated and optionally converted to an addition salt with an acid.